# EUROPEAN PATENT APPLICATION

(11) **EP 4 717 689 A2**
(43) Date of publication of application: **01.04.2026**
(21) Application number: 25217993.2
(22) Date of filing: 19.10.2022
(51) Int. Cl.: C07C 309/04

(54) **CRYSTALLINE FORMS OF QUINAZOLINE DERIVATIVES, PREPARATION, COMPOSITION AND USE THEREOF**

(30) Priority: 20.10.2021 WO PCT/CN2021/125016
(62) Divisional of application: 22882901.6
(71) Applicant: F. Hoffmann-La Roche AG, 4070 Basel (CH)
(72) Inventor: WANG, Zheng, Shanghai, 200135 (CN); ZHOU, Ding, Shanghai, 200230 (CN); CHENG, Ziqiang, Shanghai, 201203 (CN)
(74) Representative: Carpmaels & Ransford LLP

(57) **Abstract**

The present application relates to crystalline forms of (R)-N-(4-([1,2,4]triazolo[1,5-c]pyrimidin-7-yloxy)-3-methylphenyl)-5-((3,3-difluoro-1-methylpiperidin-4-yl)oxy)-6-methoxyquinazolin-4-amine, methods for the preparation thereof, pharmaceutical compositions comprising one or more of the crystalline forms as an active ingredient, and use of the crystalline forms in the treatment of hyperproliferative diseases.

## Description

### CROSS-REFERENCE TO RELATED APPLICATION

This application claims the benefit of priority to International Patent Application No. PCT/CN2021/125016 filed 10/20/2021, the disclosure of which is incorporated herein in its entirety by reference.

### FIELD OF THE INVENTION

The present application relates to crystalline forms of (R)-N-(4-([1,2,4]triazolo[1,5-c]pyrimidin-7-yloxy)-3-methylphenyl)-5-((3,3-difluoro-1-methylpiperidin-4-yl)oxy)-6-methoxyquinazolin-4-amine, methods for the preparation thereof, pharmaceutical compositions comprising one or more of the crystalline forms as an active ingredient, and use of the crystalline forms in the treatment of hyperproliferative diseases.

### BACKGROUND OF THE INVENTION

The type I tyrosine kinase receptor family consists of four structurally related receptors: EGFR (ErbB1 or HER1), ErbB2 (HER2), ErbB3 (HER3), and ErbB4 (HER4) (Reviewed in Riese and Stern, Bioessays, 1998, 20: 41-48; Olayioye et al., EMBO Journal, 2000, 19. 3159-3167, and Schlessinger, Cell, 2002, 110: 669-672). The structures of all the four family members are nearly the same, made up of an extracellular region or ectodomain or ligand binding region, a single transmembrane-spanning region, and an intracellular cytoplasmic tyrosine kinase domain.

It has been demonstrated that ErbB2 plays a role in development of cancer. ErbB2 overexpression occurs in 20-25% of breast cancer (BC) patients (Leyland-Jones B, J Clin Oncol., 2009, 5278-86). About 1.7 million new BC incidences are diagnosed every year (Cardoso F, et al., Breast, 2018, 131-138) and 80% of BC are invasive, which require chemotherapy, radiation or target therapy besides surgery (Dai X., et al., Am J Cancer Res, 2015, 2929-2943). Brain metastases are a frequent occurrence in metastatic breast cancer patients. Overall survival for breast cancer brain metastases (BCBM) patients ranges from 2-25.3 months (Leone J. P. Exp. Hematol. Oncol., 2015, 4, 33). Surgery, whole brain radiation therapy (WBRT) and stereotactic radiosurgery (SRS) are the three main treatment options for BCBM. Surgery is used for solitary or up to three brain metastases. SRS can be used in patients with four or fewer intracranial lesions. WBRT is used to manage multiple brain metastases, but can lead to significant neuro-cognitive decline (Venur VA. et al., Int. J. Mol. Sci., 2016, 1543).

Compared to other types of breast cancer, ErbB2 positive tumors have a higher incidence of brain metastases, up to 50% of ErbB2 positive breast cancer patients develop intracranial metastases (Leyland-Jones B, J Clin Oncol., 2009, 5278-86). The high prevalence of BCBM in ErbB2 positive patients is ascribed to inherent tropism of ErbB2 positive breast cancer cells to the brain, prolonged survival of patients treated with anti-ErbB2 therapy and limited intracranial activity of anti-ErbB2 therapy (Venur V.A. et al., Int. J. Mol. Sci., 2016, 17, 1543).

Several anti-ErbB2 agents have been developed for clinical use, including monoclonal antibodies such as Trastuzumab, antibody drug conjugates (ADC) such as T-DM1, and tyrosine kinase inhibitors (TKIs) such as lapatinib, neratinib, afatinib and tucatinib (Kabraji S. et al., Clinical Cancer Research, 2018, 3351; Askoxylakis V., et al., JNCI J Natl Cancer Inst, 2015, 763-763; Tanaka, Y. et al., Scientific Reports, 2018, 343; Zhang, Shirong, et al., Acta Pharmacologica Sinica, 2017, 233-240; Dinkel V, et al., Cancer Research, 2012, 72). However, none of these antibodies, ADC and TKIs are believed as central nervous system (CNS) penetrable. Limited clinical efficacy observed when treating BCBM patients with non-brain penetrable aforementioned antibody, ADC and TKIs.

International Patent Publication No. WO 2020/057511 A1, which is incorporated herein by reference in its entirety, discloses quinazoline compounds that inhibit type I receptor tyrosine kinases, demonstrate good brain penetration in animals, and possess favorable toxicity profiles (for example a decreased activity against hERG), and thus particularly useful in the treatment of type I receptor tyrosine kinases mediated diseases or conditions, in particular ErbB2-associated disease or conditions, including cancer (e.g., metastatic cancer, such as brain metastases). A specific compound, which is identified as (R)-N-(4-([1,2,4]triazolo[1,5-c]pyrimidin-7-yloxy)-3-methylphenyl)-5-((3,3-difluoro-1-methylpiperidin-4-yl)oxy)-6-methoxyquinazolin-4-amine (also referred to as compound (I) herein), 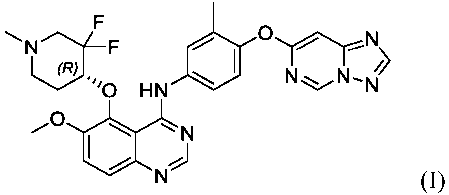 has been considered as an efficient blood-brain barrier (BBB)-penetrable ErbB2 (HER2) inhibitor exhibiting high selectivity against wild type EGFR to minimize EGFR mediated diarrhea and skin rash. and thus useful for treating ErbB2 positive BC patients with or without brain metastasis (see, Example 31 and Biochemical Assay in WO 2020/057511 A1).

Polymorphism is the occurrence of different crystalline forms of a single compound and it is a property of some compounds and complexes. Thus, polymorphs are distinct solids sharing the same molecular formula, yet each polymorph may have distinct solid state physical properties. Therefore, a single compound may give rise to a variety of polymorphic forms where each form has different and distinct solid state physical properties, such as different solubility profiles, melting point temperatures, flowability, dissolution rates and/or different X-ray diffraction peaks. These practical physical characteristics are influenced by the conformation and orientation of molecules in the unit cell, which defines a particular polymorphic form of a substance. Due to the possibility of variable solubility of each polymorph, identifying the existence of pharmaceutical polymorphs is essential for providing pharmaceuticals with predictable solubility profiles. It is desirable to investigate all the solid-state forms of a drug, including all polymorphic forms, and to determine the stability, dissolution and flow properties of each polymorphic form. Polymorphic forms of a compound can be distinguished in a laboratory by X-ray diffraction spectroscopy, such as X-ray powder diffraction ("XRPD"), and by other methods, such as infrared spectrometry. Additionally, polymorphic forms of the same drug substance or active pharmaceutical ingredient can be administered by itself or formulated as a drug product pharmaceutical composition and are well known in the pharmaceutical art to affect, for example, the solubility, stability, flowability, tractability and compressibility of drug substances and the safety and efficacy of drug products. For more, see Hilfiker, Rolf (ed.), Polymorphism in the Pharmaceutical Industry, Weinheim, Germany: Wiley-VCH 2006.

Discovery of new polymorphic forms of a pharmaceutically useful compound provides a new opportunity to improve the performance characteristics of a pharmaceutical product. Therefore, there is a continuous need to investigate polymorphic forms of aforementioned quinazoline compounds which exhibit type I receptor tyrosine kinases inhibitory activity. It has now been found that new polymorphic forms of compound (I) exist.

### SUMMARY OF THE INVENTION

In one aspect, the present invention is directed to crystalline forms of (*R*)-N-(4-([1,2,4]triazolo[1,5-c]pyrimidin-7-yloxy)-3-methylphenyl)-5-((3,3-difluoro-1-methylpiperidin-4-yl)oxy)-6-methoxyquinazolin-4-amine (compound (I)), which is a complex of free base with a pharmaceutically acceptable acid, or free base.

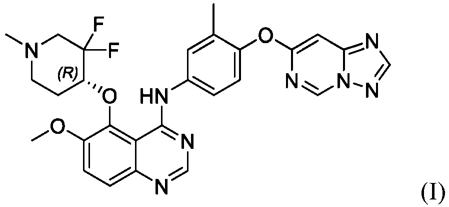

In some embodiments, the complex of free base with a pharmaceutically acceptable acid or the free base is a solvate or non-solvate. In some embodiments, the complex of free base with a pharmaceutically acceptable acid is a salt or a cocrystal or a cocrystal of salt. In some embodiments, disclosed herein are crystalline forms of compound (I), designated as Fumarate Type A, Fumarate Type B, Fumarate Type C, Fumarate Type E, Freebase Type A, Freebase Type B, Freebase Type C, Freebase Type D, Freebase Type E, Freebase Type F, Freebase Type G, HCl Salt Type A, HCl Salt Type B, Mesylate Type A, Mesylate Type B, Phosphate Type A, L-tartrate Type A and Adipate Type A.

In another aspect, the present invention is directed to amorphous forms of compound (I), which is a pharmaceutically acceptable salt or free base. In some embodiments, disclosed herein are amorphous forms of compound (I), designated as Amorphous Fumarate and Amorphous Freebase.

In another aspect, the present invention is directed to methods for the preparation of the crystalline forms of compound (I).

Also, disclosed herein are methods for the preparation of the amorphous forms of compound (I).

In another aspect, the present invention is directed to pharmaceutical compositions comprising the crystalline form or amorphous form of compound (I), and a pharmaceutically acceptable carrier or excipient.

In another aspect, the present invention is directed to dosage forms comprising a therapeutically effective amount of the crystalline form, the amorphous form, or the pharmaceutical composition described herein.

In another aspect, the present invention is directed to methods of treating or ameliorating a hyperproliferative disease in a subject, comprising administering to the subject in need thereof a therapeutically effective amount of the crystalline form or amorphous form of compound (I).

In another aspect, the present invention is directed to crystalline forms or amorphous forms of compound (I) for use in treating or ameliorating a hyperproliferative disease.

In another aspect, the present invention is directed to use of crystalline forms or amorphous forms of compound (I) in the manufacture of a medicament for treating or ameliorating a hyperproliferative disease.

### BRIEF DESCRIPTION OF THE FIGURES

The summary, as well as the following detailed description, is further understood when read in conjunction with the appended drawings. For the purpose of illustrating the invention, there are shown in the drawings' exemplary embodiments of the invention; however, the invention is not limited to the specific disclosure of the drawings. In the drawings:
Figure 1 is a representative XRPD pattern of Fumarate Type A.
Figure 2 is a representative TGA curve of Fumarate Type A.
Figure 3 is a representative DSC curve of Fumarate Type A.
Figure 4 is a representative ¹H NMR spectrum of Fumarate Type A,.
Figure 5 is a representative PLM image of Fumarate Type A.
Figure 6 is a representative XRPD pattern of Fumarate Type B.
Figure 7 is a representative TGA curve of Fumarate Type B.
Figure 8 is a representative DSC curve of Fumarate Type B.
Figure 9 is a representative ¹H NMR spectrum of Fumarate Type B.
Figure 10 is a representative XRPD pattern of Fumarate Type C.
Figure 11 is a representative XRPD pattern of Fumarate Type E.
Figure 12 is a representative TGA curve of Fumarate Type E.
Figure 13 is a representative DSC curve of Fumarate Type E.
Figure 14 is a representative XRPD pattern of Freebase Type B.
Figure 15 is a representative TGA curve of Freebase Type B.
Figure 16 is a representative DSC curve of Freebase Type B.
Figure 17 is a representative ¹H NMR spectrum of Freebase Type B.
Figure 18 is a representative PLM image of Freebase Type B.
Figure 19 is a representative XRPD pattern of Freebase Type C.
Figure 20 is a representative TGA curve of Freebase Type C.
Figure 21 is a representative DSC curve of Freebase Type C.
Figure 22 is a representative ¹H NMR spectrum of Freebase Type C.
Figure 23 is a representative XRPD pattern of Freebase Type D.
Figure 24 is a representative XRPD pattern of Freebase Type E.
Figure 25 is a representative XRPD pattern of Freebase Type F.
Figure 26 is a representative TGA curve of Freebase Type F.
Figure 27 is a representative DSC curve of Freebase Type F.
Figure 28 is a representative XRPD pattern of Freebase Type G.
Figure 29 is a representative TGA curve of Freebase Type G.
Figure 30 is a representative DSC curve of Freebase Type G.
Figure 31 is a representative XRPD pattern of Freebase Type A.
Figure 32 is a representative TGA curve of Freebase Type A.
Figure 33 is a representative DSC curve of Freebase Type A.
Figure 34 is a representative ¹H NMR spectrum of Freebase Type A.
Figure 35 is a representative XRPD pattern of HCl salt Type A.
Figure 36 is a representative TGA curve of HCl salt Type A.
Figure 37 is a representative DSC curve of HCl salt Type A.
Figure 38 is a representative XRPD pattern of HCl salt Type B.
Figure 39 is a representative TGA curve of HCl salt Type B.
Figure 40 is a representative DSC curve of HCl salt Type B.
Figure 41 is a representative XRPD pattern of Mesylate Type A.
Figure 42 is a representative TGA curve of Mesylate Type A.
Figure 43 is a representative DSC curve of Mesylate Type A.
Figure 44 is a representative XRPD pattern of Mesylate Type B.
Figure 45 is a representative TGA curve of Mesylate Type B.
Figure 46 is a representative DSC curve of Mesylate Type B.
Figure 47 is a representative XRPD pattern of Phosphate Type A.
Figure 48 is a representative TGA curve of Phosphate Type A.
Figure 49 is a representative DSC curve of Phosphate Type A.
Figure 50 is a representative XRPD pattern of L-tartrate Type A.
Figure 51 is a representative TGA curve of L-tartrate Type A.
Figure 52 is a representative DSC curve of L-tartrate Type A.
Figure 53 is a representative XRPD pattern of Adipate Type A.
Figure 54 is a representative TGA curve of Adipate Type A.
Figure 55 is a representative DSC curve of Adipate Type A.
Figure 56 is a representative XRPD pattern of Amorphous Fumarate.
Figure 57 is a representative TGA curve of Amorphous Fumarate.
Figure 58 is a representative mDSC curve of Amorphous Fumarate.
Figure 59 is a representative XRPD pattern of Amorphous Freebase.
Figure 60 is a representative TGA curve of Amorphous Freebase.
Figure 61 is a representative mDSC curve of Amorphous Freebase.

### DETAILED DESCRIPTION OF THE INVENTION

Reference will now be made in detail to certain embodiments, examples of which are illustrated in the accompanying detailed description. While enumerated embodiments will be described, it will be understood that they are not intended to limit the invention to those embodiments. On the contrary, the invention is intended to cover all alternatives, modifications, and equivalents, which may be included within the scope of the present invention as defined by the claims. One skilled in the art will recognize many methods and materials similar or equivalent to those described herein, which could be used in the practice of the present invention. The present invention is in no way limited to the methods and materials described. In the event that one or more of the incorporated literatures and similar materials differs from or contradicts this application, including but not limited to defined terms, term usage, described techniques, or the like, this application controls.

It is appreciated that certain features of the present invention, which are, for clarity, described in the context of separate embodiments, can also be provided in combination in a single embodiment. Conversely, various features of the present invention, which are, for brevity, described in the context of a single embodiment, can also be provided separately or in any suitable sub-combination.

### DEFINITIONS

The terms used herein have their ordinary meaning and the meaning of such terms is independent at each occurrence thereof. Nevertheless, unless otherwise stated, the following definitions apply throughout the specification and claims.

As used herein, the terms "comprise", "comprising", "include", and "including" are intended to specify the presence of stated features, integers, components, or steps, but they do not preclude the presence or addition of one or more other features, integers, components, steps, or groups thereof.

As used herein, the term "about" means approximately, in the region of, roughly, or around. When the term "about" is used in conjunction with a numerical range, it modifies that range by extending the boundaries above and below the numerical values set forth. In general, the term "about" is used herein to modify a numerical value above and below the given value by a variance of 20%, typically 10%, more typically 5%, even more typically 1% and even more typically 0.1%. Sometimes, such a range can lie within the experimental error, type of standard methods used for the measurement and/or determination of a given value or range. When the term "about" is used with reference to temperatures from a differential scanning calorimetry thermogram (DSC) curve (e.g., onset of endothermic transition, melt, etc.), each of the temperature values is understood to mean the given value ± 5 °C, more typically ± 2 °C, unless otherwise indicated.

As used herein, the term "substantially the same" with reference to X-ray powder diffraction means that variabilities in reflection positions and relative intensities of the reflections are to be taken into account. For example, a typical precision of the 2-Theta (2θ) values is in the range of ± 0.2° of the given value in 2θ, more typically in the range of ± 0.1° in 2θ. Thus, for example, a reflection that usually appears at 6.9° in 2θ can appear between 6.7° and 7.1° in 20, more typically between 6.8 and 7.0° in 2θ on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

As used herein, when a polymorphic form is identified using one or more temperatures from a differential scanning calorimetry thermogram (DSC) curve (e.g., onset of endothermic transition, melt, etc.), each of the temperature values is understood to mean the given value ± 5 °C, more typically ± 2 °C, unless otherwise indicated.

### CRYSTALLINE FORMS AND AMORPHOUS FORMS

In one aspect, provided herein are crystalline forms of compound (I), particularly those designated as Fumarate Type A, Fumarate Type B, Fumarate Type C, Fumarate Type E, Freebase Type A, Freebase Type B, Freebase Type C, Freebase Type D, Freebase Type E, Freebase Type F, Freebase Type G, HCl Salt Type A, HCl Salt Type B, Mesylate Type A, Mesylate Type B, Phosphate Type A, L-tartrate Type A and Adipate Type A. Also, provided herein are amorphous forms of compound (I), particularly those designated as Amorphous Fumarate and Amorphous Freebase. Methods for the preparation of the crystalline forms or amorphous forms, and pharmaceutical compositions comprising the crystalline forms or amorphous forms are also provided.

The crystalline forms of compound (I) can be a complex of free base with a pharmaceutically acceptable acid, or free base. Contemplated such complexes include, but are not limited to, a salt or a cocrystal or a cocrystal of salt. The amorphous forms of compound (I) can be a pharmaceutically acceptable salt or free base.

As used herein, the term "crystalline form" means crystal structures in which a compound (or a salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions.

As used herein, the term "polymorphic form" or "polymorph" in the context of this specification refer to crystalline and amorphous forms as well as solvate and hydrate forms. Crystalline forms have different arrangements and/or conformations of the molecules in the crystal lattice. Amorphous forms consist of disordered arrangements of molecules that do not possess a distinguishable crystal lattice. Solvates are crystal forms containing either stoichiometric or nonstoichiometric amounts of a solvent. When a drug substance exists in polymorphic forms, it is said to exhibit polymorphism.

As used herein, the term "complex" refers to crystalline materials composed of two or more different molecules, one of which is the active pharmaceutical ingredient (API), in the same crystal lattice that are associated by ionic/nonionic and electrovalent/noncovalent bonds. In the context of this specification, the API may refer to compound (I).

As used herein, the term "cocrystal" refers to crystalline materials composed of two or more different molecules, one of which is the API, in the same crystal lattice that are associated by nonionic and noncovalent bonds.

As used herein, the term "salt" refers to any of numerous compounds that result from replacement of part or all of the acid hydrogen of an acid to form an ionic or electrovalent compound.

As used herein, the term "cocrystal of salt" refers to crystalline forms wherein a salified API and a co-former (or vice versa) are in the same crystal lattice that are associated by nonionic and noncovalent bonds.

As used herein, the term "pharmaceutically acceptable" indicates that the substance or composition is compatible chemically and/or toxicologically, with the other ingredients comprising a formulation, and/or the subjects being treated therewith.

As used herein, the term "pharmaceutically acceptable acid" may be such acids containing the pharmaceutically acceptable anions. Preferably, illustrative examples of pharmaceutically acceptable acids described herein include, but not limited to, hydrochloride, methanesulfonic acid, phosphoric acid, tartaric acid, fumaric acid, and adipic acid. More examples of appropriate acids may be found in e.g., P. H. Stahl and C. G. Wermuth, editors, Handbook of Pharmaceutical Salts: Properties, Selection and Use, Weinheim/Zürich: Wiley-VCH/VHCA, 2002.

As used herein, the term "pharmaceutically acceptable salt", unless otherwise indicated, includes salts that retain the biological effectiveness of the free base of the specified compound and that are not biologically or otherwise undesirable. Contemplated pharmaceutically acceptable salt forms include, but are not limited to, mono, bis, tris, tetrakis, and so on. Pharmaceutically acceptable salts are non-toxic in the amounts and concentrations at which they are administered. The preparation of such salts can facilitate the pharmacological use by altering the physical characteristics of a compound without preventing it from exerting its physiological effect. Useful alterations in physical properties include lowering the melting point to facilitate transmucosal administration and increasing the solubility to facilitate administering higher concentrations of the drug.

Pharmaceutically acceptable salts may include acid addition salts such as those containing sulfate, chloride, hydrochloride, fumarate, maleate, phosphate, sulfamate, acetate, citrate, lactate, tartrate, methane sulfonate, ethane sulfonate, benzene sulfonate, *p*-toluene sulfonate, cyclohexyl sulfamate and quinate. Preferably, illustrative examples of pharmaceutically acceptable salts described herein include hydrochloride salt, mesylate salt, phosphate salt, tartrate salt, fumarate salt and adipate salt. The salt described herein may have an acid/base molar ratio of about 0.5:1 to about 3:1, typically about 0.5:1 to about 2.5:1, more typically, about 1:1 to about 1.5:1. For example, the salt described herein may have an acid/base molar ratio of about 0.5:1, about 0.6:1, about 0.7:1, about 0.8:1, about 0.9:1, about 1:1, about 1.1:1, about 1.2:1, about 1.3:1, about 1.4:1, about 1.5:1, about 1.6:1, about 1.7:1, about 1.8:1, about 1.9:1, about 2:1, about 2.1:1, about 2.2:1, about 2.3:1, about 2.4:1 and about 2.5:1.

Pharmaceutically acceptable salts can be obtained from appropriate acids such as hydrochloric acid, maleic acid, sulfuric acid, phosphoric acid, sulfamic acid, acetic acid, citric acid, lactic acid, tartaric acid, malonic acid, methane sulfonic acid, ethane sulfonic acid, benzenesulfonic acid, p-toluene sulfonic acid, cyclohexyl sulfamic acid, fumaric acid, and quinic acid. Preferably, illustrative examples of appropriate acids may include hydrochloride, methane sulfonic acid, phosphoric acid, tartaric acid, fumaric acid and adipic acid.

Pharmaceutically acceptable salts can be prepared by standard techniques. For example, the free-base form of a compound can be dissolved in a suitable solvent, such as an aqueous or aqueous-alcohol solution containing the appropriate acid and then isolated by evaporating the solution. Thus, where the particular compound is a base, the desired pharmaceutically acceptable salt may be prepared by any suitable method available in the art, for example, treatment of the free base with an inorganic acid or with an organic acid.

It is to be understood that the crystalline or amorphous compound described herein can exist in non-solvated forms or solvated forms, and the present invention is intended to encompass all such forms.

As used herein, the terms "solvate" and "solvated" refer to solvent addition forms that contain either stoichiometric or non-stoichiometric amounts of solvent. Some compounds have a tendency to trap a fixed molar ratio of solvent molecules in the crystalline solid state, thus forming a solvate. For example, if the solvent is water, the solvate formed is a hydrate; if the solvent is alcohol, the solvate formed is an alcoholate; if the solvent is acetone, the solvate formed is an acetone solvate. Hydrates are formed by the combination of one or more molecules of water with one molecule of the substance in which the water retains its molecular state as H₂O. Examples of solvents that form solvates include, but are not limited to, water, isopropanol, ethanol, methanol, acetone, DMSO, ethyl acetate, acetic acid, and ethanolamine.

As used herein, the terms "non-solvate" and "non-solvated", indicate that no organic solvent is cooperated in or accommodated by the solid structure, which including both crystal structure and amorphous structure. Non-solvated forms may still contain residual organic solvents, which are not part of the solid structure but may be adsorbed on the surface or absorbed in disordered regions of the solid structure. Typically, a non-solvated form does not contain more than 2.0 weight %, typically not more than 1.0 weight % and more typically not more than 0.5 weight % of organic solvents, based on the weight of the crystalline form. The organic solvent content can be determined by thermogravimetric analysis (TGA), e.g., by determining the weight loss in the range of from 25° C to the melting point of the solid form at a heating rate of 10 K/min and/or by gas chromatography.

Accordingly, in one aspect, there is provided herein a crystalline form of compound (I) represented by the following structural formula: 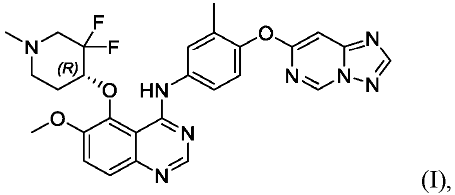 wherein the crystalline form is a complex of free base with a pharmaceutically acceptable acid, or free base.

In certain embodiments, the complex or free base is a solvate or non-solvate.

In certain embodiments, the complex is a salt or a cocrystal or a cocrystal of salt.

In certain embodiments, the complex has an acid/base molar ratio of about 3:1, preferably, about 0.5:1 to about 2.5:1, more preferably, about 1:1 to about 1.5:1.

In certain embodiments, the pharmaceutically acceptable acid is selected from a group consisting of hydrochloride, methanesulfonic acid, phosphoric acid, tartaric acid, fumaric acid and adipic acid.

In certain embodiments, the pharmaceutically acceptable acid is fumaric acid.

### Fumarate Type A

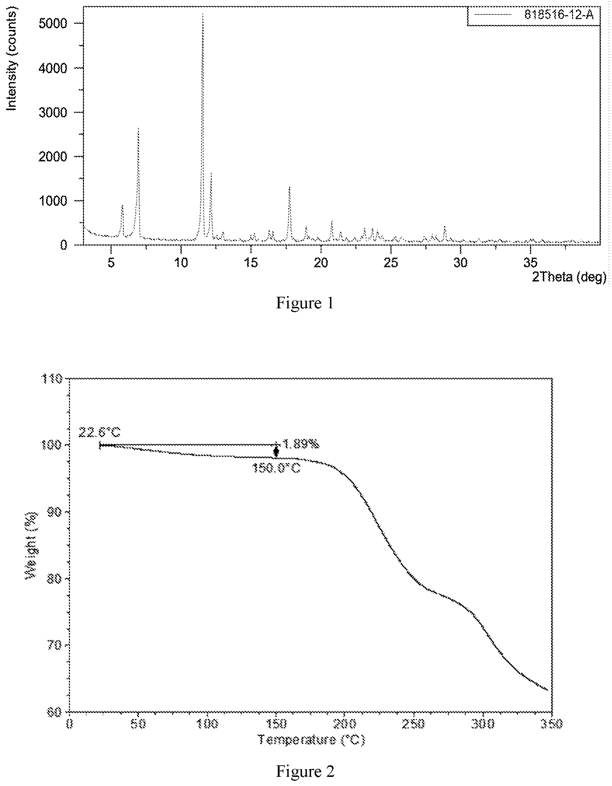

In certain embodiments, provided herein is a crystalline form of compound (I), Fumarate Type A, characterized by an X-ray powder diffraction pattern which comprises at least peaks at 2θ (± 0.2°) of 6.9 and 11.5; typically, by an X-ray powder diffraction pattern which comprises at least peaks at 2θ (± 0.2°) of 5.8, 6.9, 11.5, 12.1 and 17.7; more typically, by an X-ray powder diffraction pattern which comprises at least peaks at 2θ (± 0.2°) of 5.8, 6.9, 11.5, 12.1, 17.7, 20.8 and 24.0 ; more typically, by an X-ray powder diffraction pattern which comprises at least peaks at 2θ (± 0.2°) of 5.8, 6.9, 11.5, 12.1, 17.7, 18.9, 20.8, 23.1, 23.7, 24.0 and 28.8; even more typically, by substantially the same X-ray powder diffraction pattern as Figure 1. In certain embodiments, provided herein is a crystalline form of compound (I), Fumarate Type A, characterized by a differential scanning calorimeter peak phase transition temperature of about 167.6 °C.

### Fumarate Type B

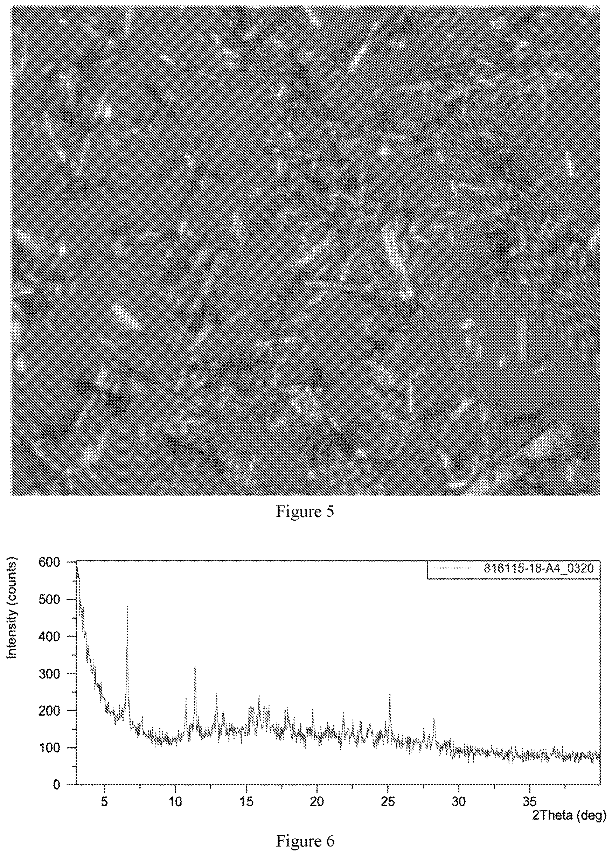

In certain embodiments, provided herein is a crystalline form of compound (I), Fumarate Type B, characterized by an X-ray powder diffraction pattern which comprises at least peaks 6.6 and 11.4; typically, by an X-ray powder diffraction pattern which comprises at least peaks at 2θ (± 0.2°) of 6.6, 10.7, 11.4, 12.9, 25.1 and 28.2; more typically, by an X-ray powder diffraction pattern which comprises at least peaks at 2θ (± 0.2°) of 6.6, 10.7, 11.4, 12.9, 15.8, 17.9, 19.7, 25.1 and 28.2; even more typically, by substantially the same X-ray powder diffraction pattern as Figure 6. In certain embodiments, provided herein is a crystalline form of compound (I), Fumarate Type B, characterized by a differential scanning calorimeter peak phase transition temperature of about 91.3 °C and about 166.3 °C.

### Fumarate Type C

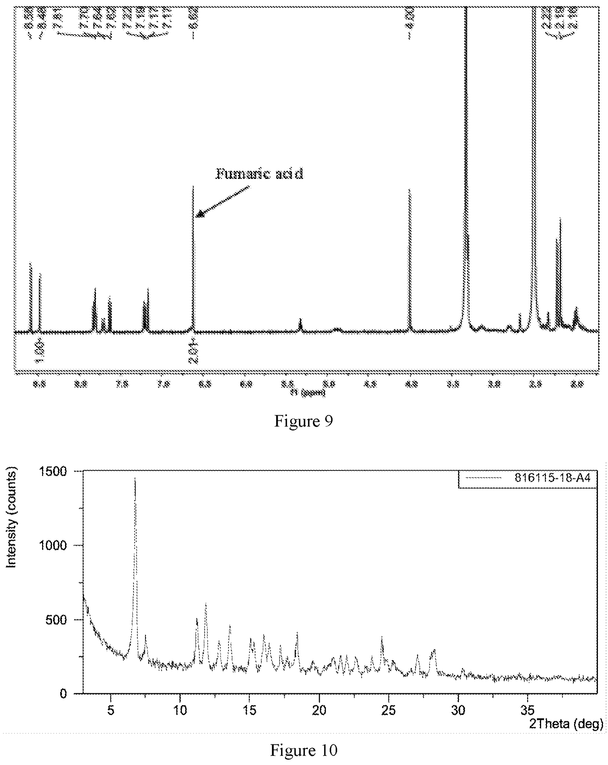

In certain embodiments, provided herein is a crystalline form of compound (I), Fumarate Type C, characterized by an X-ray powder diffraction pattern which comprises at least peaks at 2θ (± 0.2°) of 6.8 and 11.8; typically, by an X-ray powder diffraction pattern which comprises at least peaks at 2θ (± 0.2°) of 6.8, 11.2, 11.8, 13.6 and 18.4; more typically, by an X-ray powder diffraction pattern which comprises at least peaks at 2θ (± 0.2°) of 6.8, 11.2, 11.8, 13.6, 15.1, 16.0, 17.2, 18.4 and 24.5; even more typically, by substantially the same X-ray powder diffraction pattern as Figure 10.

### Fumarate Type E

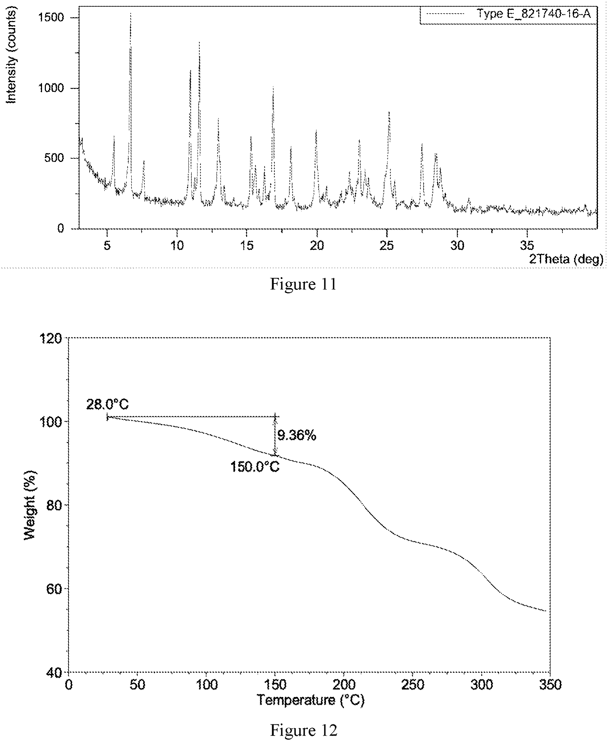

In certain embodiments, provided herein is a crystalline form of compound (I), Fumarate Type E, characterized by an X-ray powder diffraction pattern which comprises at least peaks at 2θ (± 0.2°) of 6.7, 11.6 and 28.5; typically, by an X-ray powder diffraction pattern which comprises at least peaks at 2θ (± 0.2°) of 6.7, 10.9, 11.6, 16.9, 25.2 and 28.5; more typically, by an X-ray powder diffraction pattern which comprises at least peaks at 2θ (± 0.2°) of 6.7, 10.9, 11.6, 12.9, 15.3, 16.9, 19.9, 25.2 and 28.5; more typically, by an X-ray powder diffraction pattern which comprises at least peaks at 2θ (± 0.2°) of 5.5, 6.7, 10.9, 11.6, 12.9, 15.3, 16.9, 18.1, 19.9, 25.2, 27.5 and 28.5; even more typically, by substantially the same X-ray powder diffraction pattern as Figure 11. In certain embodiments, provided herein is a crystalline form of compound (I), Fumarate Type E, characterized by a differential scanning calorimeter peak phase transition temperature of about 134.5 °C and about 166.0 °C.

### Freebase Type B

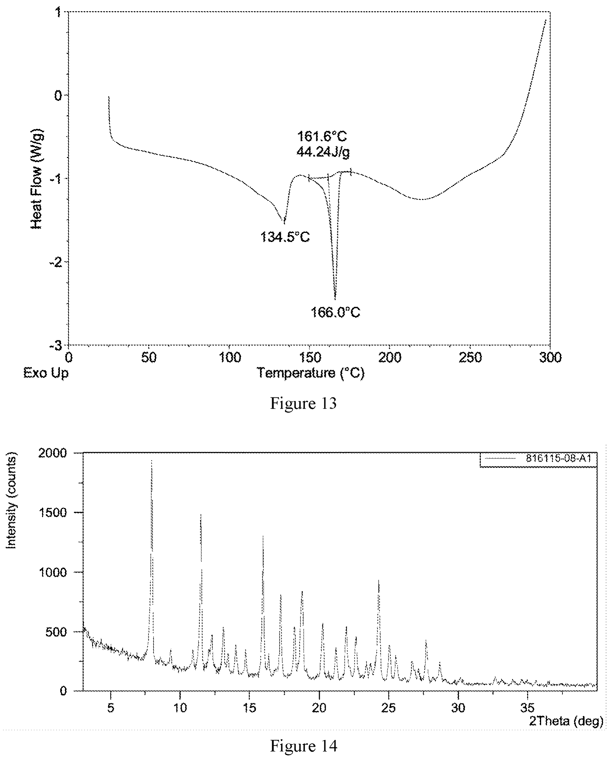

In certain embodiments, provided herein is a crystalline form of compound (I), Freebase Type B, characterized by an X-ray powder diffraction pattern which comprises at least peaks at 2θ (± 0.2°) of 8.0 and 11.5; typically, by an X-ray powder diffraction pattern which comprises at least peaks at 2θ (± 0.2°) of 8.0, 11.5, 16.0, 17.2, 18.8 and 24.3; more typically, by an X-ray powder diffraction pattern which comprises at least peaks at 2θ (± 0.2°) of 8.0, 11.5, 16.0, 17.2, 18.2, 18.8, 20.3, 21.9 and 24.3; more typically, by an X-ray powder diffraction pattern which comprises at least peaks at 2θ (± 0.2°) of 8.0, 11.5, 13.1, 16.0, 17.2, 18.2, 18.8, 20.3, 21.2, 21.9, 24.3 and 27.7; even more typically, by substantially the same X-ray powder diffraction pattern as Figure 14. In certain embodiments, provided herein is a crystalline form of compound (I), Freebase Type B, characterized by a differential scanning calorimeter peak phase transition temperature of about 169.4 °C.

### Freebase Type C

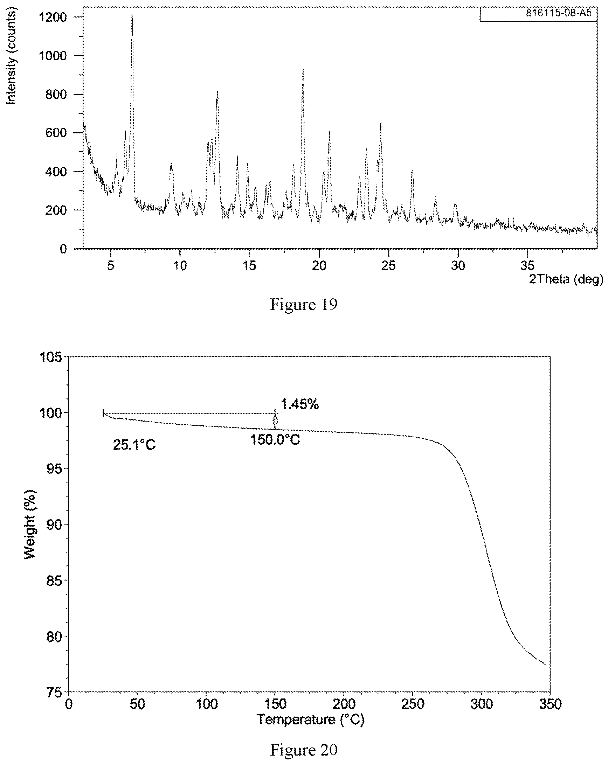

In certain embodiments, provided herein is a crystalline form of compound (I), Freebase Type C, characterized by an X-ray powder diffraction pattern which comprises at least peaks at 2θ (± 0.2°) of 6.6 and 18.8; typically, by an X-ray powder diffraction pattern which comprises at least peaks at 2θ (± 0.2°) of 6.6, 12.7, 18.8, 20.7 and 24.4; more typically, by an X-ray powder diffraction pattern which comprises at least peaks at 2θ (± 0.2°) of 6.6, 12.7, 14.1, 18.1, 18.8, 20.7, 23.4, 24.4 and 26.7; more typically, by an X-ray powder diffraction pattern which comprises at least peaks at 2θ (± 0.2°) of 6.6, 9.4, 12.7, 14.1, 14.9, 18.1, 18.8, 20.7, 22.9, 23.4, 24.4 and 26.7; even more typically, by substantially the same X-ray powder diffraction pattern as Figure 19. In certain embodiments, provided herein is a crystalline form of compound (I), Freebase Type C, characterized by a differential scanning calorimeter peak phase transition temperature of about 86.2 °C and about 114.4 °C.

### Freebase Type D

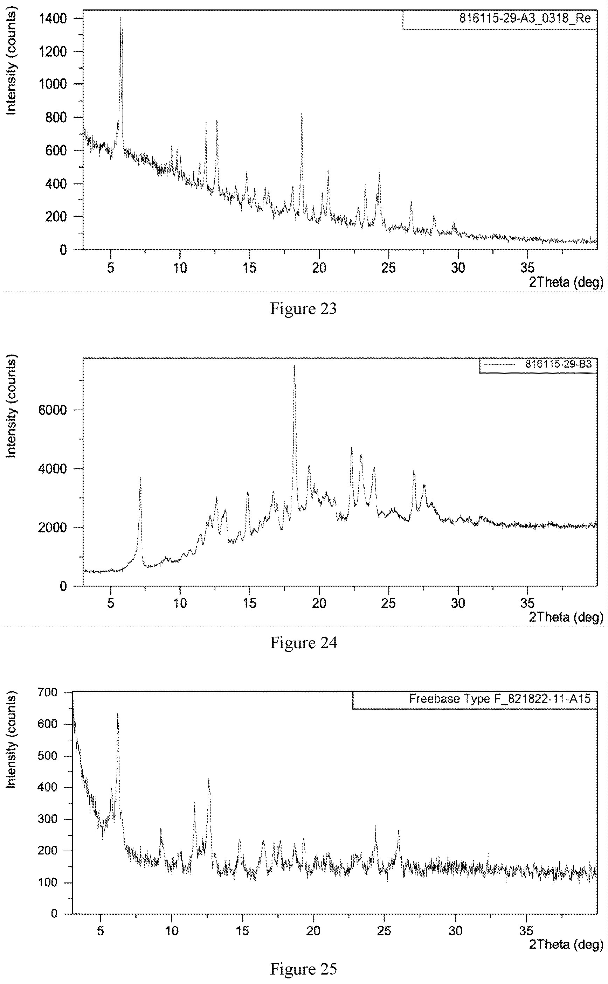

In certain embodiments, provided herein is a crystalline form of compound (I), Freebase Type D, characterized by an X-ray powder diffraction pattern which comprises at least peaks at 2θ (± 0.2°) of 5.7, 5.8 and 18.8; typically, by an X-ray powder diffraction pattern which comprises at least peaks at 2θ (± 0.2°) of 5.7, 5.8, 11.8, 12.6, 18.8, 20.6 and 24.3; more typically, by an X-ray powder diffraction pattern which comprises at least peaks at 2θ (± 0.2°) of 5.7, 5.8, 11.8, 12.6, 18.8, 20.6, 22.8, 23.3 and 24.3; even more typically, by substantially the same X-ray powder diffraction pattern as Figure 23.

### Freebase Type E

In certain embodiments, provided herein is a crystalline form of compound (I), Freebase Type E, characterized by an X-ray powder diffraction pattern which comprises at least peaks at 2θ (± 0.2°) of 7.2, 18.2 and 22.3; typically, by an X-ray powder diffraction pattern which comprises at least peaks at 2θ (± 0.2°) of 7.2, 18.2, 19.2, 22.3, 23.0 and 24.0; more typically, by an X-ray powder diffraction pattern which comprises at least peaks at 2θ (± 0.2°) of 7.2, 14.9, 16.7, 18.2, 19.2, 22.3, 23.0, 24.0 and 26.8; more typically, by an X-ray powder diffraction pattern which comprises at least peaks at 2θ (± 0.2°) of 7.2, 12.6, 14.9, 16.7, 18.2, 19.2, 19.7, 20.5, 22.3, 23.0, 24.0 and 26.8; even more typically, by substantially the same X-ray powder diffraction pattern as Figure 24.

### Freebase Type F

In certain embodiments, provided herein is a crystalline form of compound (I), Freebase Type F, characterized by an X-ray powder diffraction pattern which comprises at least peaks at 2θ (± 0.2°) of 6.2, 11.6 and 12.6; typically, by an X-ray powder diffraction pattern which comprises at least peaks at 2θ (± 0.2°) of 6.2, 11.6, 12.6, 14.8, 16.5 and 24.4; more typically, by an X-ray powder diffraction pattern which comprises at least peaks at 2θ (± 0.2°) of 6.2, 11.6, 12.6, 14.8, 16.5, 17.6, 19.3, 24.4 and 26.0; more typically, by an X-ray powder diffraction pattern which comprises at least peaks at 2θ (± 0.2°) of 6.2, 9.3, 11.6, 12.6, 14.8, 16.5, 17.6, 18.7, 19.3, 24.4 and 26.0; even more typically, by substantially the same X-ray powder diffraction pattern as Figure 25. In certain embodiments, provided herein is a crystalline form of compound (I), Freebase Type F, characterized by a differential scanning calorimeter peak phase transition temperature of about 55.4 °C and about 109.5 °C.

### Freebase Type G

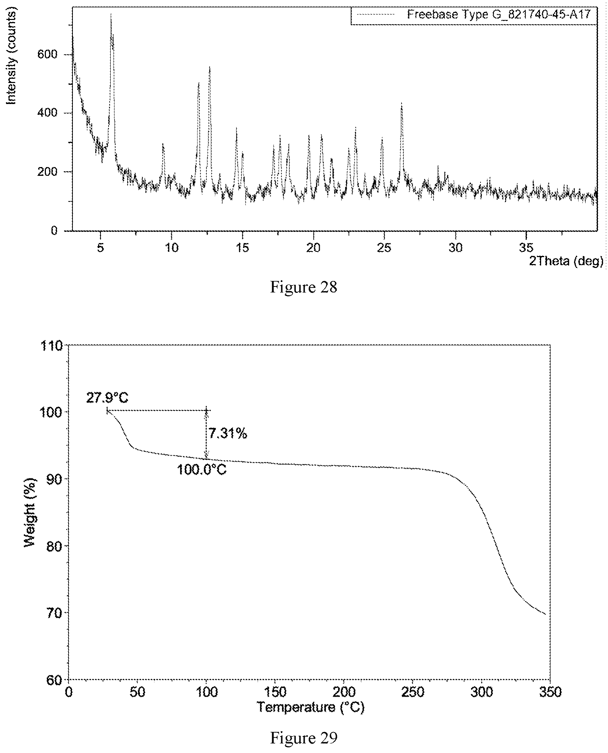

In certain embodiments, provided herein is a crystalline form of compound (I), Freebase Type G, characterized by an X-ray powder diffraction pattern which comprises at least peaks at 2θ (± 0.2°) of 5.7, 5.9 and 12.7; typically, by an X-ray powder diffraction pattern which comprises at least peaks at 2θ (± 0.2°) of 5.7, 5.9, 11.9, 12.7, 14.5 and 26.2; more typically, by an X-ray powder diffraction pattern which comprises at least peaks at 2θ (± 0.2°) of 5.7, 5.9, 11.9, 12.7, 14.5, 17.6, 19.7, 22.9 and 26.2; more typically, by an X-ray powder diffraction pattern which comprises at least peaks at 2θ (± 0.2°) of 5.7, 5.9, 11.9, 12.7, 14.5, 17.2, 17.6, 19.7, 20.6, 22.9, 24.8 and 26.2; even more typically, by substantially the same X-ray powder diffraction pattern as Figure 28. In certain embodiments, provided herein is a crystalline form of compound (I), Freebase Type G, characterized by a differential scanning calorimeter peak phase transition temperature of about 32.9 °C, about 59.2 °C and about 110.2 °C.

### Freebase Type A

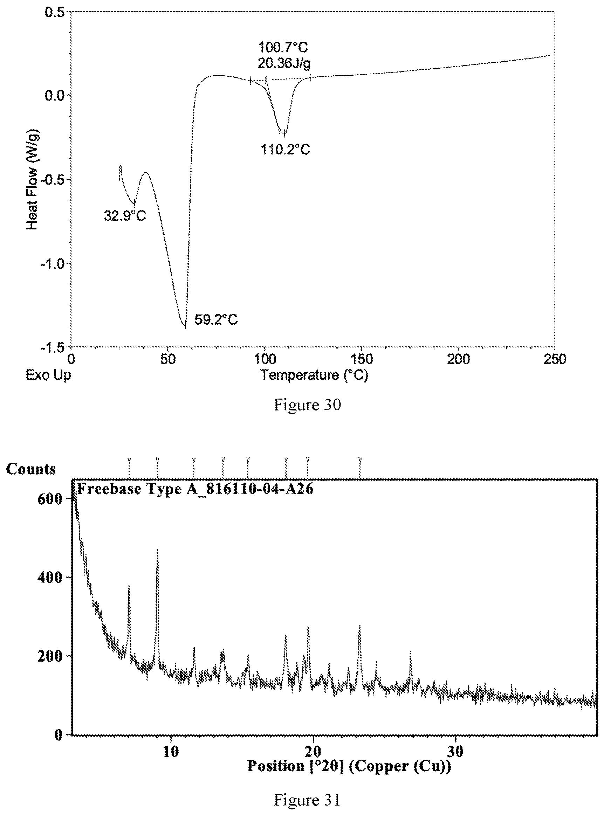

In certain embodiments, provided herein is a crystalline form of compound (I), Freebase Type A, characterized by an X-ray powder diffraction which comprises at least peaks at 2θ (± 0.2°) of 7.0, 9.0 and 23.3; typically, by an X-ray powder diffraction which comprises at least peaks at 2θ (± 0.2°) of 7.0, 9.0, 11.6, 13.6, 15.4, 18.1, 19.6 and 23.3; more typically, by substantially the same X-ray powder diffraction pattern as Figure 31. In certain embodiments, provided herein is a crystalline form of compound (I), Freebase Type A, characterized by a differential scanning calorimeter peak phase transition temperature of about 71.3 °C.

### HCl Salt Type A

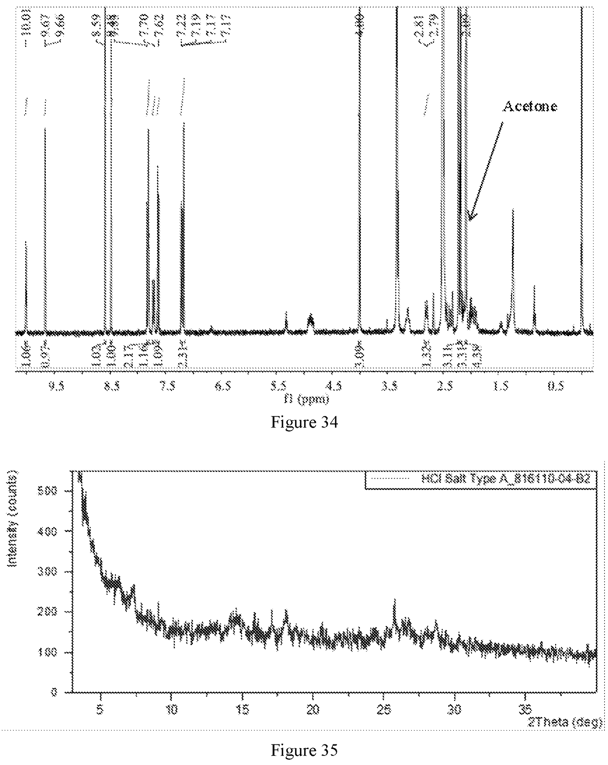

In certain embodiments, provided herein is a crystalline form of compound (I), HCl Salt Type A, characterized by an X-ray powder diffraction which comprises at least peaks at 2θ (± 0.2°) of 18.1; typically, by substantially the same X-ray powder diffraction pattern shown in Figure 35. In certain embodiments, provided herein is a crystalline form of compound (I), HCl Salt Type A, characterized by a differential scanning calorimeter peak phase transition temperature of about 110.0 °C.

### HCl Salt Type B

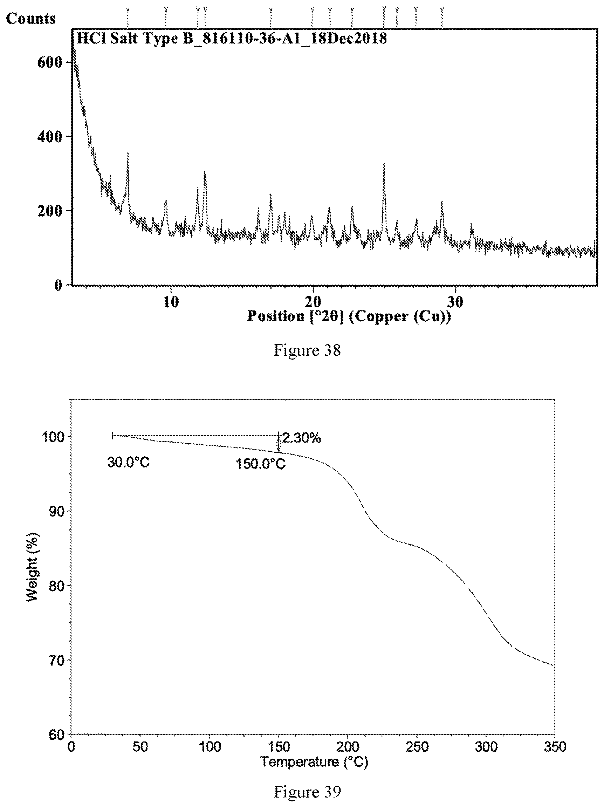

In certain embodiments, provided herein is a crystalline form of compound (I), HCl Salt Type B, characterized by an X-ray powder diffraction pattern which comprises at least peaks at 2θ (± 0.2°) of 6.9, 12.4 and 25.0; typically, by an X-ray powder diffraction pattern which comprises at least peaks at 2θ (± 0.2°) of 6.9, 11.9, 12.4, 17.0, 25.0 and 29.1; more typically, by an X-ray powder diffraction pattern which comprises at least peaks at 2θ (± 0.2°) of 6.9, 9.6, 11.9, 12.4, 17.0, 21.2, 22.7, 25.0 and 29.1; typically, by an X-ray powder diffraction pattern which comprises at least peaks at 2θ (± 0.2°) of 6.9, 9.6, 11.9, 12.4, 17.0, 19.9, 21.2, 22.7, 25.0, 25.9, 27.2 and 29.1; more typically, by substantially the same X-ray powder diffraction pattern shown in Figure 38. In certain embodiments, provided herein is a crystalline form of compound (I), HCl Salt Type B, characterized by a differential scanning calorimeter peak phase transition temperature of about 241.7 °C.

### Mesylate Type A

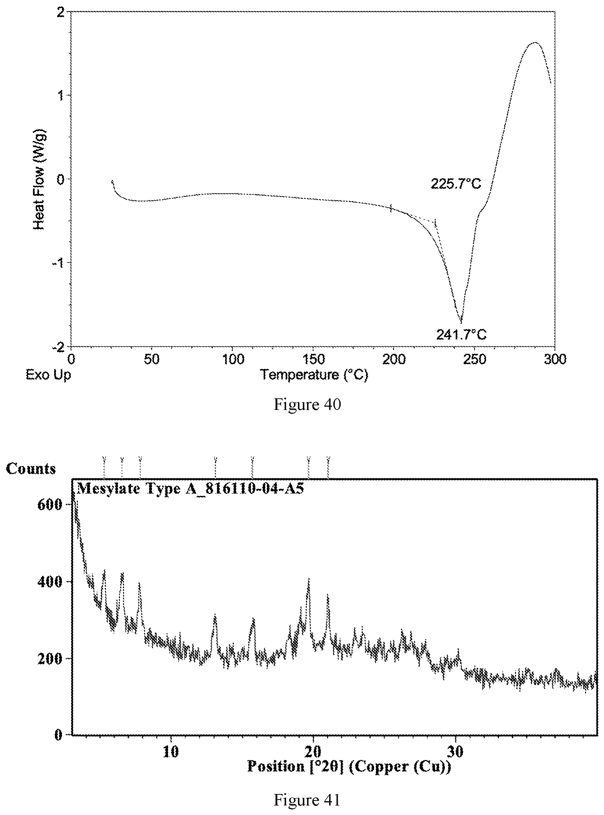

In certain embodiments, provided herein is a crystalline form of compound (I), Mesylate Type A, characterized by an X-ray powder diffraction which comprises at least peaks at 2θ (± 0.2°) of 6.5, 19.6 and 21.0; typically, by an X-ray powder diffraction which comprises at least peaks at 2θ (± 0.2°) of 5.3, 6.5, 7.8, 13.1, 15.7, 19.6 and 21.0; more typically, by substantially the same X-ray powder diffraction pattern shown in Figure 41. In certain embodiments, provided herein is a crystalline form of compound (I), Mesylate Type A, characterized by a differential scanning calorimeter peak phase transition temperature of about 65.1 °C.

### Mesylate Type B

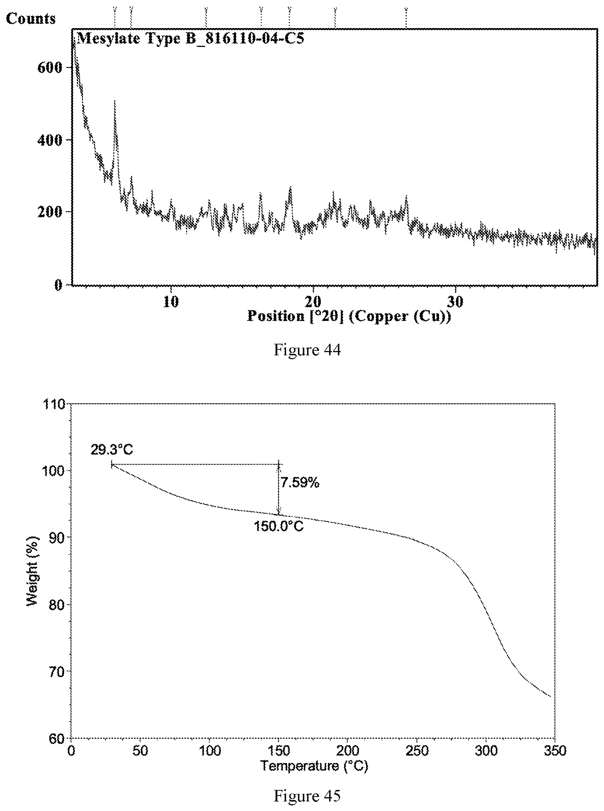

In certain embodiments, provided herein is a crystalline form of compound (I), Mesylate Type B, characterized by an X-ray powder diffraction which comprises at least peaks at 2θ (± 0.2°) of 6.0, 16.3 and 18.3; typically, by an X-ray powder diffraction which comprises at least peaks at 2θ (± 0.2°) of 6.0, 7.2, 12.4, 16.3, 18.3, 21.5 and 26.5; more typically, by substantially the same X-ray powder diffraction pattern shown in Figure 44. In certain embodiments, provided herein is a crystalline form of compound (I), Mesylate Type B, characterized by a differential scanning calorimeter peak phase transition temperature of about 63.4 °C.

### Phosphate Type A

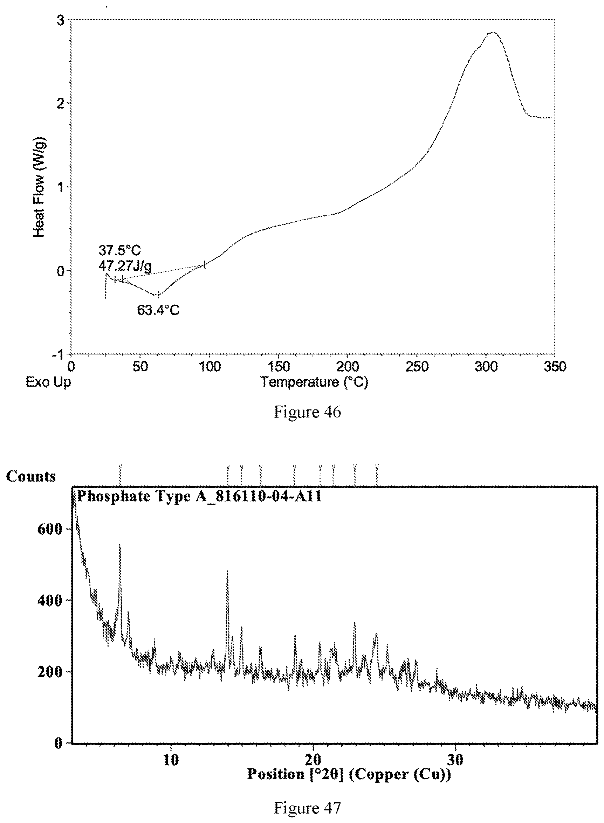

In certain embodiments, provided herein is a crystalline form of compound (I), Phosphate Type A, characterized by an X-ray powder diffraction which comprises at least peaks at 2θ (± 0.2°) of 6.4, 14.0 and 22.9; typically, by an X-ray powder diffraction which comprises at least peaks at 2θ (± 0.2°) of 6.4, 14.0, 14.9, 20.5, 22.9 and 24.5; more typically, by an X-ray powder diffraction which comprises at least peaks at 2θ (± 0.2°) of 6.4, 14.0, 14.9, 16.3, 18.7, 20.5, 21.4, 22.9 and 24.5; even more typically, by substantially the same X-ray powder diffraction pattern shown in Figure 47. In certain embodiments, provided herein is a crystalline form of compound (I), Phosphate Type A, characterized by a differential scanning calorimeter peak phase transition temperature of about 79.1°C and about 194.8 °C.

### L-tartrate Type A

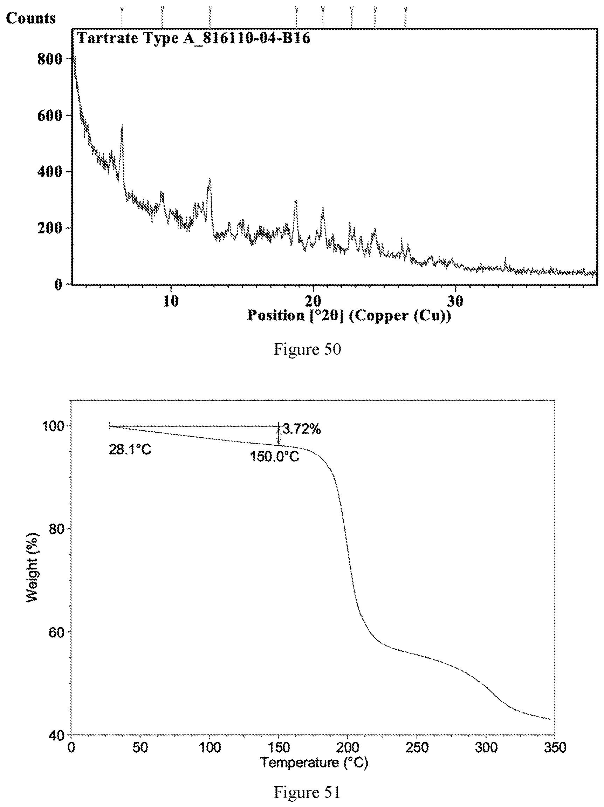

In certain embodiments, provided herein is a crystalline form of compound (I), L-tartrate Type A, characterized by an X-ray powder diffraction which comprises at least peaks at 2θ (± 0.2°) of 6.5, 12.7 and 18.8; typically, by an X-ray powder diffraction which comprises at least peaks at 2θ (± 0.2°) of 6.5, 9.4, 12.7, 18.8, 20.7, 22.7, 24.4 and 26.5; more typically, by substantially the same X-ray powder diffraction pattern shown in Figure 50. In certain embodiments, provided herein is a crystalline form of compound (I), L-tartrate Type A, characterized by a differential scanning calorimeter peak phase transition temperature of about 77.6 °C and about 164.7 °C.

### Adipate Type A

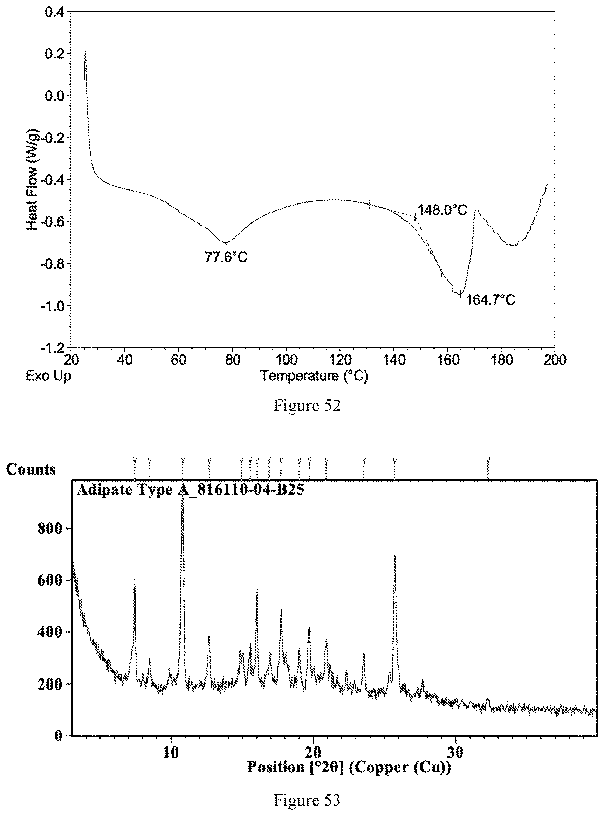

In certain embodiments, provided herein is a crystalline form of compound (I), Adipate Type A, characterized by an X-ray powder diffraction which comprises at least peaks at 2θ (± 0.2°) of 7.4, 10.8 and 25.7; typically, by an X-ray powder diffraction which comprises at least peaks at 2θ (± 0.2°) of 7.4, 10.8, 16.0, 17.7, 19.7 and 25.7; more typically, by an X-ray powder diffraction which comprises at least peaks at 2θ (± 0.2°) of 7.4, 10.8, 12.6, 16.0, 17.7, 19.7, 20.9, 23.6 and 25.7; more typically, by an X-ray powder diffraction which comprises at least peaks at 2θ (± 0.2°) of 7.4, 8.5, 10.8, 12.6, 14.9, 15.5, 16.0, 16.9, 17.7, 19.0, 19.7, 20.9, 23.6, 25.7 and 32.3; even more typically, by substantially the same X-ray powder diffraction pattern shown in Figure 53. In certain embodiments, provided herein is a crystalline form of compound (I), Adipate Type A, characterized by a differential scanning calorimeter peak phase transition temperature of about 106.7°C.

In another aspect, there is provided herein an amorphous form of compound (I) represented by the following structural formula: 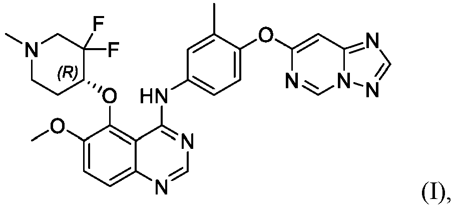 wherein the amorphous form is a pharmaceutically acceptable salt or free base.

### Amorphous Fumarate

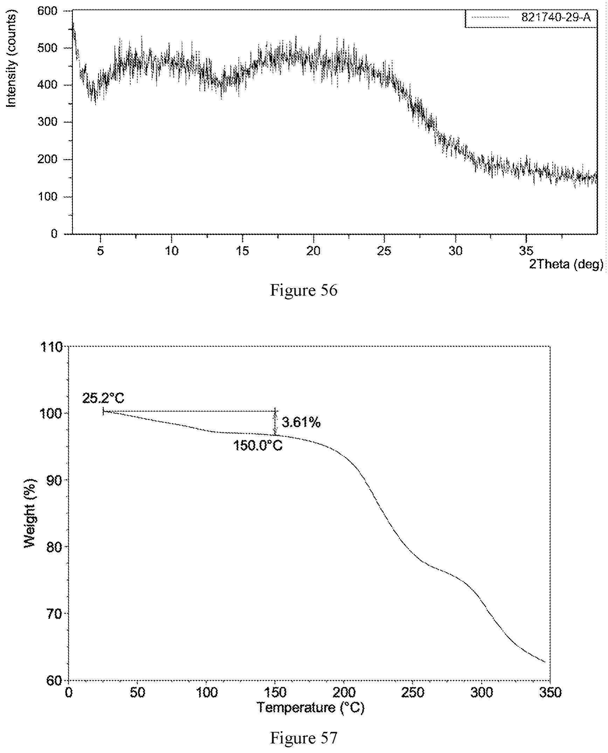

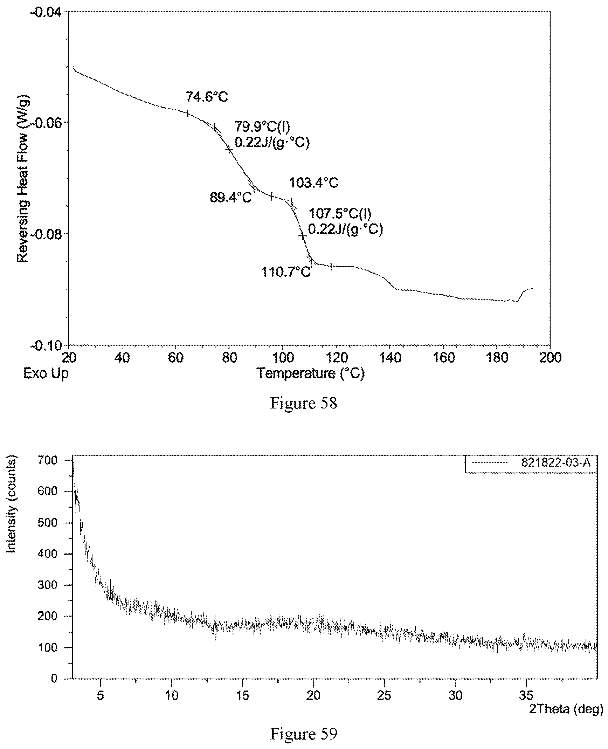

In certain embodiments, provided herein is an amorphous form of compound (I), Amorphous Fumarate, characterized by substantially the same X-ray powder diffraction pattern as Figure 56. In certain embodiments, provided herein is an amorphous form of compound (I), Amorphous Fumarate, characterized by substantially the same differential scanning calorimeter thermogram curve as Figure 58.

### Amorphous Freebase

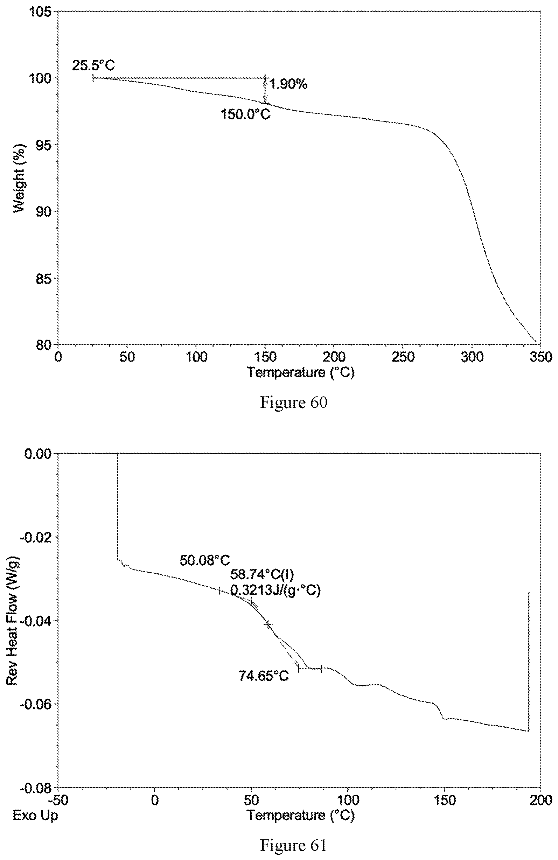

In certain embodiments, provided herein is an amorphous form of compound (I), Amorphous Freebase, characterized by substantially the same X-ray powder diffraction pattern as Figure 59. In certain embodiments, provided herein is an amorphous form of compound (I), Amorphous Freebase, characterized by substantially the same differential scanning calorimeter thermogram curve as Figure 61.

In certain embodiments, the crystalline form or amorphous form of compound (I) described herein may be provided in a substantially pure form. Specifically, the crystalline forms of compound (I), particularly those designated as Fumarate Type A, Fumarate Type B, Fumarate Type C, Fumarate Type E, Freebase Type A, Freebase Type B, Freebase Type C, Freebase Type D, Freebase Type E, Freebase Type F, Freebase Type G, HCl Salt Type A, HCl Salt Type B, Mesylate Type A, Mesylate Type B, Phosphate Type A, L-tartrate Type A and Adipate Type A, and the amorphous forms of compound (I), particularly those designated as Amorphous Fumarate and Amorphous Freebase, may exist in a substantially pure form.

As used herein, the term "substantially pure" means the polymorphic form or amorphous material includes less than about 15% by weight of impurities, including other polymorphic forms. In certain embodiments, the substantially pure polymorphic form or amorphous material includes less than about 10% by weight of impurities, including other polymorphic forms. In certain embodiments, the substantially pure polymorphic form or amorphous material includes less than about 5% by weight of impurities, including other polymorphic forms. In certain embodiments, the substantially pure polymorphic form or amorphous material includes less than about 1% by weight of impurities, including other polymorphic forms. In certain embodiments, the substantially pure polymorphic form or amorphous material does not include impurities, including other polymorphic forms.

The present invention also includes crystalline forms and amorphous forms of isotopically-labeled (R)-N-(4-([1,2,4]triazolo[1,5-c]pyrimidin-7-yloxy)-3-methylphenyl)-5-((3,3-difluoro-1-methylpiperidin-4-yl)oxy)-6-methoxyquinazolin-4-amine (compound (I)) which is identical, but for the fact that one or more atoms are replaced by an atom having an atomic mass or mass number different from the atomic mass or mass number usually found in nature. Examples of isotopes that can be incorporated into the compound of the present invention include isotopes of hydrogen, carbon, nitrogen and oxygen, such as ²H, ³H, ¹³C, ¹⁴C, ¹⁵N, ¹⁷O and ¹⁸O, respectively. The polymorphs described herein, which contain the aforementioned isotopes and/or other isotopes of other atoms are within the scope of this invention. Certain isotopically-labeled compound of the present invention, for example those into which radioactive isotopes, such as ³H and ¹⁴C are incorporated, are useful in drug and/or substrate tissue distribution assays. Tritiated, *i.e.,* ³H and carbon-14, *i.e.,* ¹⁴C, isotopes are particularly widely used as a result of their ease of preparation and detectability. Further, substitution with heavier isotopes such as deuterium, i.e., ²H, can afford certain therapeutic advantages resulting from greater metabolic stability, for example increased in vivo half-life or reduced dosage requirements and, hence, may be utilized in some particular circumstances. Isotopically labeled salts of the present invention can generally be prepared by carrying out procedures disclosed in WO 2020/057511A1 by substituting a readily available isotopically labeled reagent for a non-isotopically labeled reagent during the preparation, or if desired, using an isotopically labeled sulfuric acid in the preparation of the salt.

### SYNTHESIS OF COMPOUND

The compound described herein may be synthesized by means of synthetic routes that include processes analogous to those well-known in the chemical arts, particularly in light of the description contained herein. The starting materials are generally available from commercial sources such as Sigma-Aldrich (St. Louis, MO), Alfa Aesar (Ward Hill, MA), or TCI (Portland, OR), or are readily prepared using methods well known to those skilled in the art (e.g., prepared by methods generally described in Louis F. Fieser and Mary Fieser, Reagents for Organic Synthesis, v. 1-23, New York: Wiley 1967-2006 ed. (also available via the Wiley InterScience® website), or Beilsteins Handbuch der organischen Chemie, 4, Aufl. ed. Springer-Verlag, Berlin, including supplements (also available via the Beilstein online database)).

Generally, compound (I) may be prepared by coupling 4-chloro-5-((3,3-difluoro-1-methylpiperidin-4-yl)oxy)-6-methoxyquinazoline with 4-([1,2,4]triazolo[1,5-c]pyrimidin-7-yloxy)-3-methylaniline. The coupling may be carried out, for example, in a solvent, e.g., propan-2-ol, under the catalyzation of toluene sulfonic acid TsOH·H₂O at 100°C followed by separating the two isomers of the racemic product by chiral Supercritical Fluid Chromatography (SFC). More detailed description of the individual reaction steps may be found, for the illustration purpose, in the Examples section of WO 2020/057511 A1, which is incorporated by reference in its entirety. Those skilled in the art will appreciate that other synthetic routes may be used to synthesize the compound.

The final crystallizations or isolations will determine the polymorphic form, which are further detailed in the Examples section herein.

### PREPARATION OF CRYSTALLINE FORMS

In one aspect, there is provided herein a method for the preparation of the crystalline form of compound (I), comprising:
where the crystalline form is a complex of free base with a pharmaceutically acceptable acid,
   a) adding compound (I) and the acid in a solvent or solvent, and
   b) slurrying at a temperature for a time sufficient to initiate precipitation of the complex;
where the crystalline form is free base,
   a) adding compound (I) in a solvent; and
   b) slurrying at a temperature for a time sufficient to initiate precipitation of the free base.

In certain embodiments, the acid is selected from a group consisting of hydrochloride, methane sulfonic acid, phosphoric acid, tartaric acid, fumaric acid and adipic acid.

In certain embodiments, in step a), where the crystalline form is a complex of free base with a pharmaceutically acceptable acid, the compound (I) and the acid are added in a solvent at an acid/base molar ratio in a range of about 0.5:1 to about 3:1, preferably, about 0.5:1 to about 2.5:1, more typically, about 1:1 to about 1.5:1, For example, the salt described herein may have an acid/base molar ratio of about 0.5:1, about 0.6:1, about 0.7:1, about 0.8:1, about 0.9:1, about 1:1, about 1.1:1, about 1.2:1, about 1.3:1, about 1.4:1, about 1.5:1, about 1.6:1, about 1.7:1, about 1.8:1, about 1.9:1, about 2:1, about 2.1:1, about 2.2:1, about 2.3:1, about 2.4:1 and about 2.5:1.

In certain embodiments, the solvent selected from a group consisting H₂O, EtOH, EtOAc, n-heptane, ethyl formate, acetone, cyclohexane, isopropyl alcohol, methyl isobutyl ketone, tetrahydrofuran, acetonitrile and methyl tert-butyl ether and combination thereof.

In certain embodiments, the method described herein further comprises seeding the solvent with a crystalline form described herein.

In certain embodiments, in step b), the temperature is about 5~50 °C.

In certain embodiments, in step b), the time is about 2~7.5 hours.

In certain embodiments, there is provided herein a method for the preparation of Fumarate Type A crystalline form of compound (I) described herein, comprising:
a) dissolving free base of compound (I) in an ester or an alcohol to form a free base solution;
b) dissolving fumaric acid in EtOH to form an acid solution;
c) adding the acid solution into the free base solution dropwise while stirring;
d) adding an alkane dropwise; then optionally seeding the mixture with a Fumarate Type A crystalline form of compound (I);
e) stirring at 0-10 °C for 12-24 hours; and
f) isolating a solid via filtration and then vacuum drying the solid at 40-60 °C.

In certain embodiments, the ester is selected from a group consisting of ethyl acetate, ethyl formate, methyl acetate and isopropyl acetate.

In certain embodiments, the ester is ethyl acetate.

In certain embodiments, the alcohol is selected from a group consisting of methanol, ethanol, n-propanol and isopropanol.

In certain embodiments, the alcohol is ethanol.

In certain embodiments, the alkane is selected from a group consisting of n-hexane, n-heptane, n-octane and a mixture thereof.

In certain embodiments, the alkane is n-heptane.

In certain embodiments, there is provided herein a method for the preparation of Fumarate Type B crystalline form of compound (I) described herein, comprising:
a) suspending a Fumarate Type A crystalline form of compound (I) in H₂O;
b) magnetically stirring at a speed of about 1000 rpm at about room temperature for about 11 days; and
c) isolating a solid via centrifugation and storing the solid at ambient conditions openly for about 4 days.

In certain embodiments, there is provided herein a method for the preparation of Fumarate Type C crystalline form of compound (I) described herein, comprising:
a) suspending a Fumarate Type A crystalline form of compound (I) in H₂O;
b) magnetically stirring at a speed of about 1000 rpm at about room temperature for about 9 days; and
c) isolating a wet solid.

In certain embodiments, there is provided herein a method for the preparation of Fumarate Type E crystalline form of compound (I) described herein, comprising:
a) dissolving a Fumarate Type crystalline form of compound (I) in ethyl formate;
b) evaporating the ethyl formate at about room temperature;
c) isolating a solid.

In certain embodiments, there is provided herein a method for the preparation of Freebase Type A crystalline form of compound (I) described herein, comprising:
a) suspending amorphous free base of compound (I) in a solvent of acetone/n-heptane with a volume ratio of about 1:4;
b) magnetically stirring at a speed of about 1000 rpm at about room temperature for about 3 days; and
c) isolating a solid via centrifugation and drying the solid at ambient conditions for about 1 day.

In certain embodiments, there is provided herein a method for the preparation of Freebase Type B crystalline form of compound (I) described herein, comprising:
a) suspending amorphous free base of compound (I) in a solvent of methyl isobutyl ketone/Cyclohexane with a volume ratio of about 1:4;
b) magnetically stirring at a speed of about 1000 rpm at about 5 °C for about 7 days; and
c) isolating a solid via centrifugation.

In certain embodiments, there is provided herein a method for the preparation of Freebase Type C crystalline form of compound (I) described herein, comprising:
a) suspending amorphous free base of compound (I) in a solvent of tetrahydrofuran/H₂O with a volume ratio of about 1:4;
b) magnetically stirring at a speed of about 1000 rpm at about 5 °C for about 7 days; and
c) isolating a solid via centrifugation.

In certain embodiments, there is provided herein a method for the preparation of Freebase Type D crystalline form of compound (I) described herein, comprising:
a) suspending amorphous free base of compound (I) in a solvent of tetrahydrofuran/H₂O with a volume ratio of about 1:4;
b) magnetically stirring at a speed of about 1000 rpm at about 5 °C for about 3 days; and
c) isolating a solid via centrifugation and drying the solid at ambient condition for about 2 hours.

In certain embodiments, there is provided herein a method for the preparation of Freebase Type E crystalline form of compound (I) described herein, comprising:
a) suspending amorphous free base of compound (I) in a solvent of tetrahydrofuran/H₂O with a volume ratio of about 1:4;
b) magnetically stirring at a speed of about 1000 rpm at about 5 °C for about 4 days;
c) isolating a solid via centrifugation and drying the solid at ambient condition for about 2 hours; and
d) sweeping the solid by N₂ for about 20 min at about 30 °C.

In certain embodiments, there is provided herein a method for the preparation of Freebase Type F crystalline form of compound (I) described herein, comprising:
a) suspending amorphous free base of compound (I) in a solvent of acetonitrile/n-heptane;
b) magnetically stirring at a speed of about 1000 rpm at about room temperature for about 2 days;
c) isolating a solid.

In certain embodiments, there is provided herein a method for the preparation of Freebase Type G crystalline form of compound (I) described herein, comprising:
a) dissolving amorphous free base of compound (I) in EtOH;
b) adding H₂O and obtaining a suspension;
c) isolating a solid from the suspension.

In certain embodiments, there is provided herein a method for the preparation of HCl salt Type A of compound (I) described herein, comprising:
a) adding amorphous free base of compound (I) and a concentrated HCl at an acid/base molar ratio of about 2:1 in a solvent of EtOAc/n-heptane with a volume ratio of about 1:2;
b) magnetically stirring at a speed of about 1000 rpm at about room temperature for about 3 days; and
c) isolating a solid via centrifugation and drying the solid at ambient conditions for about 1 day.

In certain embodiments, there is provided herein a method for the preparation of HCl salt Type B of compound (I) described herein, comprising:
a) dissolving amorphous free base of compound (I) in EtOAc to form a free base solution;
b) diluting a EtOAc solution of HCl in EtOH to form an acid solution;
c) optionally adding a seed of HCl salt Type B of compound (I) into the free base solution wherein the seed is not completely dissolved;
d) adding the acid solution dropwise while stirring at a speed of about 1000 rpm;
e) further stirring at room temperature for about 8 hours, then about 5 °C for about 13 hours;
f) isolating a solid via filtration, then vacuum drying the solid at about room temperature overnight;
wherein the molar ratio of acid/base is about 2:1.

In certain embodiments, there is provided herein a method for the preparation of Mesylate Type A of compound (I) described herein, comprising:
a) suspending amorphous free base of compound (I) and a methane sulfonic acid at an acid/base molar ratio of about 2:1 in a solvent of acetone/n-heptane with a volume ratio of about 1:4;
b) magnetically stirring at a speed of about 1000 rpm at about room temperature for about 3 days; and
c) isolating a solid via centrifugation and drying the solid at ambient conditions for about 1 day.

In certain embodiments, there is provided herein a method for the preparation of Mesylate Type B of compound (I) described herein, comprising:
a) suspending amorphous free base of compound (I) and a methanesulfonic acid at an acid/base charging molar ratio of about 2:1 in a solvent of isopropyl alcohol/cyclohexane with a volume ratio of about 1:4;
b) magnetically stirring at a speed of about 1000 rpm at about room temperature for about 3 days; and
c) isolating a solid via centrifugation and drying the solid at ambient conditions for about 1 day.

In certain embodiments, there is provided herein a method for the preparation of Phosphate Type A of compound (I) described herein, comprising:
a) suspending amorphous free base of compound (I) and concentrated H₃PO₄ at an acid/base molar ratio of about 1:1 in a solvent of acetone/n-heptane with a volume ratio of about 1:4;
b) magnetically stirring at a speed of about 1000 rpm at room temperature for about 3 days; and
c) isolating a solid via centrifugation and drying the solid at ambient conditions for about 1 day.

In certain embodiments, there is provided herein a method for the preparation of L-tartrate Type A of compound (I) described herein, comprising:
a) suspending amorphous free base of compound (I) and a L-tartaric acid at an acid/base molar ratio of about 1:1 in a solvent of EtOAc/n-heptane with a volume ratio of about 1:2;
b) magnetically stirring at a speed of about 1000 rpm at about room temperature for about 3 days; and
c) isolating a solid via centrifugation and drying the solid at ambient conditions for about 1 day.

In certain embodiments, there is provided herein a method for the preparation of Adipate Type A of compound (I) described herein, comprising:
a) suspending amorphous free base of compound (I) and an adipic acid at an acid/base molar ratio of about 1:1 in a solvent of EtOAc/n-heptane with a volume ratio of about 1:2;
b) magnetically stirring at a speed of about 1000 rpm at about room temperature for about 3 days; and
c) isolating a solid via centrifugation and drying the solid at ambient conditions for about 1 day.

Moreover, in another aspect, there is provided herein a method for the preparation of an amorphous form of compound (I) described herein, comprising:
a) dissolving compound (I) in a solvent; and
b) removing the solvent.

In certain embodiments, the solvent is selected from a group consisting of H₂O, MeOH, EtOH, EtOAc, DCM, n-heptane, ethyl formate, acetone, cyclohexane, isopropyl alcohol, methyl isobutyl ketone, tetrahydrofuran, acetonitrile, methyl tert-butyl ether and combination thereof.

In certain embodiments, there is provided herein a method for the preparation of Amorphous Fumarate of compound (I) described herein, comprising:
a) dissolving Fumarate Type A of compound (I) in MeOH; and
b) removing the MeOH by rotatory evaporation at about 60 °C.

In certain embodiments, there is provided herein a method for the preparation of Amorphous Freebase of compound (I) described herein, comprising:
a) dissolving Freebase Type B of compound (I) in DCM; and
b) removing the DCM by rotatory evaporation at about 40 °C.

As used herein, the term "seeding" or "adding a seed" refers to the addition of crystalline material to a solution or mixture to initiate crystallisation or recrystallisation.

### USAGE

The crystalline form and amorphous form of compound (I) as described herein may show high inhibitory activity against type I receptor tyrosine kinase, in particular HER2.

As used herein, the term "inhibitory activity against type I receptor tyrosine kinase" refers to a decrease in the activity of type I receptor tyrosine kinase as a direct or indirect response to the presence of the crystalline form or amorphous form of compound (I), relative to the activity of type I receptor tyrosine kinase in the absence of the crystalline form or amorphous form of compound (I). Such a decrease in activity may be due to the direct interaction of the crystalline form or amorphous form of compound (I) with type I receptor tyrosine kinase, or due to the interaction of the crystalline form or amorphous form of compound (I) with one or more other factors that in turn affect activity of type I receptor tyrosine kinase. For example, the crystalline form or amorphous form of compound (I) as described herein may decrease activity of type I receptor tyrosine kinase by directly binding to the type I receptor tyrosine kinase, by causing (directly or indirectly) another factor to decrease type I receptor tyrosine kinase activity, or by (directly or indirectly) decreasing the amount of type I receptor tyrosine kinase present in the cell or organism.

As a result of their inhibitory activity against type I receptor tyrosine kinase, the crystalline form and amorphous form of compound (I) are useful in therapy, for example in the treatment of diseases or medical conditions mediated at least in part by one or more type I receptor tyrosine kinases, including cancer.

As used herein, the term "cancer" refers to or describe the physiological condition in mammals that is typically characterized by abnormal or unregulated cell growth. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, brain, endometrial or uterine carcinoma, salivary gland carcinoma, kidney or renal cancer, prostate cancer, vulval cancer, thyroid cancer, hepatic carcinoma, anal carcinoma, penile carcinoma, skin cancer, including melanoma, as well as head and neck cancer. Typically, such cancer include breast, gastric, biliary, colorectal, brain, lung, NSCLC, pancreatic, head and neck, ovarian and uterine cancer. As used herein, the term "cancer" is intended to encompass both non-metastatic cancer and metastatic cancer. In this context, treating cancer involves treatment of both primary tumors and tumor metastases.

As used herein, the term "mammal" means a warm-blooded animal that has or is at risk of developing a disease described herein and includes, but is not limited to, guinea pigs, dogs, cats, rats, mice, hamsters, and primates, including humans.

As used herein, the term "therapy" is intended to have its normal meaning of dealing with a disease in order to entirely or partially relieve one, some or all of its symptoms, or to correct or compensate for the underlying pathology, thereby achieving beneficial or desired clinical results. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Therapy" can also mean prolonging survival as compared to expected survival if not receiving it. Those in need of therapy include those already with the condition or disorder as well as those prone to have the condition or disorder or those in which the condition or disorder is to be prevented. The term "therapy" also encompasses prophylaxis unless there are specific indications to the contrary. The terms "therapeutic" and "therapeutically" should be interpreted in a corresponding manner.

As used herein, the term "prophylaxis" is intended to have its normal meaning and includes primary prophylaxis to prevent the development of the disease and secondary prophylaxis whereby the disease has already developed and the patient is temporarily or permanently protected against exacerbation or worsening of the disease or the development of new symptoms associated with the disease.

The term "treatment" is used synonymously with "therapy". Similarly, the term "treat" can be regarded as "applying therapy" where "therapy" is as defined herein.

In some embodiments, the compounds of the present invention possess anti-cell-proliferation properties, which are believed to arise from their type I receptor tyrosine kinase inhibitory activity. Accordingly, the compounds of the present invention are expected to be useful in the treatment of diseases or conditions mediated alone or in part by type I receptor tyrosine kinases, i.e. the compounds may be used to produce an anti-proliferative effect mediated alone or in part by inhibiting type I receptor tyrosine kinases. In some embodiments, such disease or condition treated by providing an anti-proliferative effect is type I receptor tyrosine kinase sensitive cancers, including but not limited to breast cancer, lung cancer, colon cancer, rectum cancer, stomach cancer, prostate cancer, bladder cancer, pancreas cancer and ovary cancer, or other cell-proliferation diseases such as psoriasis.

Therefore, in one aspect, there is provided a crystalline form or amorphous form of compound (I) described herein for use in therapy. In some embodiments, there is provided a crystalline forms and amorphous forms of compound (I) described herein for use as a medicament. In some embodiments, the present invention provides crystalline forms and amorphous forms of compound (I) described herein for use in the treatment of diseases or conditions mediated alone or in part by type I receptor tyrosine kinases. In some embodiments, the present invention provides crystalline forms and amorphous forms of compound (I) described herein for use in treating or ameliorating a hyperproliferative disease, typically a cancer, more typically a ErbB2 positive cancer.

In another aspect, there is provided a crystalline form or amorphous form of compound (I) described herein for use in the manufacture of a medicament for the treatment of type I receptor tyrosine kinase-associated diseases or conditions, preferably ErbB2-associated diseases or conditions. In some embodiments, there is provided a crystalline form or amorphous form of compound (I) described herein for use in the manufacture of a medicament for treating or ameliorating a hyperproliferative disease, typically a cancer, more typically a ErbB2 positive cancer.

### PHARMACEUTICAL COMPOSITION/DOSAGE FORM

The crystalline form and amorphous form of compound (I) described herein may be administered by any convenient route appropriate to the condition to be treated. Suitable routes include oral, parenteral (including subcutaneous, intramuscular, intravenous, intraarterial, intradermal, intrathecal and epidural), transdermal, rectal, nasal, topical (including buccal and sublingual), ocular, vaginal, intraperitoneal, intrapulmonary and intranasal.

The crystalline form and the amorphous form of compound (I) described herein may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents. For example, if parenteral administration is desired, the compositions will be sterile and be in a solution or suspension form suitable for injection or infusion.

A typical formulation is prepared by mixing the crystalline form and the amorphous form of compound (I) described herein and a pharmaceutically acceptable carrier or excipient.

As used herein, the term "pharmaceutically acceptable carrier or excipient" means a carrier or excipient that is useful in preparing a pharmaceutical composition that is generally safe, non-toxic and neither biologically nor otherwise undesirable, and includes carrier or excipient that is acceptable for veterinary use as well as human pharmaceutical use. A "pharmaceutically acceptable carrier or excipient" as used in the specification and claims includes both one and more than one such carrier or excipient. The particular excipient, carrier, or diluent or used will depend upon the means and purpose for which the compounds of the present invention is being applied. Suitable carriers and excipients are well known to those skilled in the art and are described in detail in, e.g., Ansel, Howard C, et al., Ansel's Pharmaceutical Dosage Forms and Drug Delivery Systems. Philadelphia: Lippincott, Williams & Wilkins, 2004; Gennaro, Alfonso R., et al., Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a crystalline form and amorphous form of compound (I) described herein or pharmaceutical composition thereof) or aid in the manufacturing of the pharmaceutical product (i.e., medicament).

Therefore, in one aspect, there is provided a pharmaceutical composition comprising a crystalline form or amorphous form of compound (I) described herein, as an active ingredient. In certain embodiments, there is provided a pharmaceutical composition comprising a crystalline form or amorphous form of compound (I) described herein, together with a pharmaceutically acceptable carrier or excipient.

The pharmaceutical composition described herein may be formulated into a dosage form comprising a therapeutically effective amount of the crystalline form, the amorphous form, or the pharmaceutical composition described herein.

As used herein, the term "therapeutically effective amount" refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; the rate of administration; the therapeutic or combination of therapeutics selected for administration; and the discretion of the prescribing physician. Therapeutically effective amounts for a given situation can be determined by routine experimentation that is within the skill and judgment of the clinician.

In some embodiments, the pharmaceutical compositions can be formulated so that a dosage of between 0.001-500 mg/kg body weight/day, for example, 0.01-400 mg/kg body weight/day, 0.01-300 mg/kg body weight/day, 0.1-200 mg/kg body weight/day, 0.1-150 mg/kg body weight/day, 0.1-100 mg/kg body weight/day, 0.5-100 mg/kg body weight/day, 0.5-80 mg/kg body weight/day, 0.5-60 mg/kg body weight/day, 0.5-50 mg/kg body weight/day, 1-50 mg/kg body weight/day, 1-40 mg/kg body weight/day of the crystalline form or amorphous form of compound (I) described herein, can be administered. In some instances, dosage levels below the lower limit of the aforesaid range may be more than adequate, while in other cases still larger doses may be employed without causing any harmful side effect, provided that such larger doses are first divided into several small doses for administration throughout the day. For further information on routes of administration and dosage regimes, see Chapter 25.3 in Volume 5 of Comprehensive Medicinal Chemistry (Corwin Hansch; Chairman of Editorial Board), Pergamon Press 1990, which is specifically incorporated herein by reference.

### METHODS OF TREATMENT

In a further aspect, there is provided a method of treating or ameliorating a hyperproliferative disease in a subject in need thereof, comprising administering to the subject a therapeutically effective amount of the crystalline form or amorphous form described herein, owning to the type I receptor tyrosine kinase inhibitory activity, and brain penetration capability of the compound of the present invention.

As used herein, the term "subject in need thereof" is a subject having a type I receptor tyrosine kinase-associated disease or condition (e.g., cancer), or a subject having an increased risk of developing a type I receptor tyrosine kinase-associated disease or condition (e.g., cancer) relative to the population at large. In the case of cancer, a subject in need thereof can have a precancerous condition. The term "subject" includes a warm-blooded animal. In some embodiments, the warm-blooded animal is a mammal. In some embodiments, the warm-blooded animal is a human.

In certain embodiments, the hyperproliferative disease is cancer.

In certain embodiments, the cancer is ErbB2 positive.

In certain embodiments, the cancer is selected from a group consisting of breast, gastric, biliary, colorectal, brain, lung, NSCLC, pancreatic, head and neck, ovarian and uterine cancer.

In certain embodiments, one or more additional compounds having anti-cancer properties are administered in combination.

### COMBINATION THERAPY

The crystalline form, the amorphous form and the pharmaceutical composition described herein may be employed alone or in combination with an additional therapeutic agent for treatment. Useful additional therapeutic agents include but not limited to an anti-tumor agent, e.g., an additional compound having anti-cancer properties. The additional therapeutic agent of the pharmaceutical combination formulation or dosing regimen preferably has complementary activities to the compound described herein, such that they do not adversely affect each other. Such molecules are suitably present in combination in amounts that are effective for the purpose intended.

As used herein, the term "combination" refers to simultaneous, separate or sequential administration. In some embodiments, "combination" refers to simultaneous administration. In some embodiments, "combination" refers to separate administration. In some embodiments, "combination" refers to sequential administration. Where the administration is sequential or separate, the delay in administering the second component should not be such as to lose the beneficial effect of the combination.

Illustrative examples of useful anti-tumor agent can be selected from the following categories:
(i) antiproliferative/anti-neoplastic drugs and combinations thereof, such as TKIs; DNA alkylating agents; antimetabolites; anti-tumor antibiotics; antimitotic agents; and topoisomerase inhibitors; inhibitors of DNA repair mechanisms such as CHK kinase; DNA-dependent protein kinase inhibitors; inhibitors of poly (ADP-ribose) polymerase (PARP inhibitors); and Hsp90 inhibitors such as tanespimycin and retaspimycin, inhibitors of ATR kinase; and inhibitors of WEE 1 kinase;
(ii) cytostatic agents such as antiestrogens, estrogen receptor down regulators; antiandrogens; LHRH antagonists or LHRH agonists; progestogens; aromatase inhibitors; inhibitors of 5α-reductase; and p38 inhibitors;
(iii) agents which inhibit cancer cell invasion;
(iv) inhibitors of growth factor function such as growth factor antibodies, growth factor receptor antibodies, antibody drug conjugates, farnesyl transferase inhibitors, tyrosine kinase inhibitors and serine-threonine kinase inhibitors; inhibitors of the platelet-derived growth factor family; inhibitors of the hepatocyte growth factor family; and MEK inhibitors and compounds such as those disclosed in U.S. Patent Publication 2004/0116710;
(v) antiangiogenic agents such as those which inhibit the effects of vascular endothelial growth factor, such as but not limited to, the anti-vascular endothelial cell growth factor antibody bevacizumab, a VEGF receptor tyrosine kinase inhibitor; compounds such as those disclosed in International Patent Applications WO 97/22596, WO 97/30035, WO 97/32856 and WO 98/13354; and compounds that work by other mechanisms, or inhibitors of angiopoietins and their receptors (Tie-1 and Tie-2), inhibitors of PLGF, inhibitors of delta-like ligand (DLL-4);
(vi) vascular damaging agents;
(vii) antisense therapies;
(viii) gene therapy approaches, including for example GVAX^{™}, approaches to replace aberrant genes such as aberrant p53 or aberrant BRCAI or BRCA2, GDEPT (gene-directed enzyme pro-drug therapy) approaches and approaches to increase patient tolerance to chemotherapy or radiotherapy such as multi-drug resistance gene therapy;
(ix) interferon;
(x) immunotherapy approaches, including, but not limited to, ex-vivo and in-vivo approaches to increase the immunogenicity of patient tumor cells; approaches to decrease T-cell anergy or regulatory T-cell function; approaches that enhance T-cell responses to tumors; approaches using transfected immune cells; approaches using cytokine-transfected tumor cell lines, approaches using antibodies to tumor associated antigens, and antibodies that deplete target cell types; approaches using anti-idiotypic antibodies; approaches that enhance Natural Killer cell function; and approaches that utilize antibody-toxin conjugates; immunotoxins; agonists of toll-like receptor 7 or toll-like receptor 9;
(xi) efficacy enhancers, such as leucovorin.

For the illustrative purpose, more detailed examples of such combination therapy can be found in WO 2020/057511 A1, which is incorporated by reference in its entirety.

In certain embodiment, the additional anti-tumor agent (additional compound having anti-cancer properties) is selected from the group consisting of TKIs (such as lapatinib, neratinib and afatinib), anti-HER2 agents (for example, monoclonal antibodies such as Trastuzumab, ADCs such as T-DM1, T-DXd) and combination thereof. In some embodiments, the additional anti-tumor agent includes capecitabine, anti-HER2 antibodies, T-DXd and T-DM1. In some embodiments, there is one additional anti-tumor agent. In some embodiments, there are two additional anti-tumor agents. In some embodiments, there are three or more additional anti-tumor agents.

### SALT SCREENING AND EVALUATION

Using Amorphous Freebase of compound (I) or Freebase Type B as the starting material, salt screening experiments were performed using diverse acids or co-formers in different solvent systems. HCl, methane sulfonic acid and *p*-toluene sulfonic acid were tested with 2 charging ratios. Seven crystalline salts, HCl salt Type A, HCl salt Type B, Mesylate Type A, Mesylate Type B, Phosphate Type A, L-tartrate Type A, Fumarate Type A and Adipate Type A, were obtained. Besides, one crystalline free base, Freebase Type A, was observed in the screening.

### POLYMORPH STUDY

Amorphous free base, Freebase Type B and Freebase Type G were used as the starting materials for polymorph screening of free base of compound (I). Polymorph screening experiments were set up using methods including, but not limited to slow evaporation, slow cooling, slurry, solid-vapor diffusion, liquid-vapor diffusion and anti-solvent addition. A total of six crystal forms of free base were observed during screening and identification processes, which were designated as Freebase Type B, C, D E, F and G. Most of the forms were characterized by XRPD, TGA, DSC and ¹H NMR. Identification results revealed Freebase Type B and E were anhydrates, Freebase Type C, D, F and G were hydrates, Freebase Type A was an acetone solvate.

Slurry competition experiments were performed to study the interconversion relationship between Freebase Type B and Freebase Type C. The results showed Freebase Type B was obtained after slurry in solvent systems with water activity (Aw) of about 0.4, 0.6 and 0.8 at 24 ± 3 °C, Freebase Type C was obtained after slurry in H₂O with a water activity of about 1, indicating the critical water activity between Freebase Type B and Type C was in the range of 0.8~1. The inter-conversion relationship among anhydrate Freebase Type B and hydrates Type C/F/G was further investigated under different water activities at RT. the saturated solution of Freebase Type B was obtained via slurry of Freebase Type B at RT overnight in corresponding solvent systems. A mixture of Freebase Type B+C+F+G was added into corresponding saturated solution of Freebase Type B to form suspensions. The suspensions were sampled for XRPD after being stirred for four days at a speed of 750 rpm under RT. Freebase Type B was observed in EtOH and EtOH/H₂O (aw=0.2, 0.4, 0.6, 0.8) systems, whereas in pure H₂O system, Freebase Type G was obtained.

### EXAMPLES

For illustrative purposes, the following Examples are included. However, it is to be understood that these Examples do not limit the invention and are only meant to suggest a method of practicing the invention. Persons skilled in the art will recognize that the chemical reactions described may be readily adapted to prepare the crystalline forms or amorphous forms described herein, and alternative methods for preparing the crystalline forms or amorphous forms are deemed to be within the scope of this invention. For example, the preparation of the crystalline forms or amorphous form described herein may be successfully performed by modifications apparent to those skilled in the art, *e.g.,* by utilizing other suitable reagents known in the art other than those described, and/or by making routine modifications of reaction conditions. Alternatively, other reactions disclosed herein or known in the art will be recognized as having applicability for preparing the crystalline forms or amorphous forms described herein. Persons skilled in the art will also recognize that the crystalline forms and amorphous forms described may be readily adapted to prepare other crystalline forms and amorphous forms, and alternative methods for preparing the crystalline forms and amorphous forms are within the scope of this invention.

In the Examples described below, unless otherwise indicated, all temperatures are set forth in degrees Celsius. Reagents were purchased from commercial suppliers such as Sigma-Aldrich, Alfa Aesar, or TCI, and were used without further purification unless otherwise indicated.

The crystalline forms or amorphous forms of compound (I) were characterized by X-ray powder diffraction (XRPD), thermogravimetric analysis (TGA) and differential scanning calorimetry (DSC). Stoichiometry was determined using ¹H solution nuclear magnetic resonance (¹H NMR) or high performance liquid chromatography (HPLC) combined with ion chromatography (IC).

### ABBREVIATIONS

Most of the abbreviations used in the present invention are listed in Table 1.

**Table 1 Abbreviations**

| **Solvents or Organic Moieties** | |
|---|---|
| **Abbreviation** | **Full Name** |
| MeOH | methanol |
| EtOH | ethanol |
| Ac | acetate |
| ACN | acetonitrile |
| DCM | dichloromethane |
| DME | dimethoxyethane |
| EtOAc | ethyl acetate |
| IPA | isopropyl alcohol |
| MIBK | methyl isobutyl ketone |
| THF | tetrahydrofuran |
| MeOAc | methyl acetate |
| MTBE | methyl *tert*-butyl ether |
| Me | methyl |
| Et | ethyl |

| **Instruments** | |
|---|---|
| DSC | Differential Scanning Calorimetry |
| mDSC | Modulated Differential Scanning Calorimetry |
| DVS | Dynamic Vapor Sorption |
| HPLC | High Performance Liquid Chromatography |
| KF | Karl Fischer Titration |
| NMR | Nuclear Magnetic Resonance |
| XRPD | X-ray Powder Diffraction |
| TGA | Thermogravimetric Analysis |
| PLM | Polarizing Microscope |
| AUC | Area Under Curve |
| PK | Pharmacokinetic |
| FaSSIF | Fasted State Simulated Intestinal Fluid |
| FeSSIF | Fed State Simulated Intestinal Fluid |
| SGF | Simulating Gastric Fluid |
| Aw | water activity |
| FWHM | Full Width at Half Maxima |
| Rel. Int. | Relative Intensity |
| TG | Thermogravimetry |

| **Units** | |
|---|---|
| c | Celsius |
| ° | Degrees |
| eq. | Equivalents |
| g | Gram |
| h | Hour |
| K | Kelvin |
| L | Liters |
| mg | Milligrams |
| mL | Milliliters |
| min | Minute |
| mA | Milliamp |
| kV | Kilovolt |
| RH | Relative Humidity |
| RT | Room Temperature |
| sec | Second |
| vol. | volume |
| v/v | Volume Ratio |
| W | Watt |
| wt. | Weight |
| wt.% | Weight Percentage |

### ANALYSIS CONDITIONS

### X-Ray Powder Diffraction (XRPD)

The XRPD analysis was conducted using a PANalytical Empyrean or a X' Pert3 X-ray powder diffractometers. Typical XRPD parameters used in salt screening and evaluation and in polymorph study are listed in Table 2.

**Table 2 Typical XRPD parameters**

| **Parameters** | **XRPD** |
|---|---|
| X-Ray | Cu, Ka |
| Kα1 (Å) | 1.54060 |
| Ka2 (Å) | 1.54443 |
| Kα2/Kα1 intensity ratio | 0.50 |
| Voltage | 45 kV |
| Current | 40 mA |
| Scan range (20/°) | 3-40 |

### Thermogravimetric Analysis and Differential Scanning Calorimetry (TGA & DSC)

In salt screening and evaluation and in polymorph study, TGA was performed using a TA Q500, TA Q5000 or Discovery 5500 TGA from TA Instruments. DSC or mDSC was performed using a TA Q200/Q2000 or Discovery 2500 DSC from TA Instruments. Typical parameters are listed in Tables 3 and 4.

**Table 3 Parameters for TGA and DSC test in salt screening and evaluation and in polymorph study**

| Parameters | TGA | DSC |
|---|---|---|
| Heating rate | 10 °C/min | 10 °C/min |
| Purge gas | N₂ | N₂ |

**Table 4 Parameters for mDSC test in salt screening and evaluation and in polymorph study**

| Parameters | mDSC |
|---|---|
| Model | Conventional mDSC |
| Amplitude | 1.0 °C |
| Modulation | 60 s |
| Ramp rate | 3.0 °C/min |
| Purge gas | N₂ |

### Dynamic Vapor Sorption (DVS)

DVS is measured via a SMS (Surface Measurement Systems) DVS Intrinsic. The relative humidity at 25 °C is calibrated against deliquescence point of LiCl, Mg(NO₃)₂ and KCl. Typical parameters for DVS test are listed in Table 5.

**Table 5 Parameters for DVS test**

| **Parameters** | **DVS** |
|---|---|
| Temperature | 25°C |
| Gas and flow rate | N₂, 200 mL/min |
| dm/dt | 0.002%/min |
| Min. Dm/dt stability duration | 10 min |
| Max. equilibrium time | 180 min |
| RH range | 0%RH ~ 95%RH ~ 0%RH for anhydrate; |
| | Room humidity ~ 95%RH ~ 0%RH ~ 95%RH for hydrate |
| RH step size | 10%RH (0%RH ~ 90%RH & 90%RH ~ 0%RH) |
| | 5%RH (90%RH ~ 95%RH & 95%RH ~ 90%RH) |

### ¹H-Nuclear Magnetic Resonance Spectroscopy (¹H-NMR)

¹H solution NMR was collected on Bruker 400M NMR Spectrometer using DMSO-*d₆* as solvent.

### Polarizing Microscope (PLM)

The PLM images were captured with Carl Zeiss Axio Scope. A1 microscope at RT.

### High Performance Liquid Chromatography (HPLC)

An Agilent 1260 HPLC instrument was utilized and detailed chromatographic conditions for purity and solubility analysis were listed in Table 6.

**Table 6 Chromatographic conditions and parameters**

| **HPLC** | **Agilent 1260 Detector** | |
|---|---|---|
| Column | Waters Xbridge C18, 150x4.6 mm, 5 µm | |
| Mobile phase | A: 0.03% NH₃·H₂O in H₂O | |
| | B: 0.03% NH₃·H₂O in ACN | |

| | Time (min) | %B |
|---|---|---|
| Gradient table | 0.0 | 10 |
| | 13.0 | 95 |
| | 16.0 | 95 |
| | 16.01 | 10 |
| | 20.0 | 10 |
| Run time | 20.0 min | |
| Post time | 0.0 min | |
| Flow rate | 1.0 mL/min | |
| Injection volume | 5 µL | |
| Detector | UV at 214 nm, 254 nm | |
| Column temperature | 25 °C | |
| Sampler temperature | RT | |
| Diluent | Acetonitrile/H₂O=1:1 (v/v) | |

| | | |
|---|---|---|
| *: Data stated in this report corresponds to the UV wavelength of 214 nm. | | |

### Ion chromatograph (IC)

Thermo Fisher ICS-1100 was utilized and detailed parameters were listed in Table 7.

**Table 7 Ion chromatograph conditions and parameters**

| | |
|---|---|
| IC | Thermo Fisher ICS-1100 |
| Column | IonPac AS18 Analytical Column (4 x 250 mm) |
| Mobile phase | 25 mM NaOH |
| Injection volume | 25 gL |
| Flow rate | 1.0 mL/min |
| Cell temperature | 35 °C |
| Column temperature | 35 °C |
| Current | 80 mA |
| Run time | 12.0 min for PO₄³⁻, 6.0 min for Cl, 8 min for SO₄²⁻ |

### Example 1

### Fumarate Type A of compound (I)

Fumarate Type A of compound (I) was prepared according to the following process:
a) dissolving 500 mg of free base of compound (I) in ~10 mL of EtOAc to form a free base solution;
b) dissolving 158.6 mg of fumaric acid (charging molar ratio of 1.5:1, acid/free base) in ~5 mL of EtOH to form an acid solution;
c) adding the acid solution into the free base solution dropwise while stirring;
d) adding ~13 mL of n-heptane dropwise; then optionally seeding the mixture with a Fumarate Type A crystalline form of compound (I);
e) stirring at about 5 °C for about 16 hours; and
f) isolating a solid via filtration and then vacuum drying the solid at about 50 °C.

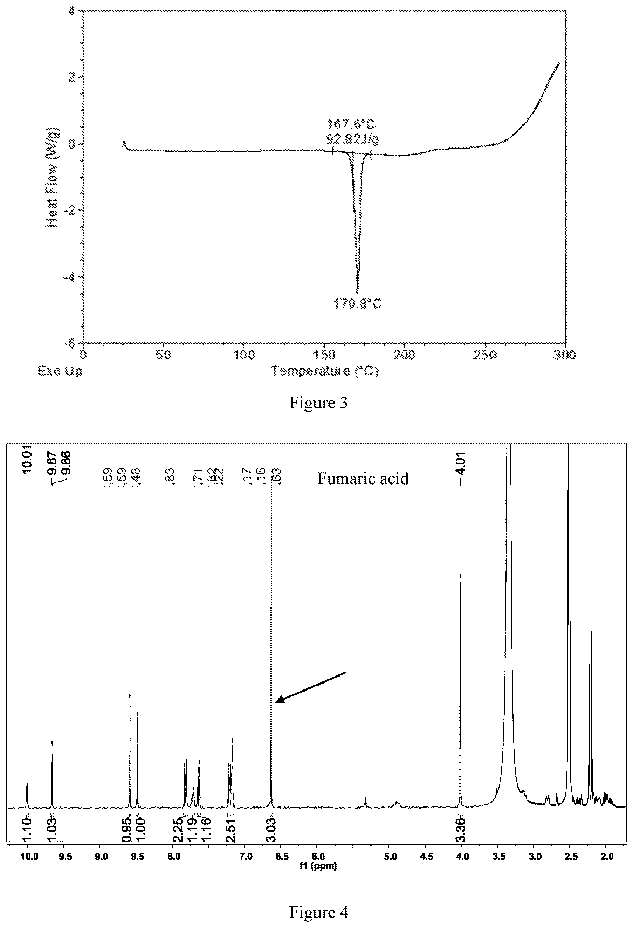

Fumarate Type A of compound (I) was an anhydrate and slightly hygroscopic rod-like crystals (shown via PLM in Figure 5), with an acid/base stoichiometric ratio of about 1.5:1, which was characterized by XRPD, TGA, DSC and ¹H NMR. XRPD patterns and data were shown in Figure 1 and Table 8, respectively. TGA/DSC results in Figure 2 and Figure 3 showed a weight loss of 1.9% up to 150 °C and a sharp endothermic peak at 167.6 °C. ¹H NMR result in Figure 4 showed a 1.5:1 molar ratio of acid/base.

**Table 8 XRPD data of Fumarate Type A of compound (I)**

| **Pos. [°2θ]** | **Height [cts]** | **FWHM Left [°20]** | **d-spacing [Å]** | **Rel. Int. [%]** |
|---|---|---|---|---|
| 5.78 | 747.66 | 0.0768 | 15.28 | 14.70 |
| 6.92 | 2413.36 | 0.1023 | 12.78 | 47.45 |
| 11.53 | 5085.81 | 0.1023 | 7.67 | 100.00 |
| 12.14 | 1527.04 | 0.1023 | 7.29 | 30.03 |
| 12.54 | 113.68 | 0.0768 | 7.06 | 2.24 |
| 12.98 | 184.78 | 0.0768 | 6.82 | 3.63 |
| 14.20 | 34.22 | 0.1535 | 6.24 | 0.67 |
| 14.96 | 110.09 | 0.0768 | 5.92 | 2.16 |
| 15.22 | 169.36 | 0.1023 | 5.82 | 3.33 |
| 16.28 | 242.45 | 0.0768 | 5.44 | 4.77 |
| 16.55 | 226.02 | 0.0768 | 5.36 | 4.44 |
| 17.73 | 1183.03 | 0.1279 | 5.00 | 23.26 |
| 18.92 | 329.05 | 0.1023 | 4.69 | 6.47 |
| 19.11 | 121.55 | 0.0768 | 4.64 | 2.39 |
| 19.39 | 48.28 | 0.1279 | 4.58 | 0.95 |
| 19.76 | 96.80 | 0.1023 | 4.49 | 1.90 |
| 20.77 | 441.19 | 0.1023 | 4.28 | 8.67 |
| 21.41 | 241.17 | 0.1023 | 4.15 | 4.74 |
| 21.81 | 68.03 | 0.1023 | 4.08 | 1.34 |
| 22.42 | 75.24 | 0.1023 | 3.97 | 1.48 |
| 22.90 | 113.57 | 0.0768 | 3.88 | 2.23 |
| 23.12 | 284.68 | 0.0768 | 3.85 | 5.60 |
| 23.67 | 277.14 | 0.1023 | 3.76 | 5.45 |
| 24.01 | 255.09 | 0.1279 | 3.71 | 5.02 |
| 24.36 | 137.29 | 0.1023 | 3.65 | 2.70 |
| 25.32 | 104.25 | 0.1535 | 3.52 | 2.05 |
| 25.71 | 89.13 | 0.1535 | 3.47 | 1.75 |
| 27.40 | 110.84 | 0.1791 | 3.25 | 2.18 |
| 27.95 | 161.33 | 0.1023 | 3.19 | 3.17 |
| 28.20 | 131.97 | 0.1023 | 3.16 | 2.59 |
| 28.83 | 358.38 | 0.1279 | 3.10 | 7.05 |
| 29.27 | 58.13 | 0.0768 | 3.05 | 1.14 |
| 30.27 | 38.63 | 0.1535 | 2.95 | 0.76 |
| 31.27 | 70.46 | 0.1023 | 2.86 | 1.39 |
| 32.14 | 29.79 | 0.2047 | 2.78 | 0.59 |
| 32.81 | 56.25 | 0.1535 | 2.73 | 1.11 |
| 34.97 | 74.29 | 0.0768 | 2.57 | 1.46 |
| 35.88 | 49.94 | 0.1535 | 2.50 | 0.98 |
| 38.00 | 42.11 | 0.1535 | 2.37 | 0.83 |
| 38.63 | 26.82 | 0.1535 | 2.33 | 0.53 |

### Example 2

### Fumarate Type B of compound (I)

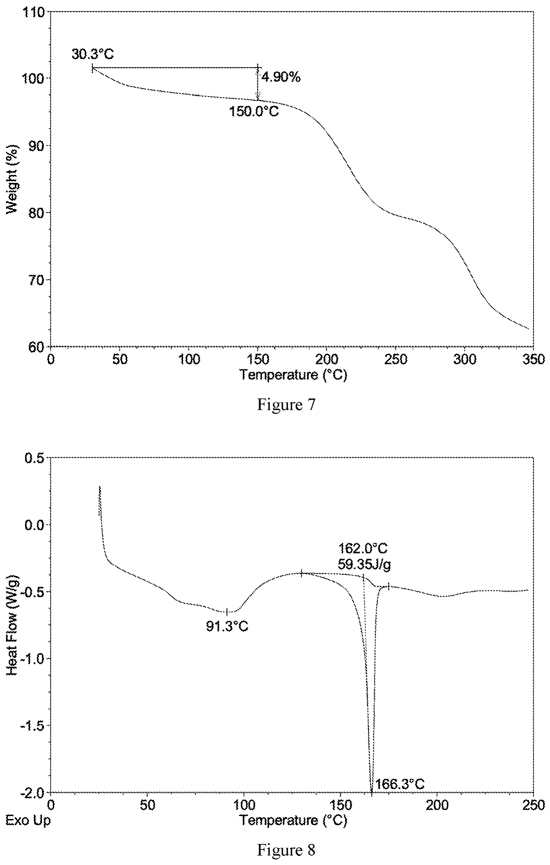

About 15 mg of Fumarate Type A of compound (I) was suspended in 0.5 mL of H₂O in an HPLC vial. The suspension was magnetically stirred at a speed of about 1000 rpm at RT for 11 days. The solids were isolated via centrifugation and stored openly at ambient conditions for 4 days to afford Fumarate Type B of compound (I). XRPD results were displayed in Figure 6 and Table 9. TGA and DSC results were displayed in Figure 7 and Figure 8. TGA curve showed a weight loss of 4.9% up to 150 °C. DSC curve showed a broad endothermic peak at 91.3 °C, which might be caused by removal of water or solvent, and a sharp endothermic peak at 166.3 °C. ¹H NMR result in Figure 9 revealed the molar ratio of fumaric acid/free base is 1:1. After heating Fumarate Type B of compound (I) to 140 °C and cooling to RT, Fumarate Type A of compound (I) was obtained. Thus, Fumarate Type B of compound (I) was a hydrate, which converted to an anhydrate after loss of crystal water by heating.

**Table 9 XRPD data of Fumarate Type B of compound (I)**

| **Pos. [°2θ]** | **Height [cts]** | **FWHM Left [°20]** | **d-spacing [Å]** | **Rel. Int. [%]** |
|---|---|---|---|---|
| 6.58 | 280.25 | 0.0768 | 13.43 | 100.00 |
| 10.73 | 87.49 | 0.1535 | 8.25 | 31.22 |
| 11.40 | 190.27 | 0.0768 | 7.77 | 67.89 |
| 12.91 | 95.03 | 0.1535 | 6.86 | 33.91 |
| 25.11 | 110.67 | 0.1023 | 3.55 | 39.49 |
| 28.24 | 75.24 | 0.1535 | 3.16 | 26.85 |

### Example 3

### Fumarate Type C of compound (I)

About 15 mg of Fumarate Type A of compound (I) was suspended in 0.5 mL of H₂O in an HPLC vial. The suspension was magnetically stirred at a speed of about 1000 rpm at RT for 9 days. The wet solids were isolated to afford Fumarate Type C of compound (I) (wet sample was analyzed). Form conversion between Fumarate Type C and Fumarate Type B of compound (I) was observed during storage at ambient conditions, thus further characterization was not performed. Fumarate Type C of compound (I) had an acid/base stoichiometric ratio of 1:1. XRPD patterns and data were shown in Figure 10 and Table 10, respectively.

**Table 10 XRPD data of Fumarate Type C of compound (I)**

| **Pos. [°2θ]** | **Height [cts]** | **FWHM Left [°2θ]** | **d-spacing [Å]** | **Rel. Int. [%]** |
|---|---|---|---|---|
| 6.78 | 1150.22 | 0.1535 | 13.04 | 100.00 |
| 7.56 | 132.31 | 0.2047 | 11.70 | 11.50 |
| 11.18 | 325.28 | 0.1791 | 7.91 | 28.28 |
| 11.83 | 416.92 | 0.1535 | 7.48 | 36.25 |
| 12.79 | 174.89 | 0.2047 | 6.92 | 15.21 |
| 13.56 | 282.12 | 0.1535 | 6.53 | 24.53 |
| 15.06 | 195.70 | 0.1535 | 5.88 | 17.01 |
| 16.03 | 216.09 | 0.2047 | 5.53 | 18.79 |
| 16.40 | 171.68 | 0.1535 | 5.40 | 14.93 |
| 17.20 | 170.28 | 0.1023 | 5.16 | 14.80 |
| 18.40 | 258.42 | 0.1023 | 4.82 | 22.47 |
| 19.62 | 42.81 | 0.4093 | 4.52 | 3.72 |
| 20.97 | 92.64 | 0.2047 | 4.24 | 8.05 |
| 21.53 | 113.77 | 0.2047 | 4.13 | 9.89 |
| 21.97 | 102.86 | 0.1535 | 4.05 | 8.94 |
| 22.63 | 98.51 | 0.2558 | 3.93 | 8.56 |
| 24.49 | 245.23 | 0.1279 | 3.63 | 21.32 |
| 27.06 | 128.48 | 0.1535 | 3.29 | 11.17 |
| 28.28 | 167.84 | 0.2047 | 3.16 | 14.59 |
| 30.32 | 46.20 | 0.3070 | 2.95 | 4.02 |

### Example 4

### Fumarate Type E of compound (I)

About 30 mg of Fumarate Type A of compound (I) was dissolved in 1 mL of ethyl formate in a glass vial. The solution was kept at RT for evaporation to afford Fumarate Type E of compound (I). XRPD pattern and data were shown in Figure 11 and Table 11, respectively. TGA and DSC results in Figure 12 and Figure 13 showed a weight loss of 9.4% up to 150 °C and endothermic peaks at 134.5 and 166.0 °C. The molar ratio of formic acid : free base was 1.5:1.

**Table 11 XRPD data of Fumarate Type E of compound (I)**

| **Pos. [°2θ]** | **Height [cts]** | **FWHM Left [°2θ]** | **d-spacing [Å]** | **Rel. Int. [%]** |
|---|---|---|---|---|
| 5.48 | 367.11 | 0.0768 | 16.13 | 29.81 |
| 6.68 | 1231.48 | 0.0768 | 13.24 | 100.00 |
| 7.64 | 271.12 | 0.0768 | 11.58 | 22.02 |
| 10.94 | 957.43 | 0.1023 | 8.09 | 77.75 |
| 11.59 | 1176.34 | 0.0768 | 7.64 | 95.52 |
| 12.93 | 556.94 | 0.1023 | 6.85 | 45.23 |
| 13.34 | 138.91 | 0.0768 | 6.64 | 11.28 |
| 15.29 | 483.18 | 0.1023 | 5.80 | 39.24 |
| 15.62 | 257.76 | 0.1535 | 5.67 | 20.93 |
| 16.25 | 286.37 | 0.1023 | 5.46 | 23.25 |
| 16.86 | 845.67 | 0.1279 | 5.26 | 68.67 |
| 18.13 | 418.70 | 0.1023 | 4.89 | 34.00 |
| 19.91 | 550.25 | 0.1023 | 4.46 | 44.68 |
| 20.62 | 104.12 | 0.3070 | 4.31 | 8.46 |
| 22.30 | 261.57 | 0.1023 | 3.99 | 21.24 |
| 23.00 | 485.96 | 0.1023 | 3.87 | 39.46 |
| 23.43 | 269.40 | 0.2047 | 3.80 | 21.88 |
| 25.16 | 653.13 | 0.1023 | 3.54 | 53.04 |
| 25.53 | 192.52 | 0.1279 | 3.49 | 15.63 |
| 27.47 | 460.42 | 0.1279 | 3.25 | 37.39 |
| 28.45 | 365.71 | 0.2558 | 3.14 | 29.70 |
| 28.82 | 273.91 | 0.1535 | 3.10 | 22.24 |
| 30.81 | 76.68 | 0.1535 | 2.90 | 6.23 |

### Example 5

### Freebase Type B of compound (I)

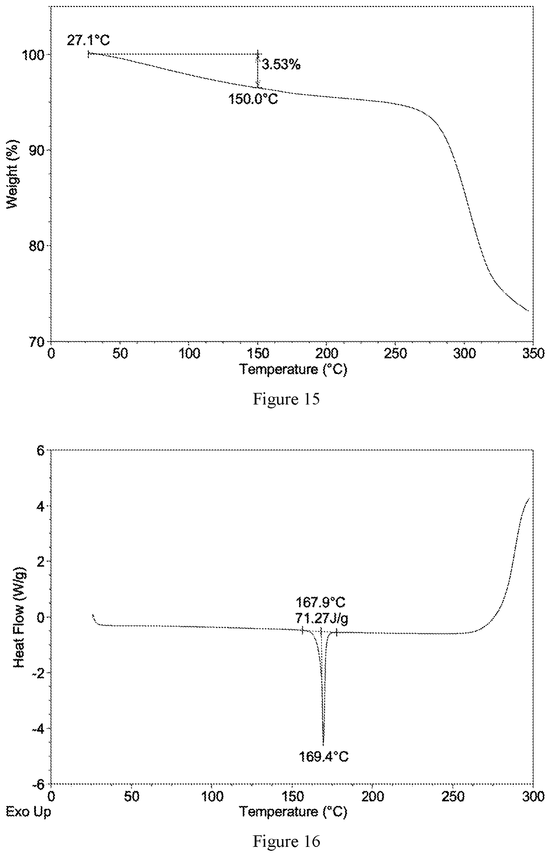

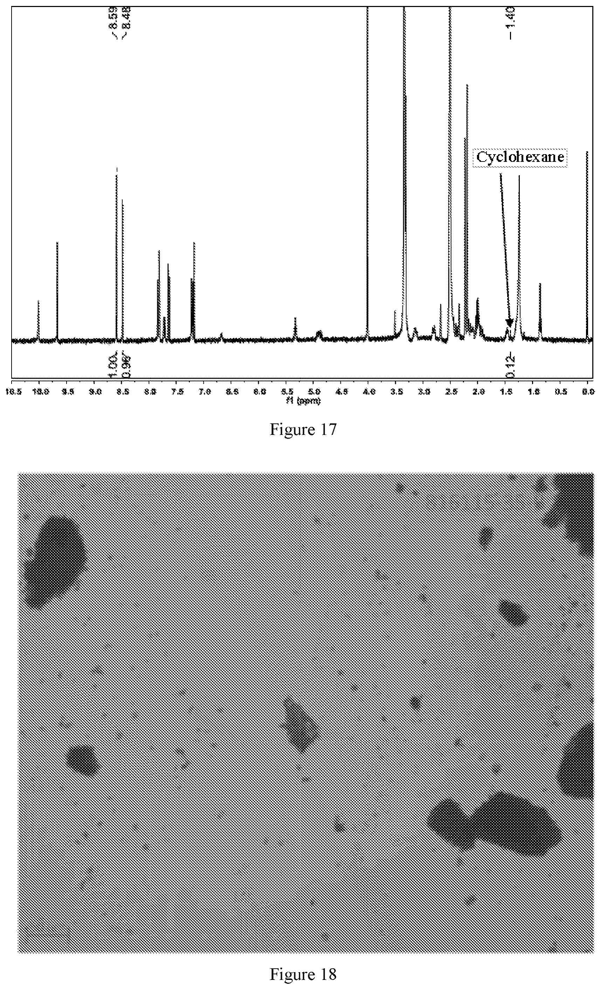

About 15 mg of Amorphous Freebase of compound (I) was suspended in 0.5 mL of MIBK/Cyclohexane (1:4, v/v) in an HPLC vial. The suspension was magnetically stirred at a speed of about 1000 rpm at 5 °C for 7 days. The solids were isolated via centrifugation to afford Freebase Type B of compound (I). XRPD pattern and data were shown in Figure 14 and Table 12, respectively. TGA and DSC results in Figure 15 and Figure 16 showed a weight loss of 3.5% up to 150 °C and a sharp endothermic peak at 169.4 °C (peak). ¹H NMR result in Figure 17 revealed the molar ratio of residual solvent cyclohexane/free base was 0.1:1 (corresponding to a TGA weight loss of 0.2%). PLM in Figure 18 showed Freebase Type B of compound (I) was an anhydrate, and slightly hygroscopic irregular particles with aggregation.

**Table 12 XRPD data of Freebase Type B of compound (I)**

| **Pos. [°2θ]** | **Height [cts]** | **FWHM Left [°2θ]** | **d-spacing [Å]** | **Rel. Int. [%]** |
|---|---|---|---|---|
| 7.95 | 1663.66 | 0.1023 | 11.12 | 100.00 |
| 9.30 | 119.65 | 0.1535 | 9.51 | 7.19 |
| 10.89 | 141.29 | 0.1535 | 8.12 | 8.49 |
| 11.47 | 1277.05 | 0.1023 | 7.71 | 76.76 |
| 12.26 | 308.52 | 0.1023 | 7.22 | 18.54 |
| 13.09 | 378.21 | 0.1023 | 6.76 | 22.73 |
| 13.43 | 164.38 | 0.1023 | 6.59 | 9.88 |
| 13.98 | 244.07 | 0.0768 | 6.34 | 14.67 |
| 14.70 | 185.36 | 0.1023 | 6.02 | 11.14 |
| 15.96 | 1164.64 | 0.1023 | 5.55 | 70.00 |
| 16.35 | 172.87 | 0.1023 | 5.42 | 10.39 |
| 17.22 | 663.22 | 0.1279 | 5.15 | 39.86 |
| 18.21 | 417.37 | 0.1279 | 4.87 | 25.09 |
| 18.79 | 673.71 | 0.2047 | 4.72 | 40.50 |
| 20.25 | 440.39 | 0.2047 | 4.39 | 26.47 |
| 21.19 | 264.03 | 0.1023 | 4.19 | 15.87 |
| 21.92 | 439.89 | 0.1535 | 4.05 | 26.44 |
| 22.61 | 350.04 | 0.1279 | 3.93 | 21.04 |
| 23.40 | 156.84 | 0.1023 | 3.80 | 9.43 |
| 23.68 | 117.14 | 0.1535 | 3.76 | 7.04 |
| 24.28 | 837.15 | 0.1279 | 3.67 | 50.32 |
| 25.03 | 292.19 | 0.1791 | 3.56 | 17.56 |
| 25.49 | 200.28 | 0.1279 | 3.49 | 12.04 |
| 26.66 | 184.47 | 0.1279 | 3.34 | 11.09 |
| 27.68 | 331.26 | 0.1535 | 3.22 | 19.91 |
| 28.65 | 179.58 | 0.1023 | 3.12 | 10.79 |
| 30.10 | 26.75 | 0.6140 | 2.97 | 1.61 |

### Example 6

### Freebase Type C of compound (I)

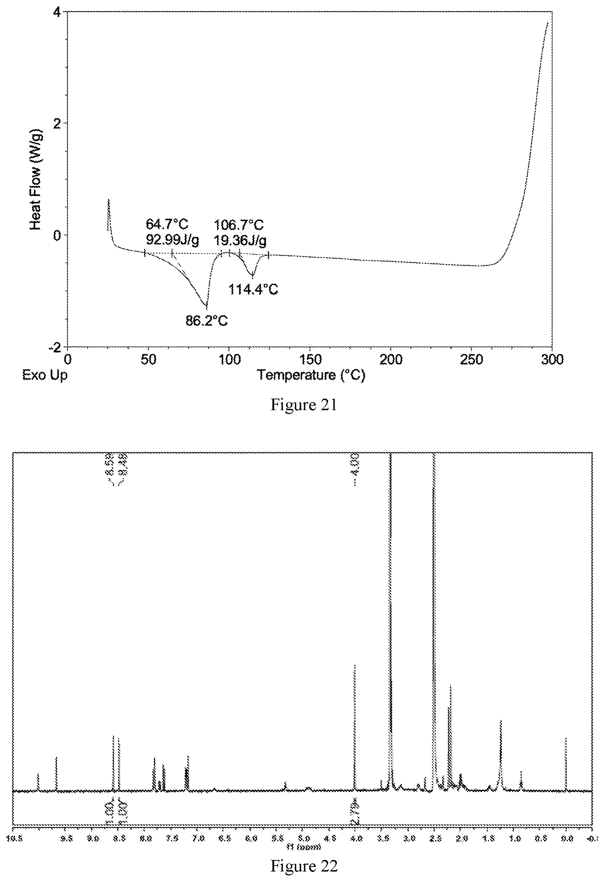

About 15 mg of Amorphous Freebase of compound (I) was suspended in 0.5 mL of THF/H₂O (1:4, v/v) in an HPLC vial. The suspension was magnetically stirred at a speed of about 1000 rpm at 5 °C for 7 days. The solids were isolated via centrifugation to afford Freebase Type C of compound (I). XRPD results were displayed in Figure 19 and Table 13. TGA and DSC results were displayed in Figure 20 and Figure 21. A weight loss of 1.5% up to 150 °C and two endotherms at 86.2 °C and 114.4 °C (peak) were observed on TGA/DSC curves. ¹H NMR result in Figure 22 indicated solvent THF was not detected. Combined with characterization results of Freebase Type D of compound (I) (refer to Example 7), Freebase Type C of compound (I) was speculated to be a hydrate.

**Table 13 XRPD data of Freebase Type C of compound (I)**

| **Pos. [°2θ]** | **Height [cts]** | **FWHM Left [°2θ]** | **d-spacing [Å]** | **Rel. Int. [%]** |
|---|---|---|---|---|
| 5.42 | 179.01 | 0.2047 | 16.32 | 18.80 |
| 6.05 | 335.53 | 0.1023 | 14.61 | 35.24 |
| 6.55 | 952.18 | 0.1279 | 13.50 | 100.00 |
| 9.36 | 226.87 | 0.2558 | 9.45 | 23.83 |
| 10.77 | 98.41 | 0.2047 | 8.22 | 10.33 |
| 11.96 | 348.89 | 0.1535 | 7.40 | 36.64 |
| 12.23 | 376.40 | 0.1279 | 7.23 | 39.53 |
| 12.65 | 590.48 | 0.2814 | 7.00 | 62.01 |
| 14.10 | 283.39 | 0.1023 | 6.28 | 29.76 |
| 14.96 | 243.74 | 0.1023 | 5.96 | 25.60 |
| 15.42 | 135.63 | 0.1535 | 5.75 | 14.24 |
| 16.14 | 129.75 | 0.1535 | 5.49 | 13.63 |
| 16.44 | 160.90 | 0.1535 | 5.39 | 16.90 |
| 18.14 | 263.71 | 0.1791 | 4.89 | 27.70 |
| 18.81 | 758.86 | 0.1791 | 4.72 | 79.70 |
| 20.29 | 226.43 | 0.1535 | 4.38 | 23.78 |
| 20.69 | 401.41 | 0.1791 | 4.29 | 42.16 |
| 22.87 | 217.22 | 0.1791 | 3.89 | 22.81 |
| 23.37 | 367.60 | 0.1791 | 3.81 | 38.61 |
| 24.41 | 501.98 | 0.1535 | 3.65 | 52.72 |
| 26.67 | 250.40 | 0.1791 | 3.34 | 26.30 |
| 28.33 | 120.06 | 0.1535 | 3.15 | 12.61 |
| 29.77 | 102.50 | 0.2047 | 3.00 | 10.76 |

### Example 7

### Freebase Type D of compound (I)

About 60 mg of Amorphous Freebase of compound (I) was suspended in 2 mL of THF/H₂O (1:4, v/v) in an HPLC vial. The suspension was magnetically stirred at a speed of about 1000 rpm at 5 °C for 3 days. The solids were isolated and dried at ambient condition for about 2 hours to afford Freebase Type D of compound (I). XRPD results were displayed in Figure 23 and Table 14. Freebase Type D of compound (I) was a hydrate and can be obtained by placing Freebase Type C of compound (I) at ambient condition.

**Table 14 XRPD data of Freebase Type D of compound (I)**

| **Pos. [°2θ]** | **Height [cts]** | **FWHM Left [°2θ]** | **d-spacing [Å]** | **Rel. Int. [%]** |
|---|---|---|---|---|
| 5.68 | 792.36 | 0.0768 | 15.56 | 100.00 |
| 5.83 | 762.93 | 0.0768 | 15.16 | 96.29 |
| 11.84 | 357.29 | 0.0768 | 7.47 | 45.09 |
| 12.64 | 422.36 | 0.1023 | 7.00 | 53.30 |
| 14.79 | 180.99 | 0.1023 | 5.99 | 22.84 |
| 16.22 | 51.86 | 0.4093 | 5.47 | 6.55 |
| 18.09 | 164.35 | 0.1023 | 4.90 | 20.74 |
| 18.75 | 611.21 | 0.1023 | 4.73 | 77.14 |
| 20.23 | 128.90 | 0.1535 | 4.39 | 16.27 |
| 20.62 | 297.92 | 0.0768 | 4.31 | 37.60 |
| 22.77 | 98.98 | 0.2047 | 3.90 | 12.49 |
| 23.31 | 246.12 | 0.1023 | 3.82 | 31.06 |
| 24.33 | 294.69 | 0.1023 | 3.66 | 37.19 |
| 26.61 | 186.89 | 0.1279 | 3.35 | 23.59 |
| 28.24 | 96.43 | 0.1535 | 3.16 | 12.17 |
| 29.71 | 43.25 | 0.3070 | 3.01 | 5.46 |

### Example 8

### Freebase Type E of compound (I)

About 60 mg of Amorphous Freebase of compound (I) was suspended in 2 mL of THF/H₂O (1:4, v/v) in an HPLC vial. The suspension was magnetically stirred at a speed of about 1000 rpm at 5 °C for 4 days. The solids were isolated via centrifugation and dried at ambient condition for about 2 hours. After sweeping the solids by N₂ for 20 min at 30 °C. Freebase Type E of compound (I) was obtained. XRPD pattern and XRPD data were displayed in Figure 24 and Table 15. Freebase Type E was speculated to be an anhydrate, which converted to Freebase Type C of compound (I) after exposed to ambient condition for 30 min.

**Table 15 XRPD data of Freebase Type E of compound (I)**

| **Pos. [°2θ]** | **Height [cts]** | **FWHM Left [°2θ]** | **d-spacing [Å]** | **Rel. Int. [%]** |
|---|---|---|---|---|
| 7.15 | 3056.24 | 0.1506 | 12.36 | 49.09 |
| 9.00 | 239.01 | 0.5353 | 9.83 | 3.84 |
| 10.21 | 320.60 | 0.2007 | 8.67 | 5.15 |
| 10.66 | 426.37 | 0.2007 | 8.30 | 6.85 |
| 11.44 | 872.10 | 0.1673 | 7.74 | 14.01 |
| 11.88 | 1254.83 | 0.1004 | 7.45 | 20.15 |
| 12.13 | 1485.86 | 0.1004 | 7.30 | 23.86 |
| 12.61 | 2002.58 | 0.0836 | 7.02 | 32.16 |
| 13.26 | 1599.15 | 0.1338 | 6.68 | 25.68 |
| 14.24 | 852.55 | 0.1673 | 6.22 | 13.69 |
| 14.89 | 2101.74 | 0.1673 | 5.95 | 33.76 |
| 15.75 | 1103.14 | 0.1673 | 5.63 | 17.72 |
| 16.72 | 2055.28 | 0.1338 | 5.30 | 33.01 |
| 16.97 | 1559.56 | 0.1338 | 5.23 | 25.05 |
| 17.51 | 1624.85 | 0.0669 | 5.07 | 26.10 |
| 18.22 | 6226.26 | 0.1338 | 4.87 | 100.00 |
| 19.23 | 2754.51 | 0.2007 | 4.62 | 44.24 |
| 19.69 | 1912.85 | 0.2007 | 4.51 | 30.72 |
| 20.53 | 1753.97 | 0.2676 | 4.33 | 28.17 |
| 21.12 | 1497.95 | 0.2007 | 4.21 | 24.06 |
| 22.32 | 3223.61 | 0.1338 | 3.98 | 51.77 |
| 22.96 | 2928.85 | 0.2342 | 3.87 | 47.04 |
| 23.98 | 2313.82 | 0.1171 | 3.71 | 37.16 |
| 24.49 | 922.49 | 0.3011 | 3.64 | 14.82 |
| 25.32 | 893.64 | 0.4015 | 3.52 | 14.35 |
| 26.79 | 2219.59 | 0.1171 | 3.33 | 35.65 |
| 27.53 | 1639.47 | 0.2007 | 3.24 | 26.33 |
| 30.12 | 444.37 | 0.2676 | 2.97 | 7.14 |
| 31.59 | 358.23 | 0.2007 | 2.83 | 5.75 |

### Example 9

### Freebase Type F of compound (I)

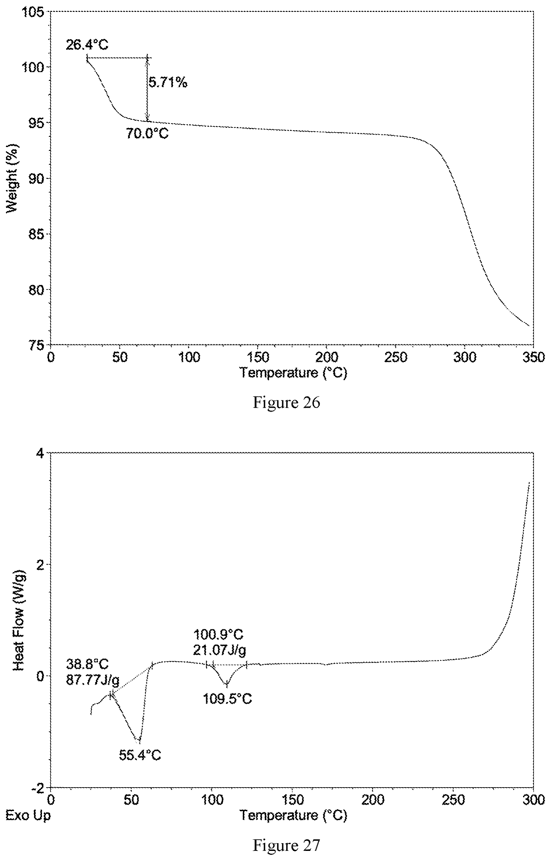

About 15 mg of Amorphous Freebase of compound (I) was suspended in 0.5 mL of ACN/n-heptane (1:9, v/v) in an HPLC vial. The suspension was magnetically stirred at a speed of about 1000 rpm at RT for 2 days. The solids were isolated via centrifugation. Freebase Type F was a hydrate. The XRPD pattern and data were shown in Figure 25 and Table 16. The TGA curve in Figure 26 showed a weight loss of 5.7% up to 70 °C. The DSC curve in Figure 27 showed endothermic peaks at 55.4 and 109.5 °C.

**Table 16 XRPD data of Freebase Type F of compound (I)**

| **Pos. [°2θ]** | **Height [cts]** | **FWHM Left [°2θ]** | **d-spacing [Å]** | **Rel. Int. [%]** |
|---|---|---|---|---|
| 6.21 | 390.60 | 0.1023 | 14.22 | 100.00 |
| 9.28 | 91.51 | 0.2558 | 9.53 | 23.43 |
| 11.61 | 184.30 | 0.1279 | 7.62 | 47.18 |
| 12.60 | 285.87 | 0.1791 | 7.03 | 73.19 |
| 14.78 | 104.67 | 0.1535 | 6.00 | 26.80 |
| 16.48 | 99.59 | 0.2047 | 5.38 | 25.50 |
| 17.63 | 93.91 | 0.2047 | 5.03 | 24.04 |
| 18.70 | 68.76 | 0.3070 | 4.74 | 17.60 |
| 19.31 | 97.94 | 0.1535 | 4.60 | 25.07 |
| 24.40 | 104.28 | 0.1535 | 3.65 | 26.70 |
| 25.98 | 95.68 | 0.3070 | 3.43 | 24.50 |

### Example 10

### Freebase Type G of compound (I)

About 15 mg of Amorphous Freebase of compound (I) was dissolved in 0.5 mL of EtOH in a glass vial. Then anti-solvent of H₂O was added into the EtOH solution and suspension was obtained. The solids were isolated via centrifugation. Freebase Type G was a hydrate. The XRPD pattern and data were shown in Figure 28 and Table 17. The TGA curve in Figure 29 showed a weight loss of 7.3% up to 70 °C. The DSC curve in Figure 30 showed endothermic peaks at 32.9, 59.2 and 110.2 °C.

**Table 17 XRPD data of Freebase Type G of compound (I)**

| **Pos. [°2θ]** | **Height [cts]** | **FWHM Left [°2θ]** | **d-spacing [Å]** | **Rel. Int. [%]** |
|---|---|---|---|---|
| 5.72 | 490.99 | 0.1023 | 15.46 | 100.00 |
| 5.88 | 419.00 | 0.0768 | 15.02 | 85.34 |
| 9.42 | 143.63 | 0.1279 | 9.38 | 29.25 |
| 11.90 | 377.30 | 0.1279 | 7.44 | 76.84 |
| 12.67 | 424.74 | 0.1279 | 6.99 | 86.51 |
| 13.38 | 59.99 | 0.1535 | 6.62 | 12.22 |
| 14.54 | 207.70 | 0.1023 | 6.09 | 42.30 |
| 15.01 | 138.61 | 0.1535 | 5.90 | 28.23 |
| 17.19 | 158.21 | 0.1023 | 5.16 | 32.22 |
| 17.64 | 197.55 | 0.1279 | 5.03 | 40.24 |
| 18.19 | 146.33 | 0.2047 | 4.88 | 29.80 |
| 19.67 | 198.37 | 0.1023 | 4.51 | 40.40 |
| 20.57 | 197.20 | 0.1535 | 4.32 | 40.16 |
| 21.28 | 121.55 | 0.2047 | 4.17 | 24.76 |
| 22.49 | 145.94 | 0.1279 | 3.95 | 29.72 |
| 22.92 | 203.47 | 0.1535 | 3.88 | 41.44 |
| 24.83 | 183.67 | 0.1023 | 3.59 | 37.41 |
| 26.21 | 296.80 | 0.1279 | 3.40 | 60.45 |
| 29.11 | 20.59 | 0.8187 | 3.07 | 4.19 |

### Example 11

### Freebase Type A of compound (I)

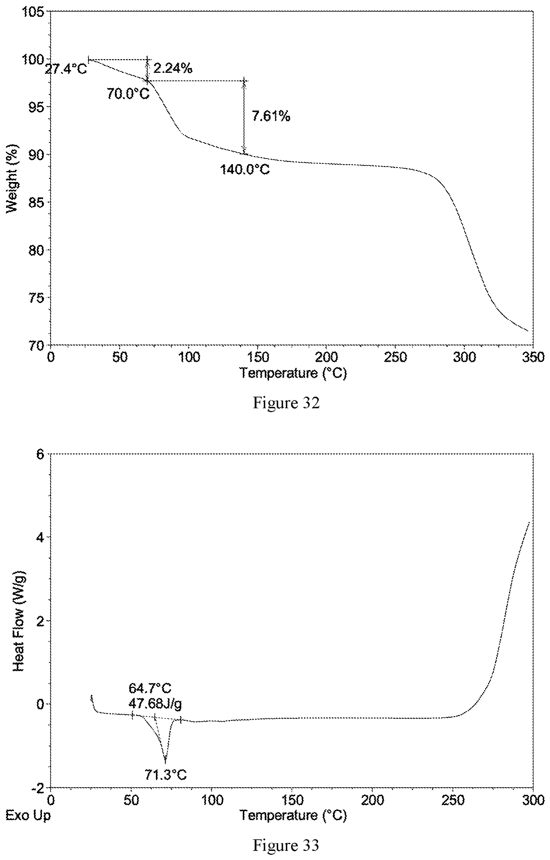

About 15 mg of Amorphous Freebase of compound (I) was suspended in 0.5 mL of acetone/n-heptane (1:4, v:v) in an HPLC vial. The suspension was magnetically stirred at a speed of about 1000 rpm at RT for 3 days. The solids were isolated via centrifugation and dried at ambient conditions for 1 day. Freebase Type A of compound (I) was an acetone solvate, obtained via slurry of Amorphous Freebase in Acetone/n-heptane (1:4) at RT for 3 days. The XRPD pattern and data were displayed in Figure 31 and Table 18, respectively. The TGA and DSC curves in Figure 32 and Figure 33 showed a weight loss of 9.9% up to 140 °C and an endotherm at 71.3 °C (peak temperature). The ¹H NMR result displayed in Figure 34 showed the molar ratio of acetone : free base was 0.7:1 (6.9 wt%).

**Table 18 XRPD data of Freebase Type A of compound (I)**

| **Pos. [°2θ]** | **Height [cts]** | **FWHM Left [°2θ]** | **d-spacing [Å]** | **Rel. Int. [%]** |
|---|---|---|---|---|
| 7.03 | 184.41 | 0.1023 | 12.57 | 58.97 |
| 9.02 | 312.73 | 0.1023 | 9.80 | 100.00 |
| 11.57 | 45.51 | 0.3070 | 7.65 | 14.55 |
| 13.60 | 60.29 | 0.6140 | 6.51 | 19.28 |
| 15.35 | 56.80 | 0.3070 | 5.77 | 18.16 |
| 18.05 | 124.01 | 0.2047 | 4.92 | 39.65 |
| 19.59 | 74.78 | 0.4093 | 4.53 | 23.91 |
| 23.26 | 161.58 | 0.1279 | 3.82 | 51.67 |

### Example 12

### HCl Salt Type A of compound (I)

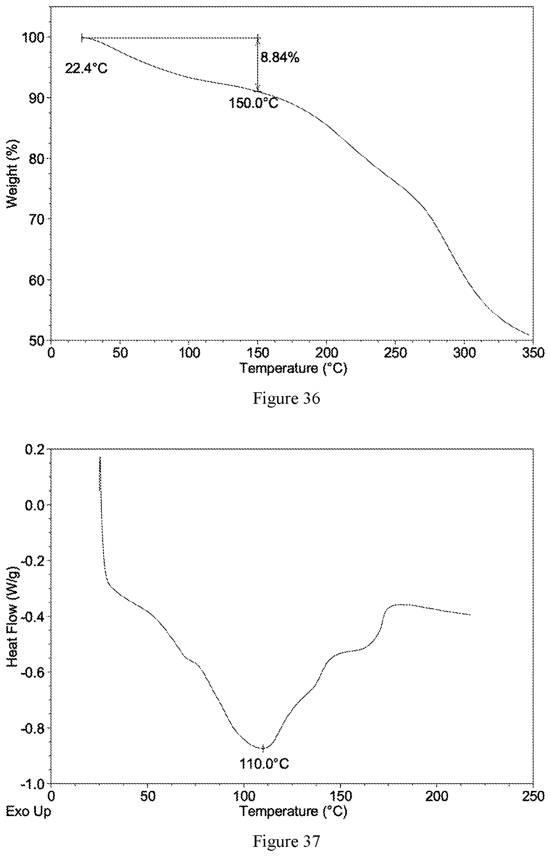

About 15 mg of Amorphous Freebase of compound (I) and 4.6 µL of concentrated HCl (charging molar ratio of 2:1, acid/free base) were suspended in 0.5 mL of EtOAc/n-heptane (1:2, v:v) in an HPLC vial. The suspension was magnetically stirred at a speed of about 1000 rpm at RT for 3 days. The solids were isolated via centrifugation and dried at ambient conditions for 1 day to afford HCl salt Type A of compound (I). The XRPD pattern and data were displayed in Figure 35 and Table 19, respectively. The TGA and DSC curves in Figure 36 and Figure 37 showed a weight loss of 8.8% up to 150 °C and an endotherm at 110.0 °C (peak temperature). The molar ratio of HCl : free base was 2.5:1.

**Table 19 XRPD data of HCl salt Type A of compound (I)**

| **Pos. [°2θ]** | **Height [cts]** | **FWHM Left [°2θ]** | **d-spacing [Å]** | **Rel. Int. [%]** |
|---|---|---|---|---|
| 18.09 | 51.60 | 0.4992 | 4.90 | 100.00 |

### Example 13

### HCl Salt Type B of compound (I)

About 500 mg Amorphous Freebase of compound (I) was dissolved in about 10 mL EtOAc. 935 µL of the EtOAc solution of HCl (charging molar ratio of 2:1, acid/free base) was diluted into 15 mL EtOH. HCl salt Type B of compound (I) was optionally added into the free base solution as seeds and it was not completely dissolved. Then the acid solution was added dropwise while stirring at a speed of about 1000 rpm. The mixture was stirred at RT for 8 hours after it turned turbid, and then further stirred at 5 °C for 13 hours. The solids precipitated was isolated via filtration, and dried under vacuum at RT overnight to afford HCl Salt Type B of compound (I). The XRPD pattern and data were displayed in Figure 38 and Table 20, respectively. The TGA and DSC curves in Figure 39 and Figure 40 showed a weight loss of 2.3% up to 150 °C and an endotherm at 241.7 °C (peak temperature). The molar ratio of HCl : free base was 2.2:1.

**Table 20 XRPD data of HCl salt Type B of compound (I)**

| **Pos. [°2θ]** | **Height [cts]** | **FWHM Left [°2θ]** | **d-spacing [Å]** | **Rel. Int. [%]** |
|---|---|---|---|---|
| 6.92 | 147.00 | 0.1535 | 12.77 | 67.72 |
| 9.62 | 81.60 | 0.2047 | 9.19 | 37.59 |
| 11.86 | 114.20 | 0.2047 | 7.46 | 52.61 |
| 12.35 | 160.57 | 0.1791 | 7.17 | 73.97 |
| 16.99 | 109.35 | 0.1535 | 5.22 | 50.37 |
| 19.89 | 55.01 | 0.3070 | 4.46 | 25.34 |
| 21.16 | 67.34 | 0.4093 | 4.20 | 31.02 |
| 22.72 | 92.52 | 0.1535 | 3.91 | 42.62 |
| 24.96 | 217.08 | 0.1535 | 3.57 | 100.00 |
| 25.88 | 46.24 | 0.3070 | 3.44 | 21.30 |
| 27.20 | 56.91 | 0.3070 | 3.28 | 26.21 |
| 29.05 | 106.71 | 0.1535 | 3.07 | 49.16 |

### Example 14

### Mesylate Type A of compound (I)

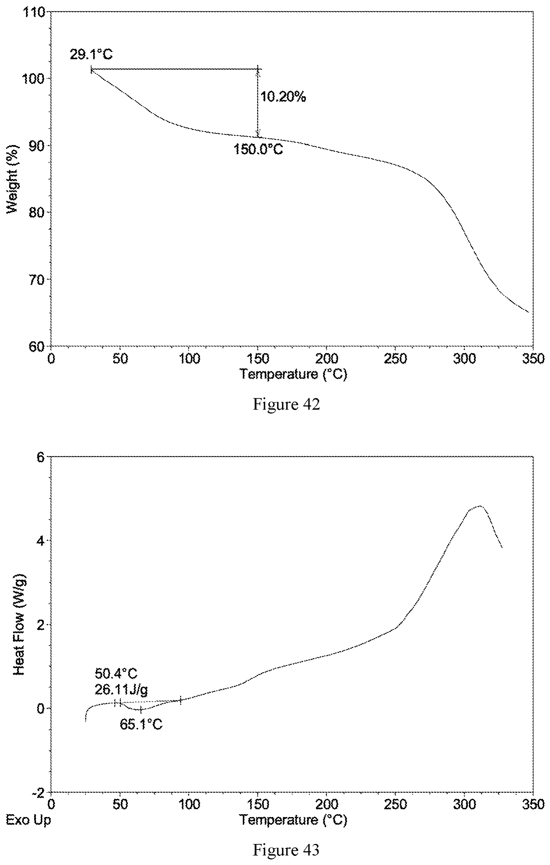

About 15 mg of Amorphous Freebase of compound (I) and 5.5 mg of methane sulfonic acid (charging molar ratio of 2:1, acid/free base) were suspended in 0.5 mL of Acetone/n-heptane (1:4, v:v) in an HPLC vial. The suspension was magnetically stirred at a speed of about 1000 rpm at RT for 3 days. The solids were isolated via centrifugation and dried at ambient conditions for 1 day to afford Mesylate Type A of compound (I). The XRPD pattern and data were displayed in Figure 41 and Table 21, respectively. The TGA and DSC curves in Figure 42 and Figure 43 showed a weight loss of 10.2% up to 150 °C and an endotherm at 65.1 °C (peak temperature). The molar ratio of acid : free base was 2.0:1.

**Table 21 XRPD data of Mesylate Type A of compound (I)**

| **Pos. [°2θ]** | **Height [cts]** | **FWHM Left [°2θ]** | **d-spacing [Å]** | **Rel. Int. [%]** |
|---|---|---|---|---|
| 5.26 | 81.87 | 0.2047 | 16.79 | 52.38 |
| 6.53 | 125.58 | 0.2558 | 13.53 | 80.34 |
| 7.80 | 106.39 | 0.3070 | 11.33 | 68.06 |
| 13.09 | 104.87 | 0.3070 | 6.76 | 67.09 |
| 15.69 | 97.94 | 0.3070 | 5.65 | 62.59 |
| 19.64 | 156.31 | 0.2047 | 4.52 | 100.00 |
| 21.03 | 121.74 | 0.1535 | 4.22 | 77.88 |

### Example 15

### Mesylate Type B of compound (I)

About 15 mg of Amorphous Freebase of compound (I) and 5.4 mg of methanesulfonic acid (charging molar ratio of 2:1, acid/free base) were suspended in 0.5 mL of IPA/Cyclohexane (1:4, v:v) in an HPLC vial. The suspension was magnetically stirred at a speed of about1000 rpm at RT for 3 days. The solids were isolated via centrifugation and dried at ambient conditions for 1 day to afford Mesylate Type B of compound (I). The XRPD pattern and data were displayed in Figure 44 and Table 22, respectively. The TGA and DSC curves in Figure 45 and Figure 46 showed a weight loss of 7.6% up to 150 °C and an endotherm at 63.4 °C (peak temperature). The molar ratio of acid : free base was 1.7:1.

**Table 22 XRPD data of Mesylate Type B of compound (I)**

| **Pos. [°2θ]** | **Height [cts]** | **FWHM Left [°2θ]** | **d-spacing [Å]** | **Rel. Int. [%]** |
|---|---|---|---|---|
| 6.03 | 227.18 | 0.1023 | 14.66 | 100.00 |
| 7.18 | 41.09 | 0.3070 | 12.31 | 18.09 |
| 12.42 | 29.20 | 0.6140 | 7.13 | 12.85 |
| 16.30 | 79.53 | 0.3070 | 5.44 | 35.01 |
| 18.32 | 99.47 | 0.4093 | 4.84 | 43.79 |
| 21.52 | 41.53 | 0.6140 | 4.13 | 18.28 |
| 26.49 | 69.51 | 0.3070 | 3.36 | 30.60 |

### Example 16

### Phosphate Type A of compound (I)

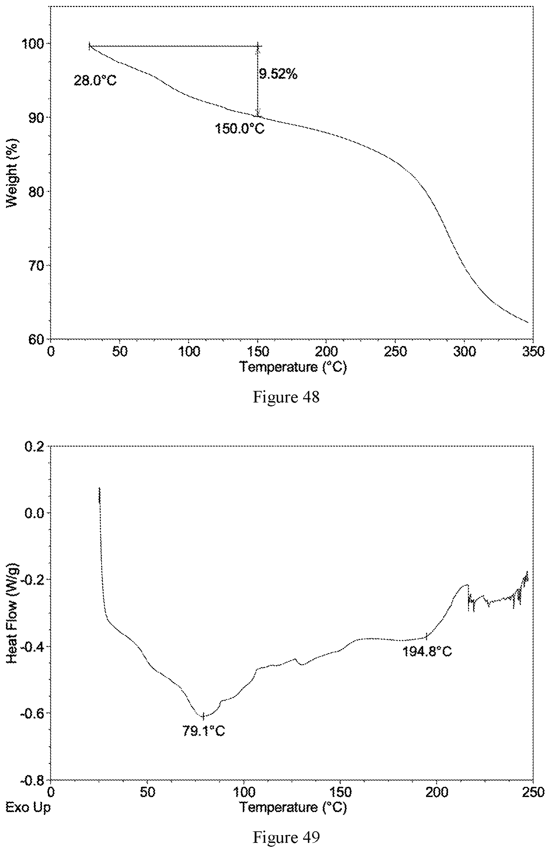

About 15 mg of Amorphous Freebase of compound (I) and 1.9 µL of concentrated H₃PO₄ (charging molar ratio of 1:1, acid/free base) were suspended in 0.5 mL of acetone/n-heptane (1:4, v:v) in an HPLC vial. The suspension was magnetically stirred at a speed of about 1000 rpm at RT for 3 days. The solids were isolated via centrifugation and dried at ambient conditions for 1 day to afford Phosphate Type A of compound (I). The XRPD pattern and data were displayed in Figure 47 and Table 23, respectively. The TGA and DSC curves in Figure 48 and Figure 49 showed a weight loss of 9.5% up to 150 °C and endotherms at 79.1 °C and 194.8 °C (peak temperature). The molar ratio of acid : free base was 1.1:1.

**Table 23 XRPD data of phosphate Type A of compound (I)**

| **Pos. [°2θ]** | **Height [cts]** | **FWHM Left [°2θ]** | **d-spacing [Å]** | **Rel. Int. [%]** |
|---|---|---|---|---|
| 6.38 | 271.85 | 0.1279 | 13.86 | 100.00 |
| 13.95 | 263.49 | 0.1023 | 6.35 | 96.92 |
| 14.93 | 119.88 | 0.1535 | 5.94 | 44.10 |
| 16.28 | 69.96 | 0.2047 | 5.45 | 25.73 |
| 18.65 | 51.33 | 0.3070 | 4.76 | 18.88 |
| 20.45 | 95.92 | 0.1535 | 4.34 | 35.28 |
| 21.40 | 72.32 | 0.4093 | 4.15 | 26.60 |
| 22.90 | 159.58 | 0.1279 | 3.88 | 58.70 |
| 24.46 | 127.00 | 0.4093 | 3.64 | 46.72 |

### Example 17

### L-tartrate Type A of compound (I)

About 15 mg of Amorphous Freebase of compound (I) and 4.3 mg of L-tartaric acid (charging molar ratio of 1:1, acid/free base) were suspended in 0.5 mL of EtOAc/n-heptane (1:2, v:v) in an HPLC vial. The suspension was magnetically stirred a speed of about 1000 rpm at RT for 3 days. The solids were isolated via centrifugation and dried at ambient conditions for 1 day to afford L-tartrate Type A of compound (I). The XRPD pattern and data were displayed in Figure 50 and Table 24, respectively. The TGA and DSC curves in Figure 51 and Figure 52 showed a weight loss of 3.7% up to 150 °C and endothermic peaks at 77.6 and 164.7 °C. The molar ratio of L-tartaric acid : free base was 0.5:1.

**Table 24 XRPD data of L-tartrate Type A of compound (I)**

| **Pos. [°2θ]** | **Height [cts]** | **FWHM Left [°2θ]** | **d-spacing [Å]** | **Rel. Int. [%]** |
|---|---|---|---|---|
| 6.51 | 205.26 | 0.1535 | 13.57 | 100.00 |
| 9.36 | 73.76 | 0.3070 | 9.44 | 35.94 |
| 12.71 | 182.52 | 0.2558 | 6.97 | 88.92 |
| 18.81 | 151.23 | 0.2303 | 4.72 | 73.68 |
| 20.66 | 113.31 | 0.2047 | 4.30 | 55.20 |
| 22.69 | 55.54 | 0.4093 | 3.92 | 27.06 |
| 24.35 | 91.10 | 0.3582 | 3.66 | 44.38 |
| 26.47 | 27.87 | 0.8187 | 3.37 | 13.58 |

### Example 18

### Adipate Type A of compound (I)

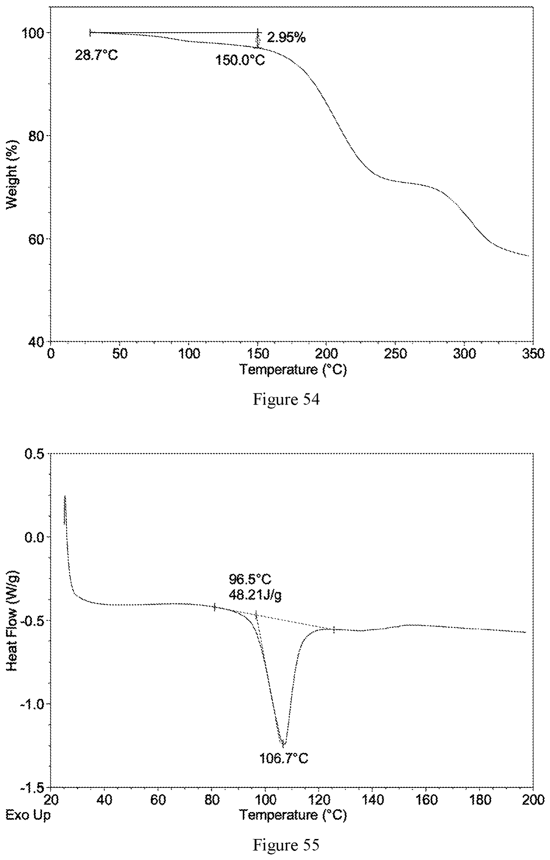

About 15 mg of Amorphous Freebase of compound (I) and 4.3 mg of adipic acid (charging molar ratio of 1:1, acid/free base) were suspended in 0.5 mL of EtOAc/n-heptane (1:2, v:v) in an HPLC vial. The suspension was magnetically stirred at a speed of about 1000 rpm at RT for 3 days. The solids were isolated via centrifugation and dried at ambient conditions for 1 day to afford Adipate Type A of compound (I). The XRPD pattern and data were displayed in Figure 53 and Table 25, respectively. The TGA and DSC curves in Figure 54 and Figure 55 showed a weight loss of 3.0% up to 150 °C and endotherms at 106.7 °C (peak temperature). The molar ratio of adipic acid : free base was 1.3:1.

**Table 25 XRPD data of Adipate Type A of compound (I)**

| **Pos. [°2θ]** | **Height [cts]** | **FWHM Left [°2θ]** | **d-spacing [Å]** | **Rel. Int. [%]** |
|---|---|---|---|---|
| 7.44 | 396.51 | 0.1023 | 11.89 | 49.17 |
| 8.47 | 98.93 | 0.1535 | 10.44 | 12.27 |
| 10.81 | 806.41 | 0.2047 | 8.18 | 100.00 |
| 12.64 | 205.47 | 0.1279 | 7.01 | 25.48 |
| 14.92 | 130.14 | 0.3070 | 5.94 | 16.14 |
| 15.51 | 143.91 | 0.1535 | 5.71 | 17.85 |
| 16.01 | 391.31 | 0.1023 | 5.54 | 48.53 |
| 16.87 | 102.66 | 0.3070 | 5.25 | 12.73 |
| 17.72 | 314.60 | 0.1279 | 5.01 | 39.01 |
| 18.99 | 154.58 | 0.1535 | 4.67 | 19.17 |
| 19.70 | 249.98 | 0.1535 | 4.51 | 31.00 |
| 20.90 | 188.93 | 0.1535 | 4.25 | 23.43 |
| 23.57 | 156.30 | 0.1023 | 3.77 | 19.38 |
| 25.73 | 533.73 | 0.1535 | 3.46 | 66.19 |
| 32.29 | 35.92 | 0.3070 | 2.77 | 4.45 |

### Example 19

### Amorphous Fumarate of compound (I)

About 3 g of Fumarate Type A of compound (I) was dissolved in about 50 mL of MeOH in a glass vial. The solution was transferred into the rotatory evaporator and the solvent was removed by rotatory evaporation at 60 °C to afford Amorphous Fumarate of compound (I). The XRPD pattern was shown in Figure 56. The TGA curve in Figure 57 showed a weight loss of 3.6% up to 150 °C. The mDSC curve in Figure 58 showed a middle temperature of thermogravimetry (TG) at 79.9 and 107.5 °C. The molar ratio of formic acid : free base was 1.5:1.

### Example 20

### Amorphous Freebase of compound (I)

About 2 g of Freebase Type B of compound (I) was dissolved in about 40 mL of DCM in a glass vial. The solution was transferred into the rotatory evaporator and the solvent was removed by rotatory evaporation at 40 °C to afford Amorphous Freebase of compound (I). The XRPD pattern was shown in Figure 59. The TGA curve in Figure 60 showed a weight loss of 1.9% up to 150 °C. The mDSC curve in Figure 61 showed a middle temperature of thermogravimetry (TG) at 58.7 °C.

### Example 21: Comparison of solubility of amorphous free base, Fumarate Type B and Fumarate Type E in FaSSIF

Kinetic solubility of amorphous freebase and Fumarate Type B/E was measured in FaSSIF to evaluate the solubility. All the solubility samples (initial solid loading of 5~10 mg/mL) were kept rolling on a rolling incubator, and sampled at 1, 4 and 24 hours at 37 °C, respectively. After centrifugation, supernatants were collected for HPLC tests.

The results were summarized in Table 26. Compared with amorphous freebase, which was seen as prior art, Fumarate Type B and Fumarate Type E showed higher solubility in FaSSIF.

**Table 26 Results summary of kinetic solubility evaluation in FaSSIF**

| **Media** | **Timepoint** | **amorphous freebase** | **Fumarate Type B** | **Fumarate Type E** |
|---|---|---|---|---|
| | 1 h | 0.28 | ≥ 0.39 | ≥ 0.36 |
| Solubility in FaSSIF (mg/mL) | 2 h | 0.27 | ≥ 0.39 | ≥ 0.36 |
| | 24 h | 0.26 | ≥ 0.39 | ≥ 0.36 |

### Example 22 Comparison of solubility of amorphous free base and Fumarate Type A/B/E in H₂O

Equilibrium solubility of amorphous free base and Fumarate Type A/B/E was assessed in H₂O at 37 °C. About 5~10 mg of solids were suspended in H₂O to get a mixture. The suspensions were stirred for 24 hours, followed by centrifugation and filtration to obtain supernatants for HPLC test.

Results were summarized in Table 27. Compared with amorphous freebase, which was seen as prior art, Fumarate Type A, Fumarate Type B and Fumarate Type E showed much better solubility in H₂O.

**Table 27 Results summary of equilibrium solubility evaluation in H₂O**

| **Media** | **amorphous free base** | **Fumarate Type A** | **Fumarate Type B** | **Fumarate Type E** |
|---|---|---|---|---|
| Solubility in H₂O (mg/mL) | 0.16 | ≥ 5.0 | ≥ 5.2 | ≥ 5.0 |

### Example 23 Comparison of solubility of amorphous free base, Freebase Type B and Fumarate Type A/B/E in pH 4.5 buffer

Equilibrium solubility of amorphous free base, Freebase Type B and Fumarate Type A/B/E was assessed in pH 4.5 buffer at 37 °C. About 5~10 mg of solids were suspended in pH 4.5 buffer to get a mixture. The suspensions were stirred for 24 hours, followed by centrifugation and filtration to obtain supernatants for HPLC test.

Results were summarized in Table 28. Compared with amorphous freebase, which was seen as prior art, Freebase Type B, Fumarate Type A, Fumarate Type B and Fumarate Type E showed better solubility in pH 4.5 buffer.

**Table 28 Results summary of equilibrium solubility evaluation in pH 4.5 buffer**

| **Media** | **amorphous free base** | **Freebase Type B** | **Fumarate Type A** | **Fumarate Type B** | **Fumarate Type E** |
|---|---|---|---|---|---|
| Solubility in pH 4.5 buffer (mg/mL) | 0.18 | 0.3 | ≥ 8.4 | 2.3 | 1.3 |

### Example 24 Comparison of solubility of amorphous free base and Fumarate Type B/E in pH 6.8 buffer

Equilibrium solubility of amorphous free base and Fumarate Type B/E was assessed in pH 6.8 buffer at 37 °C. About 5~10 mg of solids were suspended in pH 6.8 buffer to get a mixture. The suspensions were stirred for 24 hours, followed by centrifugation and filtration to obtain supernatants for HPLC test.

Results were summarized in Table 29. Compared with amorphous freebase, which was seen as prior art, Fumarate Type B and Fumarate Type E showed improved solubility in pH 6.8 buffer.

**Table 29 Results summary of equilibrium solubility evaluation in pH 6.8 buffer**

| **Media** | **amorphous free base** | **Fumarate Type B** | **Fumarate Type E** |
|---|---|---|---|
| Solubility in pH 6.8 buffer (mg/mL) | 0.15 | 0.90 | 0.92 |

### Example 25: Flowability and Compressibility

Bulk density was determined by adding appropriate amount (m) of material into a 5 mL graduate cylinder and recording its apparent volume (v₀). The bulk density was obtained by dividing the amount of material by its apparent volume of the unsettled material (ρ₀=m/v₀). For tap density, the cylinder was then tapped for 200 times. The tap density was then calculated by dividing the amount of material by its final tapped volume (ρ₁=m/v₁). Carr index = (ρ₁-ρ₀)/ρ₁.

Form the angle of repose on a fixed base with a retaining lip to retain a layer of powder on the base. Carefully build up a symmetrical cone of powder. Determine the angle of repose by measuring the height (h) and base radius (r) of the cone of powder and calculating the angle of repose α from the following equation α=tan⁻¹(h/r).

The results of amorphous freebase, Freebase Type F, Fumarate Type B and Fumarate Type E for flowability and compressibility were summarized in Table 30. Generally, powders with smaller Karl index would show better compressibility and flowability. And powders with smaller angle of repose would show better flowability.

The results showed that, compared with amorphous freebase, Freebase Type F, Fumarate Type B and Fumarate Type E showed relatively better flowability and compressibility.

**Table 30 Data summary of powder properties**

| **Solid form (Sample ID)** | **Bulk density (g/cm³)** | **Tapped density (g/cm³)** | **Carr index** | **Angle of repose** |
|---|---|---|---|---|
| Freebase amorphous | 0.23 | 0.40 | 43.8% | 39.9° |
| Freebase Type F | 0.19 | 0.28 | 31.9% | 35.8° |
| Fumarate Type B | 0.36 | 0.52 | 30.4% | 26.6° |
| Fumarate Type E | 0.30 | 0.51 | 41.7% | 38.3° |

### Example 26: Contact Angle

The wettability of the solid forms was evaluated by the contact angle against water. The results summarized in Table 31 showed that all the samples of the selected solid forms can be wetted by water. Compared with amorphous freebase sample, Fumarate Type A and Fumarate Type B showed relatively smaller contact angle, suggesting that Fumarate Type A and Fumarate Type B could be wetted by water more easily.

**Table 31 Summary of contact angle results**

| **Solid form** | **Contact angle** |
|---|---|
| Freebase amorphous | 37.2° |
| Fumarate Type A | 20.3° |
| Fumarate Type B | 29.0° |

### Example 27: Crystal Habit

PLM was used to observe the crystal habit of the particles (amorphous freebase and Fumarate Type A). The results showed that the amorphous freebase sample was composed of irregular-shaped particles without defined boundary. Fumarate Type A was rod-like particles with regular shape. Compared with amorphous freebase, Fumarate Type A revealed better crystal habit, which is more suitable for further development.

### Example 28: Hygroscopicity

DVS isotherm plot was collected at 25 °C to investigate the selected solid forms stability as a function of humidity. The results were summarized in Table 32. Compared with amorphous freebase, the hygroscopicity of Freebase Type B, Fumarate Type A, Fumarate Type B and Fumarate Type E was lower under 80%RH/25 °C.

**Table 32 Summary of DVS results**

| **Solid form** | **Weight change at 80%RH/25 °C by DVS** |
|---|---|
| Freebase amorphous | 4.2% |
| Freebase Type B | 0.2% |
| Fumarate Type A | 0.8% |
| Fumarate Type B | 2.1% |
| Fumarate Type E | 2.1% |

### Example 29: Mechanical Stability

Mechanical stability of the solid forms was evaluated by monitoring the XRPD form change after grinding (~5 min) and tableting (~234 Mpa). The XRPD results showed that no form change was observed for Freebase Type B, Fumarate Type A and Fumarate Type E after grinding or tableting (Table 33), which confirmed Freebase Type B, Fumarate Type A and Fumarate Type E showed good mechanical stability.

**Table 33 Summary of mechanical stability results**

| **Solid form** | **XRPD form change after grinding** | **XRPD form change after tableting** |
|---|---|---|
| Freebase Type B | No | No |
| Fumarate Type A | No | No |
| Fumarate Type E | No | No |

### Example 30: Solid State Stability

To evaluate the solid state stability of the selected forms (amorphous freebase, Freebase Type F, Fumarate Type A and Fumarate Type B) samples were stored under 40 °C/75%RH/open for 1 month. Stability samples were characterized by XRPD to check any solid form change and by HPLC to check purity change. All of the results were summarized in Table 34.

The results showed that: (1) Freebase Type F, Fumarate Type A and Fumarate Type B revealed good physical stability as evidenced by no form change in all conditions; (2) Compared with the purity change of amorphous freebase after being stored under 40 °C/75%RH for 1 month, Freebase Type F, Fumarate Type A and Fumarate Type B showed better chemical stability with lower purity change.

**Table 34 Evaluation results of solid state stability**

| Initial Solid Form | Condition | Time | Form Change | Purity/Initial by HPLC area% |
|---|---|---|---|---|
| Freebase amorphous | | | No | 99.2% |
| Freebase Type F | 40 °C, 75%RH | 1 month | No | 100.3% |
| Fumarate Type A | | | No | 100.1% |
| Fumarate Type B | | | No | 99.6% |

### CHARACTERIZATION RESULTS

Characterization results of fumarate forms, free base forms and crystalline salt forms of compound (I) were summarized in Tables 35~37.

**Table 35 Characterization results of fumarate forms**

| **Solid form** | **TGA weight loss (%, temp.)** | **DSC endotherm (°C, peak)** | **Molar ratio (acid/base)** | **Speculated form** |
|---|---|---|---|---|
| Fumarate Type A | 1.9 (to 150 °C) | 167.6 | 1.5:1 | Anhydrate |
| Fumarate Type B | 4.9 (to 150 °C) | 91.3, 166.3 | 1:1 | Hydrate |
| Fumarate Type C | | -- | -- | -- |
| Fumarate Type E | 9.4 (to 150 °C) | 134.5, 166.0 | 1.5:1 | Solvate of formic acid |
| Amorphous Fumarate | 3.6 (to 150 °C) | 79.9*. 107.5* | 1.5:1 | -- |

| | | | | |
|---|---|---|---|---|
| --: Fumarate Type C was not characterized and identified due to form conversion between Fumarate Type B and C. *: middle temperature of thermogravimetry | | | | |

**Table 36 Characterization results of free base forms**

| **Form** | **TGA weight loss (%, temp.)** | **DSC endotherm (°C, peak)** | **Speculated form** |
|---|---|---|---|
| Freebase Type A | 9.9 (to 140 °C) | 71.3 | Acetone solvate |
| Freebase Type B | 3.5 (to 150 °C) | 169.4 | Anhydrate |
| Freebase Type C | 1.5 (to 150 °C) | 86.2, 114.4 | Hydrate |
| Freebase Type D | -- | -- | Hydrate |
| Freebase Type E | -- | -- | Anhydrate |
| Freebase Type F | 5.7 (to 70 °C) | 55.4, 109.5 | Hydrate |
| Freebase Type G | 7.3 (to 70 °C) | 32.9, 59.2, 110.2 | Hydrate |
| Amorphous Freebase | 1.9 (to 150 °C) | 58.7* | -- |

| | | | |
|---|---|---|---|
| --: Freebase Type D was not characterized by TGA/DSC due to limited amount of material and form change to Freebase Type E after N₂ purge. Freebase Type E converted to Type D after exposed to ambient condition and was not characterized by TGA/DSC. *: Tg, middle temperature. | | | |

**Table 37 Characterization results of crystalline salts**

| **Solid form** | **TGA weight loss (150 °C, %)** | **DSC endotherm (°C, peak)** | **Molar ratio (acid/free base)** |
|---|---|---|---|
| HCl salt Type A | 8.8 | 110.0 | 2.5:1 |
| HCl salt Type B | 2.3 | 241.7 | 2.2:1 |
| Mesylate Type A | 10.2 | 65.1 | 2.0:1 |
| Mesylate Type B | 7.6 | 63.4 | 1.7:1 |
| Phosphate Type A | 9.5 | 79.1, 194.8 | 1.1:1 |
| L-tartrate Type A | 3.7 | 77.6, 164.7 | 0.5:1 |
| Fumarate Type A | 1.9 | 167.6 | 1.5:1 |
| Fumarate Type B | 4.9 | 91.3, 166.3 | 1:1 |
| Fumarate Type C | 1.5 | -- | -- |
| Fumarate Type E | 9.4 | 134.5, 161.6 | 1.5:1 |
| Adipate Type A | 3.0 | 106.7 | 1.3 |

The foregoing description is considered as illustrative only of the principles of the present invention. Further, since numerous modifications and changes will be readily apparent to those skilled in the art, it is not desired to limit the invention to the exact construction and process shown as described above. Accordingly, all suitable modifications and equivalents may be considered to fall within the scope of the invention as defined by the claims that follow.

### Numbered Clauses

1. A crystalline form of compound (I) represented by the following structural formula: 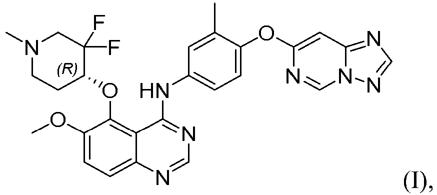 wherein the crystalline form is a complex of free base with a pharmaceutically acceptable acid, or free base.
2. The crystalline form according to clause 1, wherein the complex or free base is a solvate or non-solvate.
3. The crystalline form according to clause 1 or 2, wherein the complex is a salt or a cocrystal or a cocrystal of salt.
4. The crystalline form according to any one of clauses 1-3, wherein the complex has an acid/base molar ratio of 0.5:1 to 3:1, preferably, 0.5:1 to 2.5:1, more preferably, 1:1 to 1.5:1.
5. The crystalline form according to any one of clauses 1-4, wherein the pharmaceutically acceptable acid is chosen from a group consisting of hydrochloride, methanesulfonic acid, phosphoric acid, tartaric acid, fumaric acid and adipic acid.
6. The crystalline form according to any one of clauses 1-5, wherein the pharmaceutically acceptable acid is fumaric acid.
7. The crystalline form according to clause 6, wherein the crystalline form is, Fumarate Type A, characterized by an X-ray powder diffraction pattern which comprises at least peaks at 2θ (± 0.2°) of 6.9 and 11.5.
8. The crystalline form according to clause 7, wherein the crystalline form is, Fumarate Type A, characterized by an X-ray powder diffraction pattern which comprises at least peaks at 2θ (± 0.2°) of 5.8, 6.9, 11.5, 12.1 and 17.7.
9. The crystalline form according to clause 8, wherein the crystalline form is, Fumarate Type A, characterized by an X-ray powder diffraction pattern which comprises at least peaks at 2θ (± 0.2°) of 5.8, 6.9, 11.5, 12.1, 17.7, 20.8 and 24.0.
10. The crystalline form according to clause 9, wherein the crystalline form is, Fumarate Type A, characterized by an X-ray powder diffraction pattern which comprises at least peaks at 2θ (± 0.2°) of 5.8, 6.9, 11.5, 12.1, 17.7, 18.9, 20.8, 23.1, 23.7, 24.0 and 28.8.
11. The crystalline form according to clause 10, wherein the crystalline form is, Fumarate Type A, characterized by substantially the same X-ray powder diffraction pattern as Figure 1.
12. The crystalline form according to any one of clauses 7-11, characterized by a differential scanning calorimeter peak phase transition temperature of about 167.6 °C.
13. The crystalline form according to clause 6, wherein the crystalline form is, Fumarate Type B, characterized by an X-ray powder diffraction pattern which comprises at least peaks at 2θ (± 0.2°) of 6.6 and 11.4.
14. The crystalline form according to clause 13, wherein the crystalline form is, Fumarate Type B, characterized by an X-ray powder diffraction pattern which comprises at least peaks at 2θ (± 0.2°) of 6.6, 10.7, 11.4, 12.9, 25.1 and 28.2.
15. The crystalline form according to clause 14, wherein the crystalline form is, Fumarate Type B, characterized by an X-ray powder diffraction pattern which comprises at least peaks at 2θ (+ 0.2°) of 6.6, 10.7, 11.4, 12.9, 15.8, 17.9, 19.7, 25.1 and 28.2.
16. The crystalline form according to clause 15, wherein the crystalline form is, Fumarate Type B, characterized by substantially the same X-ray powder diffraction pattern as Figure 6.
17. The crystalline form according to any one of clauses 13-16, characterized by a differential scanning calorimeter peak phase transition temperature of about 91.3 °C and about 166.3 °C.
18. The crystalline form according to clause 6, wherein the crystalline form is, Fumarate Type C, characterized by an X-ray powder diffraction pattern which comprises at least peaks at 2θ (± 0.2°) of 6.8 and 11.8.
19. The crystalline form according to clause 18, wherein the crystalline form is, Fumarate Type C, characterized by an X-ray powder diffraction pattern which comprises at least peaks at 2θ (± 0.2°) of 6.8, 11.2, 11.8, 13.6 and 18.4.
20. The crystalline form according to clause 19, wherein the crystalline form is, Fumarate Type C, characterized by an X-ray powder diffraction pattern which comprises at least peaks at 2θ (± 0.2°) of 6.8, 11.2, 11.8, 13.6, 15.1, 16.0, 17.2, 18.4 and 24.5.
21. The crystalline form according to clause 20, wherein the crystalline form is, Fumarate Type C, by substantially the same X-ray powder diffraction pattern as Figure 10.
22. The crystalline form according to clause 6, wherein the crystalline form is, Fumarate Type E, characterized by an X-ray powder diffraction pattern which comprises at least peaks at 2θ (± 0.2°) of 6.7, 11.6 and 28.5.
23. The crystalline form according to clause 22, wherein the crystalline form is, Fumarate Type E, characterized by an X-ray powder diffraction pattern which comprises at least peaks at 2θ (± 0.2°) of 6.7, 10.9, 11.6, 16.9, 25.2 and 28.5.
24. The crystalline form according to clause 23, wherein the crystalline form is, Fumarate Type E, characterized by an X-ray powder diffraction pattern which comprises at least peaks at 2θ (± 0.2°) of 6.7, 10.9, 11.6, 12.9, 15.3, 16.9, 19.9, 25.2 and 28.5.
25. The crystalline form according to clause 24, wherein the crystalline form is, Fumarate Type E, characterized by an X-ray powder diffraction pattern which comprises at least peaks at 2θ (± 0.2°) of 5.5, 6.7, 10.9, 11.6, 12.9, 15.3, 16.9, 18.1, 19.9, 25.2, 27.5 and 28.5.
26. The crystalline form according to clause 25, wherein the crystalline form is, Fumarate Type E, by substantially the same X-ray powder diffraction pattern as Figure 11.
27. The crystalline form according to any one of clauses 22-26, characterized by a differential scanning calorimeter peak phase transition temperature of about 134.5 °C and about 166.0 °C.
28. The crystalline form according to clause 1 or 2, wherein the crystalline form is, Free base Type B, characterized by an X-ray powder diffraction pattern which comprises at least peaks at 2θ (± 0.2°) of 8.0 and 11.5.
29. The crystalline form according to clause 28, wherein the crystalline form is, Free base Type B, characterized by an X-ray powder diffraction pattern which comprises at least peaks at 2θ (± 0.2°) of 8.0, 11.5, 16.0, 17.2, 18.8 and 24.3.
30. The crystalline form according to clause 29, wherein the crystalline form is, Freebase Type B, characterized by an X-ray powder diffraction pattern which comprises at least peaks at 2θ (± 0.2°) of 8.0, 11.5, 16.0, 17.2, 18.2, 18.8, 20.3, 21.9 and 24.3.
31. The crystalline form according to clause 30, wherein the crystalline form is, Freebase Type B, characterized by an X-ray powder diffraction pattern which comprises at least peaks at 2θ (± 0.2°) of 8.0, 11.5, 13.1, 16.0, 17.2, 18.2, 18.8, 20.3, 21.2, 21.9, 24.3 and 27.7.
32. The crystalline form according to clause 31, the crystalline form is, Freebase Type B, characterized by substantially the same X-ray powder diffraction pattern as Figure 14.
33. The crystalline form according to any one of clauses 28-32, characterized by a differential scanning calorimeter peak phase transition temperature of about 169.4 °C.
34. The crystalline form according to clause 1 or 2, wherein the crystalline form is, Freebase Type C, characterized by an X-ray powder diffraction pattern which comprises at least peaks at 2θ (± 0.2°) of 6.6 and 18.8.
35. The crystalline form according to clause 34, wherein the crystalline form is, Freebase Type C, characterized by an X-ray powder diffraction pattern which comprises at least peaks at 2θ (± 0.2°) of 6.6, 12.7, 18.8, 20.7 and 24.4.
36. The crystalline form according to clause 35, wherein the crystalline form is, Freebase Type C, characterized by an X-ray powder diffraction pattern which comprises at least peaks at 2θ (± 0.2°) of 6.6, 12.7, 14.1, 18.1, 18.8, 20.7, 23.4, 24.4 and 26.7.
37. The crystalline form according to clause 36, wherein the crystalline form is, Freebase Type C, characterized by an X-ray powder diffraction pattern which comprises at least peaks at 2θ (± 0.2°) of 6.6, 9.4, 12.7, 14.1, 14.9, 18.1, 18.8, 20.7, 22.9, 23.4, 24.4 and 26.7.
38. The crystalline form according to clause 37, wherein the crystalline form is, Freebase Type C, characterized by substantially the same X-ray powder diffraction pattern as Figure 19.
39. The crystalline form according to any one of clauses 34-38, characterized by a differential scanning calorimeter peak phase transition temperature of about 86.2 °C and about 114.4 °C.
40. The crystalline form according to clause 1 or 2, wherein the crystalline form is, Freebase Type D, characterized by an X-ray powder diffraction pattern which comprises at least peaks at 2θ (± 0.2°) of 5.7, 5.8 and 18.8.
41. The crystalline form according to clause 40, wherein the crystalline form is, Freebase Type D, characterized by an X-ray powder diffraction pattern which comprises at least peaks at 2θ (± 0.2°) of 5.7, 5.8, 11.8, 12.6, 18.8, 20.6 and 24.3.
42. The crystalline form according to clause 41, wherein the crystalline form is, Freebase Type D, characterized by an X-ray powder diffraction pattern which comprises at least peaks at 2θ (± 0.2°) of 5.7, 5.8, 11.8, 12.6, 18.8, 20.6, 22.8, 23.3 and 24.3.
43. The crystalline form according to clause 42, wherein the crystalline form is, Freebase Type D, characterized by substantially the same X-ray powder diffraction pattern as Figure 23.
44. The crystalline form according to clause 1 or 2, wherein the crystalline form is, Freebase Type E, characterized by an X-ray powder diffraction pattern which comprises at least peaks at 2θ (± 0.2°) of 7.2, 18.2 and 22.3.
45. The crystalline form according to clause 44, wherein the crystalline form is, Freebase Type E, characterized by an X-ray powder diffraction pattern which comprises at least peaks at 2θ (± 0.2°) of 7.2, 18.2, 19.2, 22.3, 23.0 and 24.0.
46. The crystalline form according to clause 45, wherein the crystalline form is, Freebase Type E, characterized by an X-ray powder diffraction pattern which comprises at least peaks at 2θ (± 0.2°) of 7.2, 14.9, 16.7, 18.2, 19.2, 22.3, 23.0, 24.0 and 26.8.
47. The crystalline form according to clause 46, wherein the crystalline form is, Freebase Type E, characterized by an X-ray powder diffraction pattern which comprises at least peaks at 2θ (± 0.2°) of 7.2, 12.6, 14.9, 16.7, 18.2, 19.2, 19.7, 20.5, 22.3, 23.0, 24.0 and 26.8.
48. The crystalline form according to clause 47, wherein the crystalline form is, Freebase Type E, characterized by substantially the same X-ray powder diffraction pattern as Figure 24.
49. The crystalline form according to clause 1 or 2, wherein the crystalline form is, Freebase Type F, characterized by an X-ray powder diffraction pattern which comprises at least peaks at 2θ (± 0.2°) of 6.2, 11.6 and 12.6.
50. The crystalline form according to clause 49, wherein the crystalline form is, Freebase Type F, characterized by an X-ray powder diffraction pattern which comprises at least peaks at 2θ (± 0.2°) of 6.2, 11.6, 12.6, 14.8, 16.5 and 24.4.
51. The crystalline form according to clause 50, wherein the crystalline form is, Freebase Type F, characterized by an X-ray powder diffraction pattern which comprises at least peaks at 2θ (± 0.2°) of 6.2, 11.6, 12.6, 14.8, 16.5, 17.6, 19.3, 24.4 and 26.0.
52. The crystalline form according to clause 51, wherein the crystalline form is, Freebase Type F, characterized by an X-ray powder diffraction pattern which comprises at least peaks at 2θ (± 0.2°) of 6.2, 9.3, 11.6, 12.6, 14.8, 16.5, 17.6, 18.7, 19.3, 24.4 and 26.0.
53. The crystalline form according to clause 52, wherein the crystalline form is, Freebase Type F, by substantially the same X-ray powder diffraction pattern as Figure 25.
54. The crystalline form according to any one of clauses 49-53, characterized by a differential scanning calorimeter peak phase transition temperature of about 55.4 °C and about 109.5 °C.
55. The crystalline form according to clause 1 or 2, wherein the crystalline form is, Freebase Type G, characterized by an X-ray powder diffraction pattern which comprises at least peaks at 2θ (± 0.2°) of 5.7, 5.9 and 12.7.
56. The crystalline form according to clause 55, wherein the crystalline form is, Freebase Type G, characterized by an X-ray powder diffraction pattern which comprises at least peaks at 2θ (± 0.2°) of 5.7, 5.9, 11.9, 12.7, 14.5 and 26.2.
57. The crystalline form according to clause 56, wherein the crystalline form is, Freebase Type G, characterized by an X-ray powder diffraction pattern which comprises at least peaks at 2θ (± 0.2°) of 5.7, 5.9, 11.9, 12.7, 14.5, 17.6, 19.7, 22.9 and 26.2.
58. The crystalline form according to clause 57, wherein the crystalline form is, Freebase Type G, characterized by an X-ray powder diffraction pattern which comprises at least peaks at 2θ (+ 0.2°) of 5.7, 5.9, 11.9, 12.7, 14.5, 17.2, 17.6, 19.7, 20.6, 22.9, 24.8 and 26.2.
59. The crystalline form according to clause 58, wherein the crystalline form is, Freebase Type G, by substantially the same X-ray powder diffraction pattern as Figure 28.
60. The crystalline form according to any one of clauses 55-59, characterized by a differential scanning calorimeter peak phase transition temperature of about 32.9 °C, about 59.2 °C and about 110.2 °C.
61. The crystalline form according to clause 1 or 2, wherein the crystalline form is an acetone solvate, Freebase Type A, characterized by an X-ray powder diffraction which comprises at least peaks at 2θ (± 0.2°) of 7.0, 9.0 and 23.3.
62. The crystalline form according to clause 61, wherein the crystalline form is an acetone solvate, Freebase Type A, characterized by an X-ray powder diffraction which comprises at least peaks at 2θ (± 0.2°) of 7.0, 9.0, 11.6, 13.6, 15.4, 18.1, 19.6 and 23.3.
63. The crystalline form according to clause 62, wherein the crystalline form is an acetone solvate, Freebase Type A, characterized by substantially the same X-ray powder diffraction pattern as Figure 31.
64. The crystalline form according to any one of clauses 61-63, characterized by a differential scanning calorimeter peak phase transition temperature of about 71.3 °C.
65. The crystalline form according to any one of clauses 1-5, wherein the salt is, HCl Salt Type A, characterized by an X-ray powder diffraction which comprises at least peaks at 2θ (± 0.2°) of 18.1.
66. The crystalline form according to clause 65, wherein the salt is, HCl Salt Type A, characterized by substantially the same X-ray powder diffraction pattern shown in Figure 35.
67. The crystalline form according to any one of clauses 65-66, characterized by a differential scanning calorimeter peak phase transition temperature of about 110.0 °C.
68. The crystalline form according to any one of clauses 1-5, wherein the salt is, HCl Salt Type B, characterized by an X-ray powder diffraction pattern which comprises at least peaks at 2θ (± 0.2°) of 6.9, 12.4 and 25.0.
69. The crystalline form according to clause 68, wherein the salt is, HCl Salt Type B, characterized by an X-ray powder diffraction pattern which comprises at least peaks at 2θ (± 0.2°) of 6.9, 11.9, 12.4, 17.0, 25.0 and 29.1.
70. The crystalline form according to clause 69, wherein the salt is, HCl Salt Type B, characterized by an X-ray powder diffraction pattern which comprises at least peaks at 2θ (± 0.2°) of 6.9, 9.6, 11.9, 12.4, 17.0, 21.2, 22.7, 25.0 and 29.1.
71. The crystalline form according to clause 70, wherein the salt is, HCl Salt Type B, characterized by an X-ray powder diffraction pattern which comprises at least peaks at 2θ (± 0.2°) of 6.9, 9.6, 11.9, 12.4, 17.0, 19.9, 21.2, 22.7, 25.0, 25.9, 27.2 and 29.1.
72. The crystalline form according to clause 71, wherein the salt is, HCl Salt Type B, characterized by substantially the same X-ray powder diffraction pattern shown in Figure 38.
73. The crystalline form according to any one of clauses 68-72, characterized by a differential scanning calorimeter peak phase transition temperature of about 241.7 °C.
74. The crystalline form according to any one of clauses 1-5, wherein the salt is, Mesylate Type A, characterized by an X-ray powder diffraction which comprises at least peaks at 2θ (± 0.2°) of 6.5, 19.6 and 21.0.
75. The crystalline form according to clause 74, wherein the salt is, Mesylate Type A, characterized by an X-ray powder diffraction which comprises at least peaks at 2θ (± 0.2°) of 5.3, 6.5, 7.8, 13.1, 15.7, 19.6 and 21.0.
76. The crystalline form according to clause 75, wherein the salt is, Mesylate Type A, characterized by substantially the same X-ray powder diffraction pattern shown in Figure 41.
77. The crystalline form according to any one of clauses 74-76, characterized by a differential scanning calorimeter peak phase transition temperature of about 65.1 °C.
78. The crystalline form according to any one of clauses 1-5, wherein the salt is, Mesylate Type B, characterized by an X-ray powder diffraction which comprises at least peaks at 2θ (± 0.2°) of 6.0, 16.3 and 18.3.
79. The crystalline form according to clause 78, wherein the salt is, Mesylate Type B, characterized by an X-ray powder diffraction which comprises at least peaks at 2θ (± 0.2°) of 6.0, 7.2, 12.4, 16.3, 18.3, 21.5 and 26.5.
80. The crystalline form according to clause 79, wherein the salt is, Mesylate Type B, characterized by substantially the same X-ray powder diffraction pattern shown in Figure 44.
81. The crystalline form according to any one of clauses 78-80, characterized by a differential scanning calorimeter peak phase transition temperature of about 63.4 °C.
82. The crystalline form according to any one of clauses 1-5, wherein the salt is, Phosphate Type A, characterized by an X-ray powder diffraction which comprises at least peaks at 2θ (± 0.2°) of 6.4, 14.0 and 22.9.
83. The crystalline form according to clause 82, wherein the salt is, Phosphate Type A, characterized by an X-ray powder diffraction which comprises at least peaks at 2θ (± 0.2°) of 6.4, 14.0, 14.9, 20.5, 22.9 and 24.5.
84. The crystalline form according to clause 83, wherein the salt is, Phosphate Type A, characterized by an X-ray powder diffraction which comprises at least peaks at 2θ (± 0.2°) of 6.4, 14.0, 14.9, 16.3, 18.7, 20.5, 21.4, 22.9 and 24.5.
85. The crystalline form according to clause 84, wherein the salt is, Phosphate Type A, characterized by substantially the same X-ray powder diffraction pattern shown in Figure 47.
86. The crystalline form according to any one of clauses 82-85, characterized by a differential scanning calorimeter peak phase transition temperature of about 79.1 °C and about 194.8 °C.
87. The crystalline form according to any one of clauses 1-5, wherein, the salt is, L-tartrate Type A, characterized by an X-ray powder diffraction which comprises at least peaks at 2θ (± 0.2°) of 6.5, 12.7 and 18.8.
88. The crystalline form according to clause 87, wherein, the salt is, L-tartrate Type A, characterized by an X-ray powder diffraction which comprises at least peaks at 2θ (± 0.2°) of 6.5, 9.4, 12.7, 18.8, 20.7, 22.7, 24.4 and 26.5.
89. The crystalline form according to clause 88, wherein, the salt is, L-tartrate Type A, characterized by substantially the same X-ray powder diffraction pattern shown in Figure 50.
90. The crystalline form according to any one of clauses 87-89, characterized by a differential scanning calorimeter peak phase transition temperature of about 77.6 °C and about 164.7 °C.
91. The crystalline form according to any one of clauses 1-5, wherein, the salt is, Adipate Type A, characterized by an X-ray powder diffraction which comprises at least peaks at 2θ (± 0.2°) of 7.4, 10.8 and 25.7.
92. The crystalline form according to clause 91, wherein, the salt is, Adipate Type A, characterized by an X-ray powder diffraction which comprises at least peaks at 2θ (± 0.2°) of 7.4, 10.8, 16.0, 17.7, 19.7 and 25.7.
93. The crystalline form according to clause 92, wherein, the salt is, Adipate Type A, characterized by an X-ray powder diffraction which comprises at least peaks at 2θ (± 0.2°) of 7.4, 10.8, 12.6, 16.0, 17.7, 19.7, 20.9, 23.6 and 25.7.
94. The crystalline form according to clause 93, wherein, the salt is, Adipate Type A, characterized by an X-ray powder diffraction which comprises at least peaks at 2θ (± 0.2°) of 7.4, 8.5, 10.8, 12.6, 14.9, 15.5, 16.0, 16.9, 17.7, 19.0, 19.7, 20.9, 23.6, 25.7 and 32.3.
95. The crystalline form according to clause 94, wherein, the salt is, Adipate Type A, characterized by substantially the same X-ray powder diffraction pattern shown in Figure 53.
96. The crystalline form according to any one of clauses 91-95, characterized by a differential scanning calorimeter peak phase transition temperature of about 106.7 °C.
97. An amorphous form of compound (I) represented by the following structural formula: 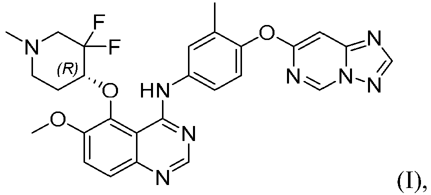 wherein the amorphous form is a pharmaceutically acceptable salt or free base.
98. The amorphous form according to clause 97, wherein the amorphous form is Amorphous Fumarate, characterized by substantially the same X-ray powder diffraction pattern as Figure 56.
99. The amorphous form according to clause 98, characterized by substantially the same modulated differential scanning calorimeter thermogram curve as Figure 58.
100. The amorphous form according to clause 97, wherein the amorphous form is Amorphous Freebase, characterized by substantially the same X-ray powder diffraction pattern as Figure 59.
101. The amorphous form according to clause 100, characterized by substantially the same modulated differential scanning calorimeter thermogram curve as Figure 61.
102. A method for the preparation of the crystalline form according to any one of clauses 1-96, comprising:
   where the crystalline form is a complex of free base with a pharmaceutically acceptable acid,
      a) adding compound (I) and the acid in a solvent, and
      b) slurrying at a temperature for a time sufficient to initiate precipitation of the complex;
   where the crystalline form is free base,
      a) adding compound (I) in a solvent; and
      b) slurrying at a temperature for a time sufficient to initiate precipitation of the free base.
103. The method according to clause 102, wherein the acid is chosen from a group consisting of hydrochloride, methane sulfonic acid, phosphoric acid, tartaric acid, fumaric acid and adipic acid.
104. The method according to clause 102 or 103, wherein in step a), where the crystalline form is a complex of free base with a pharmaceutically acceptable acid, the compound (I) and the acid are added in the solvent at an acid/base molar ratio in a range of 0.5:1 to 3:1; preferably, 0.5:1 to 2.5:1; more preferably, 1:1 to 1.5:1.
105. The method according to any one of clauses 102-104, wherein the solvent is selected from a group consisting of H₂O, EtOH, EtOAc, n-heptane, ethyl formate, acetone, cyclohexane, isopropyl alcohol, methyl isobutyl ketone, tetrahydrofuran, acetonitrile methyl tert-butyl ether and combination thereof.
106. The method according to any one of clauses 102-105, further comprises seeding the solvent with a crystalline form of any one of clauses 1-96.
107. The method according to any one of clauses 102-106, wherein the temperature is about 5~50 °C.
108. The method according to any one of clauses 102-107, wherein the time is about 2~7.5 hours.
109. A method for the preparation of Fumarate Type A crystalline form of compound (I) according to any one of clauses 7-12, comprising:
   a) dissolving free base of compound (I) in an ester or an alcohol to form a free base solution;
   b) dissolving fumaric acid in EtOH to form an acid solution;
   c) adding the acid solution into the free base solution dropwise while stirring;
   d) adding an alkane dropwise; then optionally seeding the mixture with a Fumarate Type A crystalline form of compound (I);
   e) stirring at 0-10 °C for 12-24 hours; and
   f) isolating a solid via filtration and then vacuum drying the solid at 40-60 °C.
110. The method according to clause 109, wherein the ester is selected from a group consisting of ethyl acetate, ethyl formate, methyl acetate and isopropyl acetate.
111. The method according to clause 110, wherein the ester is ethyl acetate.
112. The method according to clause 109, wherein the alcohol is selected from a group consisting of methanol, ethanol, n-propanol and isopropanol.
113. The method according to clause 112, wherein the alcohol is ethanol.
114. The method according to clause 109, wherein the alkane is selected from a group consisting of n-hexane, n-heptane, n-octane and a mixture thereof.
115. The method according to clause 114, wherein the alkane is n-heptane.
116. A method for the preparation of Fumarate Type B crystalline form of compound (I) according to any one of clauses 13-17, comprising:
   a) suspending a Fumarate Type A crystalline form of compound (I) in H₂O;
   b) magnetically stirring at a speed of about 1000 rpm at about room temperature for about 11 days; and
   c) isolating a solid via centrifugation and storing the solid at ambient conditions openly for about 4 days.
117. A method for the preparation of Fumarate Type C crystalline form of compound (I) according to any one of clauses 18-21, comprising:
   a) suspending a Fumarate Type A crystalline form of compound (I) in H₂O;
   b) magnetically stirring at a speed of about 1000 rpm at about room temperature for about 9 days; and
   c) isolating a wet solid.
118. A method for the preparation of Fumarate Type E crystalline form of compound (I) according to any one of clauses 22-27, comprising:
   a) dissolving a Fumarate Type crystalline form of compound (I) in ethyl formate;
   b) evaporating the ethyl formate at about room temperature;
   c) isolating a solid.
119. A method for the preparation of Freebase Type A crystalline form of compound (I) according to any one of clauses61-64, comprising:
   a) suspending amorphous free base of compound (I) in a solvent of acetone/n-heptane with a volume ratio of about 1:4;
   b) magnetically stirring at a speed of about 1000 rpm at about room temperature for about 3 days; and
   c) isolating a solid via centrifugation and drying the solid at ambient conditions for about 1 day.
120. A method for the preparation of Freebase Type B crystalline form of compound (I) according to any one of clauses 28-33, comprising:
   a) suspending amorphous free base of compound (I) in a solvent of methyl isobutyl ketone/Cyclohexane with a volume ratio of about 1:4;
   b) magnetically stirring at a speed of about 1000 rpm at about 5 °C for about 7 days; and
   c) isolating a solid via centrifugation.
121. A method for the preparation of Freebase Type C crystalline form of compound (I) according to any one of clauses 34-39, comprising:
   a) suspending amorphous free base of compound (I) in a solvent of tetrahydrofuran/H₂O with a volume ratio of about 1:4;
   b) magnetically stirring at a speed of about 1000 rpm at about 5 °C for about 7 days; and
   c) isolating a solid via centrifugation.
122. A method for the preparation of Freebase Type D crystalline form of compound (I) according to any one of clauses 40-43, comprising:
   a) suspending amorphous free base of compound (I) in a solvent of tetrahydrofuran/H₂O with a volume ratio of about 1:4;
   b) magnetically stirring at a speed of about 1000 rpm at about 5 °C for about 3 days; and
   c) isolating a solid via centrifugation and drying the solid at ambient condition for about 2 hours.
123. A method for the preparation of Freebase Type E crystalline form of compound (I) according to any one of clauses 44-48, comprising:
   a) suspending amorphous free base of compound (I) in a solvent of tetrahydrofuran/H₂O with a volume ratio of about 1:4;
   b) magnetically stirring at a speed of about 1000 rpm at about 5 °C for about 4 days;
   c) isolating a solid via centrifugation and drying the solid at ambient condition for about 2 hours; and
   d) sweeping the solid by N₂ for about 20 min at about 30 °C.
124. A method for the preparation of Freebase Type F crystalline form of compound (I) according to any one of clauses 49-54, comprising:
   a) suspending amorphous free base of compound (I) in a solvent of acetonitrile/n-heptane;
   b) magnetically stirring at a speed of about 1000 rpm at about room temperature for about 2 days;
   c) isolating a solid.
125. A method for the preparation of Freebase Type G crystalline form of compound (I) according to any one of clauses 55-60, comprising:
   a) dissolving amorphous free base of compound (I) in EtOH;
   b) adding H₂O and obtaining a suspension;
   c) isolating a solid from the suspension.
126. A method for the preparation of HCl salt Type A of compound (I) according to any one of clauses 65-67, comprising:
   a) adding amorphous free base of compound (I) and a concentrated HCl at an acid/base molar ratio of about 2:1 in a solvent of EtOAc/n-heptane with a volume ratio of about 1:2;
   b) magnetically stirring at a speed of about 1000 rpm at about room temperature for about 3 days; and
   c) isolating a solid via centrifugation and drying the solid at ambient conditions for about 1 day.
127. A method for the preparation of HCl salt Type B of compound (I) according to any one of clauses 68-73, comprising:
   a) dissolving amorphous free base of compound (I) in EtOAc to form a free base solution;
   b) diluting a EtOAc solution of HCl in EtOH to form an acid solution;
   c) optionally adding a seed of HCl salt Type B of compound (I) into the free base solution wherein the seed is not completely dissolved;
   d) adding the acid solution dropwise while stirring at a speed of about 1000 rpm;
   e) further stirring at room temperature for about 8 hours, then about 5 °C for about 13 hours;
   f) isolating a solid via filtration, then vacuum drying the solid at about room temperature overnight.;
   wherein the molar ratio of acid/base is about 2:1.
128. A method for the preparation of Mesylate Type A of compound (I) according to any one of clauses 74-77, comprising:
   a) suspending amorphous free base of compound (I) and a methanesulfonic acid at an acid/base molar ratio of about 2:1 in a solvent of acetone/n-heptane with a volume ratio of about 1:4;
   b) magnetically stirring at a speed of about 1000 rpm at about room temperature for about 3 days; and
   c) isolating a solid via centrifugation and drying the solid at ambient conditions for about 1 day.
129. A method for the preparation of Mesylate Type B of compound (I) according to any one of clauses 78-81, comprising:
   a) suspending amorphous free base of compound (I) and a methanesulfonic acid at an acid/base charging molar ratio of about 2:1 in a solvent of isopropyl alcohol/cyclohexane with a volume ratio of about 1:4;
   b) magnetically stirring at a speed of about 1000 rpm at about room temperature for about 3 days; and
   c) isolating a solid via centrifugation and drying the solid at ambient conditions for about 1 day.
130. A method for the preparation of Phosphate Type A of compound (I) according to any one of clauses 82-86, comprising:
   a) suspending amorphous free base of compound (I) and concentrated H₃PO₄ at an acid/base molar ratio of about 1:1 in a solvent of acetone/n-heptane with a volume ratio of about 1:4;
   b) magnetically stirring at a speed of about 1000 rpm at room temperature for about 3 days; and
   c) isolating a solid via centrifugation and drying the solid at ambient conditions for about 1 day.
131. A method for the preparation of L-Tartrate Type A of compound (I) according to any one of clauses 87-90, comprising:
   a) suspending amorphous free base of compound (I) and a L-tartaric acid at an acid/base molar ratio of about 1:1 in a solvent of EtOAc/n-heptane with a volume ratio of about 1:2;
   b) magnetically stirring at a speed of about 1000 rpm at about room temperature for about 3 days; and
   c) isolating a solid via centrifugation and drying the solid at ambient conditions for about 1 day.
132. A method for the preparation of Adipate Type A of compound (I) according to any one of clauses 91-96, comprising:
   a) suspending amorphous free base of compound (I) and an adipic acid at an acid/base molar ratio of about 1:1 in a solvent of EtOAc/n-heptane with a volume ratio of about 1:2;
   b) magnetically stirring at a speed of about 1000 rpm at about room temperature for about 3 days; and
   c) isolating a solid via centrifugation and drying the solid at ambient conditions for about 1 day.
133. A method for the preparation of an amorphous form of compound (I) according to any one of clauses 97-101, comprising:
   a) dissolving compound (I) in a solvent; and
   b) removing the solvent.
134. The method according to clause 133, wherein the solvent is selected from a group consisting of H₂O, EtOH, EtOAc, n-heptane, ethyl formate, acetone, cyclohexane, isopropyl alcohol, methyl isobutyl ketone, tetrahydrofuran, acetonitrile, methyl tert-butyl ether and combination thereof.
135. A method for the preparation of Amorphous Fumarate of compound (I) according to clause 98 or 99, comprising:
   a) dissolving Fumarate Type A of compound (I) in MeOH; and
   b) removing the MeOH by rotatory evaporation at about 60 °C.
136. A method for the preparation of Amorphous Freebase of compound (I) according to clause 100 or 101, comprising:
   a) dissolving Freebase Type B of compound (I) in DCM; and
   b) removing the DCM by rotatory evaporation at about 40 °C.
137. A pharmaceutical composition comprising the crystalline form according to any one of clauses 1-96 or the amorphous form according to any one of clauses 97-101, and a pharmaceutically acceptable carrier or excipient.
138. A dosage form comprising a therapeutically effective amount of the crystalline form according to any one of clauses 1-96, the amorphous form according to any one of clauses 97-101, or the pharmaceutical composition according to clause 137.
139. A method of treating or ameliorating a hyperproliferative disease in a subject, comprising administering to the subject in need thereof a therapeutically effective amount of the crystalline form according to any one of clauses 1-96 or the amorphous form according to any one of clauses 97-101.
140. The method according to clause 139, wherein the hyperproliferative disease is cancer.
141. The method according to clause 140, wherein the cancer is ErbB2 positive.
142. The method according to clause 140 or 141, wherein the cancer is selected from a group consisting of breast, gastric, biliary, colorectal, brain, lung, NSCLC, pancreatic, head and neck, ovarian and uterine cancer.
143. The method according to any one of clauses 139 to 142, wherein one or more additional compounds having anti-cancer properties are administered in combination.
144. The crystalline form according to any one of clauses 1-96 or amorphous form according to any one of clauses 97-101 for use in treating or ameliorating a hyperproliferative disease.
145. The crystalline form or amorphous form according to clause 144, wherein the hyperproliferative disease is cancer.
146. The crystalline form or amorphous form according to clause 145, wherein the cancer is ErbB2 positive.
147. The crystalline form or amorphous form according to clause 145 or 146, wherein the cancer is selected from breast, gastric, biliary, colorectal, brain, lung, NSCLC, pancreatic, head and neck, ovarian and uterine cancer.
148. The crystalline form or amorphous form according to any one of clauses 144 to 147, wherein one or more additional compounds having anti-cancer properties are administered in combination.
149. Use of the crystalline form according to any one of clauses 1-96 or amorphous form according to any one of clauses 97-101, in the manufacture of a medicament for treating or ameliorating a hyperproliferative disease.
150. The use according to clause 149, wherein the hyperproliferative disease is cancer.
151. The use according to clause 150, wherein the cancer is ErbB2 positive.
152. The use according to clause 150 or 151, wherein the cancer is selected from a group consisting of breast, gastric, biliary, colorectal, brain, lung, NSCLC, pancreatic, head and neck, ovarian and uterine cancer.
153. The use according to any one of clauses 149 to 152, wherein one or more anti-tumor agents are administered in combination.

## Claims

1. A crystalline form of compound (I) represented by the following structural formula: 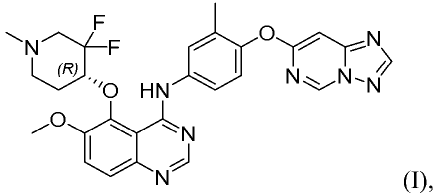 wherein the crystalline form is a complex of free base with a pharmaceutically acceptable acid, or free base.

2. The crystalline form according to claim 1, wherein:
(a) the complex or free base is a solvate or non-solvate;
(b) the complex is a salt or a cocrystal or a cocrystal of salt;
(c) the complex has an acid/base molar ratio of 0.5:1 to 3:1, preferably, 0.5:1 to 2.5:1, more preferably, 1:1 to 1.5:1;
(d) the pharmaceutically acceptable acid is chosen from a group consisting of hydrochloride, methanesulfonic acid, phosphoric acid, tartaric acid, fumaric acid and adipic acid; and/or
(e) the pharmaceutically acceptable acid is fumaric acid.

3. The crystalline form according to claim 2(e), wherein the crystalline form is:
(a) Fumarate Type A, **characterized by**:
(i) a differential scanning calorimeter peak phase transition temperature of about 167.6 °C; and
(ii) an X-ray powder diffraction pattern which comprises at least peaks at 2θ (± 0.2°) of 6.9 and 11.5, optionally
an X-ray powder diffraction pattern which comprises at least peaks at 2θ (± 0.2°) of 5.8, 6.9, 11.5, 12.1 and 17.7, further optionally
an X-ray powder diffraction pattern which comprises at least peaks at 2θ (± 0.2°) of 5.8, 6.9, 11.5, 12.1, 17.7, 20.8 and 24.0, further optionally
an X-ray powder diffraction pattern which comprises at least peaks at 2θ (± 0.2°) of 5.8, 6.9, 11.5, 12.1, 17.7, 18.9, 20.8, 23.1, 23.7, 24.0 and 28.8, further optionally
substantially the same X-ray powder diffraction pattern as Figure 1; or
(b) Fumarate Type B, **characterized by**:
(i) a differential scanning calorimeter peak phase transition temperature of about 91.3 °C and about 166.3 °C; and
(ii) an X-ray powder diffraction pattern which comprises at least peaks at 2θ (± 0.2°) of 6.6 and 11.4, optionally
an X-ray powder diffraction pattern which comprises at least peaks at 2θ (± 0.2°) of 6.6, 10.7, 11.4, 12.9, 25.1 and 28.2, further optionally
an X-ray powder diffraction pattern which comprises at least peaks at 2θ (± 0.2°) of 6.6, 10.7, 11.4, 12.9, 15.8, 17.9, 19.7, 25.1 and 28.2, further optionally
substantially the same X-ray powder diffraction pattern as Figure 6; or
(c) Fumarate Type C, **characterized by**:
an X-ray powder diffraction pattern which comprises at least peaks at 2θ (± 0.2°) of 6.8 and 11.8, optionally
an X-ray powder diffraction pattern which comprises at least peaks at 2θ (± 0.2°) of 6.8, 11.2, 11.8, 13.6 and 18.4, further optionally
an X-ray powder diffraction pattern which comprises at least peaks at 2θ (± 0.2°) of 6.8, 11.2, 11.8, 13.6, 15.1, 16.0, 17.2, 18.4 and 24.5, further optionally
substantially the same X-ray powder diffraction pattern as Figure 10; or
(d) Fumarate Type E, **characterized by**:
(i) a differential scanning calorimeter peak phase transition temperature of about 134.5 °C and about 166.0 °C; and
(ii) an X-ray powder diffraction pattern which comprises at least peaks at 2θ (± 0.2°) of 6.7, 11.6 and 28.5, optionally
an X-ray powder diffraction pattern which comprises at least peaks at 2θ (+ 0.2°) of 6.7, 10.9, 11.6, 16.9, 25.2 and 28.5, further optionally
an X-ray powder diffraction pattern which comprises at least peaks at 2θ (+ 0.2°) of 6.7, 10.9, 11.6, 12.9, 15.3, 16.9, 19.9, 25.2 and 28.5, further optionally
an X-ray powder diffraction pattern which comprises at least peaks at 2θ (+ 0.2°) of 5.5, 6.7, 10.9, 11.6, 12.9, 15.3, 16.9, 18.1, 19.9, 25.2, 27.5 and 28.5, further optionally
substantially the same X-ray powder diffraction pattern as Figure 11.

4. The crystalline form according to claim 1 or 2(a), wherein the crystalline form is:
(a) Free base Type B, **characterized by**:
(i) a differential scanning calorimeter peak phase transition temperature of about 169.4 °C; and
(ii) an X-ray powder diffraction pattern which comprises at least peaks at 2θ (± 0.2°) of 8.0 and 11.5, optionally
an X-ray powder diffraction pattern which comprises at least peaks at 2θ (± 0.2°) of 8.0, 11.5, 16.0, 17.2, 18.8 and 24.3, further optionally
an X-ray powder diffraction pattern which comprises at least peaks at 2θ (± 0.2°) of 8.0, 11.5, 16.0, 17.2, 18.2, 18.8, 20.3, 21.9 and 24.3, further optionally
an X-ray powder diffraction pattern which comprises at least peaks at 2θ (± 0.2°) of 8.0, 11.5, 13.1, 16.0, 17.2, 18.2, 18.8, 20.3, 21.2, 21.9, 24.3 and 27.7, further optionally
substantially the same X-ray powder diffraction pattern as Figure 14; or
(b) Freebase Type C, **characterized by**:
(i) a differential scanning calorimeter peak phase transition temperature of about 86.2 °C and about 114.4 °C; and
(ii) an X-ray powder diffraction pattern which comprises at least peaks at 2θ (± 0.2°) of 6.6 and 18.8, optionally
an X-ray powder diffraction pattern which comprises at least peaks at 2θ (± 0.2°) of 6.6, 12.7, 18.8, 20.7 and 24.4, further optionally
an X-ray powder diffraction pattern which comprises at least peaks at 2θ (± 0.2°) of 6.6, 12.7, 14.1, 18.1, 18.8, 20.7, 23.4, 24.4 and 26.7, further optionally
an X-ray powder diffraction pattern which comprises at least peaks at 2θ (± 0.2°) of 6.6, 9.4, 12.7, 14.1, 14.9, 18.1, 18.8, 20.7, 22.9, 23.4, 24.4 and 26.7, further optionally
substantially the same X-ray powder diffraction pattern as Figure 19; or
(c) Freebase Type D, **characterized by** an X-ray powder diffraction pattern which comprises at least peaks at 2θ (± 0.2°) of 5.7, 5.8 and 18.8, optionally
an X-ray powder diffraction pattern which comprises at least peaks at 2θ (± 0.2°) of 5.7, 5.8, 11.8, 12.6, 18.8, 20.6 and 24.3, further optionally
an X-ray powder diffraction pattern which comprises at least peaks at 2θ (± 0.2°) of 5.7, 5.8, 11.8, 12.6, 18.8, 20.6, 22.8, 23.3 and 24.3, further optionally
substantially the same X-ray powder diffraction pattern as Figure 23; or
(d) Freebase Type E, **characterized by** an X-ray powder diffraction pattern which comprises at least peaks at 2θ (+ 0.2°) of 7.2, 18.2 and 22.3, optionally
an X-ray powder diffraction pattern which comprises at least peaks at 2θ (+ 0.2°) of 7.2, 18.2, 19.2, 22.3, 23.0 and 24.0, further optionally
an X-ray powder diffraction pattern which comprises at least peaks at 2θ (± 0.2°) of 7.2, 14.9, 16.7, 18.2, 19.2, 22.3, 23.0, 24.0 and 26.8, further optionally
an X-ray powder diffraction pattern which comprises at least peaks at 2θ (± 0.2°) of 7.2, 12.6, 14.9, 16.7, 18.2, 19.2, 19.7, 20.5, 22.3, 23.0, 24.0 and 26.8, further optionally
substantially the same X-ray powder diffraction pattern as Figure 24; or
(e) Freebase Type F, **characterized by**:
(i) a differential scanning calorimeter peak phase transition temperature of about 55.4 °C and about 109.5 °C; and
(ii) an X-ray powder diffraction pattern which comprises at least peaks at 2θ (± 0.2°) of 6.2, 11.6 and 12.6, optionally
an X-ray powder diffraction pattern which comprises at least peaks at 2θ (± 0.2°) of 6.2, 11.6, 12.6, 14.8, 16.5 and 24.4, further optionally
an X-ray powder diffraction pattern which comprises at least peaks at 2θ (± 0.2°) of 6.2, 11.6, 12.6, 14.8, 16.5, 17.6, 19.3, 24.4 and 26.0, further optionally
an X-ray powder diffraction pattern which comprises at least peaks at 2θ (± 0.2°) of 6.2, 9.3, 11.6, 12.6, 14.8, 16.5, 17.6, 18.7, 19.3, 24.4 and 26.0, further optionally
substantially the same X-ray powder diffraction pattern as Figure 25; or
(f) Freebase Type G, **characterized by**:
(i) a differential scanning calorimeter peak phase transition temperature of about 32.9 °C, about 59.2 °C and about 110.2 °C; and
(ii) an X-ray powder diffraction pattern which comprises at least peaks at 2θ (± 0.2°) of 5.7, 5.9 and 12.7, optionally
an X-ray powder diffraction pattern which comprises at least peaks at 2θ (± 0.2°) of 5.7, 5.9, 11.9, 12.7, 14.5 and 26.2, further optionally
an X-ray powder diffraction pattern which comprises at least peaks at 2θ (± 0.2°) of 5.7, 5.9, 11.9, 12.7, 14.5, 17.6, 19.7, 22.9 and 26.2, further optionally
an X-ray powder diffraction pattern which comprises at least peaks at 2θ (± 0.2°) of 5.7, 5.9, 11.9, 12.7, 14.5, 17.2, 17.6, 19.7, 20.6, 22.9, 24.8 and 26.2, further optionally
substantially the same X-ray powder diffraction pattern as Figure 28; or
(g) an acetone solvate, Freebase Type A, **characterized by**:
(i) a differential scanning calorimeter peak phase transition temperature of about 71.3 °C; and
(ii) an X-ray powder diffraction which comprises at least peaks at 2θ (± 0.2°) of 7.0, 9.0 and 23.3, optionally
an X-ray powder diffraction which comprises at least peaks at 2θ (± 0.2°) of 7.0, 9.0, 11.6, 13.6, 15.4, 18.1, 19.6 and 23.3, further optionally
substantially the same X-ray powder diffraction pattern as Figure 31; or

5. The crystalline form according to any one of claims 1 or 2(a)-(d), wherein the salt is:
(a) HCl Salt Type A, **characterized by**:
(i) a differential scanning calorimeter peak phase transition temperature of about 110.0 °C; and
(ii) an X-ray powder diffraction which comprises at least peaks at 2θ (± 0.2°) of 18.1, optionally
substantially the same X-ray powder diffraction pattern shown in Figure 35; or
(b) HCl Salt Type B, **characterized by**:
(i) a differential scanning calorimeter peak phase transition temperature of about 241.7 °C; and
(ii) an X-ray powder diffraction pattern which comprises at least peaks at 2θ (± 0.2°) of 6.9, 12.4 and 25.0, optionally
an X-ray powder diffraction pattern which comprises at least peaks at 2θ (± 0.2°) of 6.9, 11.9, 12.4, 17.0, 25.0 and 29.1, further optionally
an X-ray powder diffraction pattern which comprises at least peaks at 2θ (± 0.2°) of 6.9, 9.6, 11.9, 12.4, 17.0, 21.2, 22.7, 25.0 and 29.1, further optionally
an X-ray powder diffraction pattern which comprises at least peaks at 2θ (± 0.2°) of 6.9, 9.6, 11.9, 12.4, 17.0, 19.9, 21.2, 22.7, 25.0, 25.9, 27.2 and 29.1, further optionally
substantially the same X-ray powder diffraction pattern shown in Figure 38; or
(c) Mesylate Type A, **characterized by**:
(i) a differential scanning calorimeter peak phase transition temperature of about 65.1 °C; and
(ii) an X-ray powder diffraction which comprises at least peaks at 2θ (± 0.2°) of 6.5, 19.6 and 21.0, optionally
an X-ray powder diffraction which comprises at least peaks at 2θ (± 0.2°) of 5.3, 6.5, 7.8, 13.1, 15.7, 19.6 and 21.0, further optionally
substantially the same X-ray powder diffraction pattern shown in Figure 41; or
(d) Mesylate Type B, **characterized by**:
(i) a differential scanning calorimeter peak phase transition temperature of about 63.4 °C; and
(ii) an X-ray powder diffraction which comprises at least peaks at 2θ (± 0.2°) of 6.0, 16.3 and 18.3, optionally
an X-ray powder diffraction which comprises at least peaks at 2θ (+ 0.2°) of 6.0, 7.2, 12.4, 16.3, 18.3, 21.5 and 26.5, further optionally
substantially the same X-ray powder diffraction pattern shown in Figure 44; or
(e) Phosphate Type A, **characterized by**:
(i) a differential scanning calorimeter peak phase transition temperature of about 79.1 °C and about 194.8 °C; and
(ii) an X-ray powder diffraction which comprises at least peaks at 2θ (± 0.2°) of 6.4, 14.0 and 22.9, optionally
an X-ray powder diffraction which comprises at least peaks at 2θ (± 0.2°) of 6.4, 14.0, 14.9, 20.5, 22.9 and 24.5, further optionally
an X-ray powder diffraction which comprises at least peaks at 2θ (± 0.2°) of 6.4, 14.0, 14.9, 16.3, 18.7, 20.5, 21.4, 22.9 and 24.5, further optionally
substantially the same X-ray powder diffraction pattern shown in Figure 47; or
(f) L-tartrate Type A, **characterized by**:
(i) a differential scanning calorimeter peak phase transition temperature of about 77.6 °C and about 164.7 °C; and
(ii) an X-ray powder diffraction which comprises at least peaks at 2θ (± 0.2°) of 6.5, 12.7 and 18.8, optionally
an X-ray powder diffraction which comprises at least peaks at 2θ (± 0.2°) of 6.5, 9.4, 12.7, 18.8, 20.7, 22.7, 24.4 and 26.5, further optionally
substantially the same X-ray powder diffraction pattern shown in Figure 50; or
(g) Adipate Type A, **characterized by**:
(i) a differential scanning calorimeter peak phase transition temperature of about 106.7 °C; and
(ii) an X-ray powder diffraction which comprises at least peaks at 2θ (± 0.2°) of 7.4, 10.8 and 25.7, optionally
an X-ray powder diffraction which comprises at least peaks at 2θ (± 0.2°) of 7.4, 10.8, 16.0, 17.7, 19.7 and 25.7, further optionally
an X-ray powder diffraction which comprises at least peaks at 2θ (± 0.2°) of 7.4, 10.8, 12.6, 16.0, 17.7, 19.7, 20.9, 23.6 and 25.7, further optionally
an X-ray powder diffraction which comprises at least peaks at 2θ (± 0.2°) of 7.4, 8.5, 10.8, 12.6, 14.9, 15.5, 16.0, 16.9, 17.7, 19.0, 19.7, 20.9, 23.6, 25.7 and 32.3, further optionally
substantially the same X-ray powder diffraction pattern shown in Figure 53.

6. An amorphous form of compound (I) represented by the following structural formula: 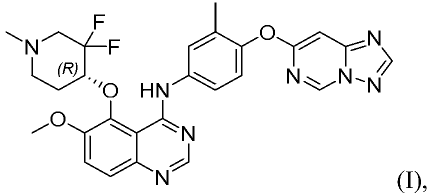 wherein the amorphous form is a pharmaceutically acceptable salt or free base.

7. The amorphous form according to claim 6, wherein the amorphous form is
(a) Amorphous Fumarate, **characterized by** substantially the same X-ray powder diffraction pattern as Figure 56, optionally
**characterized by** substantially the same modulated differential scanning calorimeter thermogram curve as Figure 58; or
(b) Amorphous Freebase, **characterized by** substantially the same X-ray powder diffraction pattern as Figure 59, optionally
**characterized by** substantially the same modulated differential scanning calorimeter thermogram curve as Figure 61.

8. A method for the preparation of the crystalline form according to any one of claims 1-5, comprising:
where the crystalline form is a complex of free base with a pharmaceutically acceptable acid,
a) adding compound (I) and the acid in a solvent, and
b) slurrying at a temperature for a time sufficient to initiate precipitation of the complex;
where the crystalline form is free base,
a) adding compound (I) in a solvent; and
b) slurrying at a temperature for a time sufficient to initiate precipitation of the free base;
optionally wherein:
(a) the acid is chosen from a group consisting of hydrochloride, methane sulfonic acid, phosphoric acid, tartaric acid, fumaric acid and adipic acid;
(b) in step a), where the crystalline form is a complex of free base with a pharmaceutically acceptable acid, the compound (I) and the acid are added in the solvent at an acid/base molar ratio in a range of 0.5:1 to 3:1; preferably, 0.5:1 to 2.5:1; more preferably, 1:1 to 1.5:1;
(c) the solvent is selected from a group consisting of H₂O, EtOH, EtOAc, n-heptane, ethyl formate, acetone, cyclohexane, isopropyl alcohol, methyl isobutyl ketone, tetrahydrofuran, acetonitrile methyl tert-butyl ether and combination thereof;
(d) the method further comprises seeding the solvent with a crystalline form of any one of claims 1-96;
(e) the temperature is about 5~50 °C; and/or
(f) the time is about 2~7.5 hours.

9. A method for the preparation of Fumarate Type A crystalline form of compound (I) according to claim 3(a), comprising:
a) dissolving free base of compound (I) in an ester or an alcohol to form a free base solution;
b) dissolving fumaric acid in EtOH to form an acid solution;
c) adding the acid solution into the free base solution dropwise while stirring;
d) adding an alkane dropwise; then optionally seeding the mixture with a Fumarate Type A crystalline form of compound (I);
e) stirring at 0-10 °C for 12-24 hours; and
f) isolating a solid via filtration and then vacuum drying the solid at 40-60 °C;
optionally wherein:
(a) the ester is selected from a group consisting of ethyl acetate, ethyl formate, methyl acetate and isopropyl acetate, optionally wherein the ester is ethyl acetate;
(b) the alcohol is selected from a group consisting of methanol, ethanol, n-propanol and isopropanol, optionally wherein the alcohol is ethanol; or
(c) the alkane is selected from a group consisting of n-hexane, n-heptane, n-octane and a mixture thereof, optionally wherein the alkane is n-heptane.

10. A method for the preparation of:
(a) Fumarate Type B crystalline form of compound (I) according to claim 3(b), comprising:
a) suspending a Fumarate Type A crystalline form of compound (I) in H₂O;
b) magnetically stirring at a speed of about 1000 rpm at about room temperature for about 11 days; and
c) isolating a solid via centrifugation and storing the solid at ambient conditions openly for about 4 days; or
(b) Fumarate Type C crystalline form of compound (I) according to claim 3(c), comprising:
a) suspending a Fumarate Type A crystalline form of compound (I) in H₂O;
b) magnetically stirring at a speed of about 1000 rpm at about room temperature for about 9 days; and
c) isolating a wet solid; or
(c) Fumarate Type E crystalline form of compound (I) according to claim 3(d), comprising:
a) dissolving a Fumarate Type crystalline form of compound (I) in ethyl formate;
b) evaporating the ethyl formate at about room temperature;
c) isolating a solid.

11. A method for the preparation of:
(a) Freebase Type A crystalline form of compound (I) according to claim 4(g), comprising:
a) suspending amorphous free base of compound (I) in a solvent of acetone/n-heptane with a volume ratio of about 1:4;
b) magnetically stirring at a speed of about 1000 rpm at about room temperature for about 3 days; and
c) isolating a solid via centrifugation and drying the solid at ambient conditions for about 1 day; or
(b) Freebase Type B crystalline form of compound (I) according to claim 4(a), comprising:
a) suspending amorphous free base of compound (I) in a solvent of methyl isobutyl ketone/Cyclohexane with a volume ratio of about 1:4;
b) magnetically stirring at a speed of about 1000 rpm at about 5 °C for about 7 days; and
c) isolating a solid via centrifugation; or
(c) Freebase Type C crystalline form of compound (I) according to claim 4(b), comprising:
a) suspending amorphous free base of compound (I) in a solvent of tetrahydrofuran/H₂O with a volume ratio of about 1:4;
b) magnetically stirring at a speed of about 1000 rpm at about 5 °C for about 7 days; and
c) isolating a solid via centrifugation; or
(d) Freebase Type D crystalline form of compound (I) according to claim 4(c), comprising:
a) suspending amorphous free base of compound (I) in a solvent of tetrahydrofuran/H₂O with a volume ratio of about 1:4;
b) magnetically stirring at a speed of about 1000 rpm at about 5 °C for about 3 days; and
c) isolating a solid via centrifugation and drying the solid at ambient condition for about 2 hours; or
(e) Freebase Type E crystalline form of compound (I) according to claim 4(d), comprising:
a) suspending amorphous free base of compound (I) in a solvent of tetrahydrofuran/H₂O with a volume ratio of about 1:4;
b) magnetically stirring at a speed of about 1000 rpm at about 5 °C for about 4 days;
c) isolating a solid via centrifugation and drying the solid at ambient condition for about 2 hours; and
d) sweeping the solid by N₂ for about 20 min at about 30 °C; or
(f) Freebase Type F crystalline form of compound (I) according to claim 4(e), comprising:
a) suspending amorphous free base of compound (I) in a solvent of acetonitrile/n-heptane;
b) magnetically stirring at a speed of about 1000 rpm at about room temperature for about 2 days;
c) isolating a solid; or
(g) Freebase Type G crystalline form of compound (I) according to claim 4(f), comprising:
a) dissolving amorphous free base of compound (I) in EtOH;
b) adding H₂O and obtaining a suspension;
c) isolating a solid from the suspension.

12. A method for the preparation of:
(a) HCl salt Type A of compound (I) according to claim 5(a), comprising:
a) adding amorphous free base of compound (I) and a concentrated HCl at an acid/base molar ratio of about 2:1 in a solvent of EtOAc/n-heptane with a volume ratio of about 1:2;
b) magnetically stirring at a speed of about 1000 rpm at about room temperature for about 3 days; and
c) isolating a solid via centrifugation and drying the solid at ambient conditions for about 1 day; or
(b) HCl salt Type B of compound (I) according to claim 5(b), comprising:
a) dissolving amorphous free base of compound (I) in EtOAc to form a free base solution;
b) diluting a EtOAc solution of HCl in EtOH to form an acid solution;
c) optionally adding a seed of HCl salt Type B of compound (I) into the free base solution wherein the seed is not completely dissolved;
d) adding the acid solution dropwise while stirring at a speed of about 1000 rpm;
e) further stirring at room temperature for about 8 hours, then about 5 °C for about 13 hours;
f) isolating a solid via filtration, then vacuum drying the solid at about room temperature overnight.;
wherein the molar ratio of acid/base is about 2:1; or
(c) Mesylate Type A of compound (I) according to claim 5(c), comprising:
a) suspending amorphous free base of compound (I) and a methanesulfonic acid at an acid/base molar ratio of about 2:1 in a solvent of acetone/n-heptane with a volume ratio of about 1:4;
b) magnetically stirring at a speed of about 1000 rpm at about room temperature for about 3 days; and
c) isolating a solid via centrifugation and drying the solid at ambient conditions for about 1 day; or
(d) Mesylate Type B of compound (I) according to claim 5(d), comprising:
a) suspending amorphous free base of compound (I) and a methanesulfonic acid at an acid/base charging molar ratio of about 2:1 in a solvent of isopropyl alcohol/cyclohexane with a volume ratio of about 1:4;
b) magnetically stirring at a speed of about 1000 rpm at about room temperature for about 3 days; and
c) isolating a solid via centrifugation and drying the solid at ambient conditions for about 1 day; or
(e) Phosphate Type A of compound (I) according to claim 5(e), comprising:
a) suspending amorphous free base of compound (I) and concentrated H₃PO₄ at an acid/base molar ratio of about 1:1 in a solvent of acetone/n-heptane with a volume ratio of about 1:4;
b) magnetically stirring at a speed of about 1000 rpm at room temperature for about 3 days; and
c) isolating a solid via centrifugation and drying the solid at ambient conditions for about 1 day; or
(f) L-Tartrate Type A of compound (I) according to claim 5(f), comprising:
a) suspending amorphous free base of compound (I) and a L-tartaric acid at an acid/base molar ratio of about 1:1 in a solvent of EtOAc/n-heptane with a volume ratio of about 1:2;
b) magnetically stirring at a speed of about 1000 rpm at about room temperature for about 3 days; and
c) isolating a solid via centrifugation and drying the solid at ambient conditions for about 1 day; or
(g) Adipate Type A of compound (I) according to claim 5(g), comprising:
a) suspending amorphous free base of compound (I) and an adipic acid at an acid/base molar ratio of about 1:1 in a solvent of EtOAc/n-heptane with a volume ratio of about 1:2;
b) magnetically stirring at a speed of about 1000 rpm at about room temperature for about 3 days; and
c) isolating a solid via centrifugation and drying the solid at ambient conditions for about 1 day; or
(h) an amorphous form of compound (I) according to any one of claims 6-7, comprising:
a) dissolving compound (I) in a solvent; and
b) removing the solvent,
optionally wherein the solvent is selected from a group consisting of H₂O, EtOH, EtOAc, n-heptane, ethyl formate, acetone, cyclohexane, isopropyl alcohol, methyl isobutyl ketone, tetrahydrofuran, acetonitrile, methyl tert-butyl ether and combination thereof; or
(j) Amorphous Fumarate of compound (I) according to claim 7(a), comprising:
a) dissolving Fumarate Type A of compound (I) in MeOH; and
b) removing the MeOH by rotatory evaporation at about 60 °C; or
(k) Amorphous Freebase of compound (I) according to claim 7(b), comprising:
a) dissolving Freebase Type B of compound (I) in DCM; and
b) removing the DCM by rotatory evaporation at about 40 °C.

13. A pharmaceutical composition comprising the crystalline form according to any one of claims 1-5 or the amorphous form according to any one of claims 6-7, and a pharmaceutically acceptable carrier or excipient.

14. A dosage form comprising a therapeutically effective amount of the crystalline form according to any one of claims 1-5, the amorphous form according to any one of claims 6-7, or the pharmaceutical composition according to claim 13.

15. The crystalline form according to any one of claims 1-5 or amorphous form according to any one of claims 6-7 for use in treating or ameliorating a hyperproliferative disease, optionally wherein:
(a) the hyperproliferative disease is cancer, further optionally wherein (i) the cancer is ErbB2 positive; and/or (ii) the cancer is selected from breast, gastric, biliary, colorectal, brain, lung, NSCLC, pancreatic, head and neck, ovarian and uterine cancer; and/or
(b) one or more additional compounds having anti-cancer properties are administered in combination.
